# EUROPEAN PATENT SPECIFICATION

(11) **EP 4 159 871 B1**
(45) Date of publication and mention of the grant of the patent: **19.02.2025**
(21) Application number: 22208796.7
(22) Date of filing: 20.03.2018
(51) Int. Cl.: C12Q 1/6813, C12Q 1/6869, G16B 25/00, G06N 20/00, G16B 25/10, G16B 5/00

(54) **SYNTHETIC MULTIPLETS FOR MULTIPLETS DETERMINATION**
SYNTHETISCHE MULTITS ZUR MULTITS-BESTIMMUNG
MULTI-POINT SYNTHÉTIQUE POUR DÉTERMINATION DE MULTI-POINT

(30) Priority: 24.03.2017 US 201762476522 P
(43) Date of publication of application: 05.04.2023
(62) Divisional of application: 18716453.8
(73) Proprietor: Becton, Dickinson and Company, Franklin Lakes, NJ 07417 (US)
(72) Inventor: FAN, Jue, Menlo Park, CA 94025 (US); FAN, Christina, Menlo Park, CA 94025 (US); ROSENFELD, David, Menlo Park, CA 94025 (US)
(74) Representative: Carpmaels & Ransford LLP

(56) References cited:
- WO-A1-2016/040476
- LAKE BLUE B. ET AL: "Neuronal subtypes and diversity revealed by single-nucleus RNA sequencing of the human brain", SCIENCE, vol. 352, no. 6293, 24 June 2016 (2016-06-24), US, pages 1586 - 1590, XP093022344, ISSN: 0036-8075, DOI: 10.1126/science.aaf1204
- LAKE B ET AL: "Supplementary Figures for XP093022344: Neuronal subtypes and diversity revealed by single-nucleus RNA sequencing of the human brain", SCIENCE, vol. 352, no. 6293, 27 September 2016 (2016-09-27), pages 1586 - 1590, XP093022371
- TOMISLAV ILICIC ET AL: "Classification of low quality cells from single-cell RNA-seq data", GENOME BIOLOGY, vol. 17, no. 1, 17 February 2016 (2016-02-17), XP055379181, DOI: 10.1186/s13059-016-0888-1
- ZIEGENHAIN CHRISTOPH ET AL: "Comparative Analysis of Single-Cell RNA Sequencing Methods", MOLECULAR CELL, ELSEVIER, AMSTERDAM, NL, vol. 65, no. 4, 16 February 2017 (2017-02-16), pages 631, XP029924365, ISSN: 1097-2765, DOI: 10.1016/J.MOLCEL.2017.01.023

## Description

### BACKGROUND

### Field

The present disclosure relates generally to the field of detection and analysis of expression profiles of cells using molecular barcoding, and more particularly identifying multiplet expression profiles.

### Description of the Related Art

Methods and techniques such as stochastic barcoding are useful for cell analysis. For example, stochastic barcoding can be used to decipher cell physiological conditions, for example protein and/or gene expression profiles of single cells, to determine their states using, for example, reverse transcription, polymerase chain reaction (PCR) amplification, and next generation sequencing (NGS). However, a detected expression profile may be associated with two or more cells of different types, which can skew the interpretation of the expression profile.

WO 2016/040476 A1 describes a droplet-based method and apparatus for composite single-cell nucleic acid analysis. Lake et al (Science, 352(6293), 1586-1590 (2016)) describes neuronal subtypes and diversity revealed by single-nucleus RNA sequencing of the human brain. Ilicic et al (Genome Biol 17, 29 (2016)) describes "Classification of low quality cells from single-cell RNA-seq data. Ziegenhain et al (Molecular cell, 65(4), 631-643.e4. (2017)) describes a comparative analysis of single-cell RNA sequencing methods.

### SUMMARY

The invention is set out in the appended set of claims. The terminologies "embodiment" and "embodiments" are to be construed as embodiment(s) of the invention only in as far as they fall within the scope of the present claims. Otherwise, they refer to embodiments of the disclosure solely.

Disclosed herein are methods for identifying a multiplet expression profile. In some embodiments, the method comprises: (a) barcoding (e.g., stochastically barcoding) a plurality of targets in a plurality of cells using a plurality of barcodes (e.g., stochastic barcodes) to create a plurality of barcoded targets (e.g., stochastically barcoded targets) for each cell of the plurality of cells, wherein each of the plurality of barcodes comprises a cell label and a molecular label, wherein molecular labels of at least two barcodes of the plurality of barcodes comprise different molecular label sequences, and wherein at least two barcodes of the plurality of barcodes comprise cell labels with an identical cell label sequence; (b) obtaining sequencing data of the plurality of barcoded targets; (c) determining a plurality of expression profiles associated with cell labels of the plurality of barcodes from the sequencing data obtained in (b), wherein an expression profile of the plurality of expression profiles associated with a cell label of the cell labels of the plurality of barcodes comprises a number of molecular labels with distinct sequences associated with the cell label and each target of the plurality of targets in the sequencing data; (d) generating a plurality of synthetic multiplet expression profiles from the plurality of expression profiles associated with the cell labels of the plurality of barcodes determined in (c); and (e) identifying an expression profile of the plurality of expression profiles associated with a cell label of the cell labels of the plurality of barcodes as a singlet or a multiplet based on expression profiles of the plurality of synthetic doublet expression profiles generated in (d).

In some embodiments, the method comprises: if the expression profile is identified as a multiplet in (e), removing sequencing data associated with the expression profile from the sequencing data obtained in (b). The method can comprise: if the expression profile is identified as a multiplet in (e), removing the expression profile from the plurality of expression profiles determined in (c). The plurality of multiplets can comprise a doublet, a triplet, or any combination thereof.

In some embodiments, generating the plurality of synthetic multiplet expression profiles from the plurality of expression profiles associated with the cell labels of the plurality of barcodes determined in (c) comprises: for a synthetic multiplet expression profile of the plurality of synthetic multiplet expression profiles, (1) selecting a first expression profile of the plurality of expression profiles determined in (c), wherein the first expression profile is associated with a first cell label sequence; (2) selecting a second expression profile of the plurality of expression profiles determined in (c), wherein the second expression profile is associated with a second cell label sequence, and wherein the first cell label sequence and the second cell label sequence comprise different cell label sequences; and (3) combining the first expression profile selected in (1) and the second expression profile selected in (2) to generate a synthetic multiplet expression profile.

In some embodiments, combining the first expression profile selected in (1) and the second expression profile selected in (2) to generate the synthetic multiplet expression profile comprises: for each of the plurality of targets, combining a number of molecular labels with distinct sequences associated with the target in the first expression profile and a number of molecular labels with distinct sequences associated with the target in the second expression profile to generate a number of molecular labels with distinct sequences associated with the synthetic multiplet expression profile.

In some embodiments, the number of molecular labels with distinct sequences associated with the synthetic multiplet expression profile is a sum of the number of molecular labels with distinct sequences associated with the target in the first expression profile and the number of molecular labels with distinct sequences associated with the target in the second expression profile. The sum can be a weighted sum of the number of molecular labels with distinct sequences associated with the target in the first expression profile and the number of molecular labels with distinct sequences associated with the target in the second expression profile.

In some embodiments, the number of molecular labels with distinct sequences associated with the synthetic multiplet expression profile is an average of the number of molecular labels with distinct sequences associated with the target in the first expression profile and the number of molecular labels with distinct sequences associated with the target in the second expression profile. The average can be a weighted average of the number of molecular labels with distinct sequences associated with the target in the first expression profile and the number of molecular labels with distinct sequences associated with the target in the second expression profile.

In some embodiments, identifying the expression profile of the plurality of expression profiles associated with the cell label of the cell labels of the plurality of barcodes as a singlet or a multiplet based on the expression profiles of the plurality of synthetic multiplet expression profiles generated in (d) comprises: (1) training a machine learning model for expression profile multiplet identification from the expression profiles of the plurality of synthetic multiplet expression profiles generated in (d) and one or more expression profiles of the plurality of expression profiles determined in (c); and (2) identifying the expression profile of the plurality of expression profiles associated with the cell label of the cell labels of the plurality of barcodes as a singlet or a multiplet based on the expression profile using the machine learning model. The one or more expression profiles of the plurality of expression profiles used in training the machine learning model can comprise a percentage of the plurality of expression profiles determined in (c). The percentage can be approximately 10%.

In some embodiments, the machine learning model comprises a classification model. The classification model can comprise a supervised classification model, a semi-supervised classification model, an unsupervised classification model, or a combination thereof. The machine learning model can comprise a neural network, a linear regression model, a logistic regression model, a decision tree, a support vector machine, a Naive Bayes network, a k-nearest neighbors (KNN) model, a k-means model, a random forest model, or any combination thereof. The machine learning model can comprise an association rule learning model, an inductive logic programming model, a reinforcement learning model, a feature learning model, a similarity learning model, a sparse dictionary learning model, a genetic algorithm model, a rule-based machine learning model, a learning classifier system model, or any combination thereof.

In some embodiments, training the machine learning model for expression profile multiplet identification from the expression profiles of the plurality of synthetic multiplet expression profiles generated in (d) and the one or more expression profiles of the plurality of expression profiles determined in (c) comprises: (1) projecting the expression profiles of the plurality of synthetic multiplet expression profiles generated in (d) from an expression profile space into a lower dimensional projection space to generate projected expression profiles of the plurality of synthetic multiplet expression profiles; (2) projecting the one or more expression profiles of the plurality of expression profiles determined in (c) from the expression profile space into the lower dimensional projection space to generate one or more projected expression profiles of the plurality of expression profiles; and (3) training the machine learning model for expression profile multiplet identification from the projected expression profiles of the plurality of synthetic multiplet expression profiles from (1) and the one or more projected expression profiles of the plurality of expression profiles in (2).

In some embodiments, the method comprises: projecting the expression profile of the plurality of barcodes associated with the cell label of the cell labels of the plurality of barcodes to generate a projected expression profile of the plurality of barcodes, wherein identifying the expression profile of the plurality of expression profiles associated with the cell label of the cell labels of the plurality of barcodes as a singlet or a multiplet based on the expression profile using the machine learning model comprises: identifying the expression profile of the plurality of expression profiles associated with the cell label of the cell labels of the plurality of barcodes as a singlet or a multiplet based on the expression profile using the machine learning model and the projected expression profile of the plurality of barcodes.

In some embodiments, the lower dimensional space is a two dimensional space. Projecting the expression profiles of the plurality of synthetic multiplet expression profiles generated in (d) from the expression profile space into the lower dimensional projection space to generate projected expression profiles of the plurality of synthetic multiplet expression profiles can comprise: projecting the expression profiles of the plurality of synthetic multiplet expression profiles generated in (d) from the expression profile space into the lower dimensional projection space to generate projected expression profiles of the plurality of synthetic multiplet expression profiles using a t-distributed stochastic neighbor embedding (tSNE) method.

In some embodiments, identifying the expression profile of the plurality of expression profiles associated with the cell label of the cell labels of the plurality of barcodes as a singlet or a multiplet based on the expression profiles of the plurality of synthetic multiplet expression profiles generated comprises: identifying the expression profile of the plurality of expression profiles associated with the cell label of the cell labels of the plurality of barcodes as a singlet or a multiplet based on the expression profile based on: a first distance between the expression profile of the plurality of expression profiles associated with the cell label and at least one expression profile of the plurality of expression profiles, and a second distance between the expression profile of the plurality of expression profiles associated with the cell label and at least one synthetic multiplet expression profile of the plurality of synthetic multiplet expression profiles.

In some embodiments, identifying the expression profile of the plurality of expression profiles associated with the cell label of the cell labels of the plurality of barcodes as a singlet or a multiplet based on the expression profiles of the plurality of synthetic multiplet expression profiles generated comprises: (1) clustering the plurality of expression profiles into a first cluster of expression profiles; (2) clustering the plurality of synthetic multiplet expression profiles into a second cluster of synthetic multiplet expression profiles; and (3) identifying the expression profile of the plurality of expression profiles associated with the cell label of the cell labels of the plurality of barcodes as a singlet or a multiplet based on the expression profile based on: a first distance between the expression profile of the plurality of expression profiles associated with the cell label and the first cluster of expression profiles, and a second distance between the expression profile of the plurality of expression profiles associated with the cell label and a second cluster of synthetic multiplet expression profiles.

In some embodiments, identifying the profile of the plurality of profiles associated with the cell of the plurality of cells as a singlet or a multiplet based on the profiles of the plurality of synthetic multiplet profiles generated in (b) and the profile comprises: clustering the plurality of profiles into a first cluster of profiles; (2) clustering the plurality of synthetic multiplet profiles into a plurality of second clusters of synthetic multiplet profiles; and (3) identifying the profile of the plurality of profiles associated with the cell of the cells as a singlet or a multiplet based on: a second distance between the profile of the plurality of profiles associated with the cells and the first cluster of profiles, and second distances between the profile of the plurality of profiles associated with the cell and one or more second clusters of the plurality of second clusters of synthetic multiplet profiles.

In some embodiments, barcoding the plurality of targets in the plurality of cells using the plurality of barcodes to create the plurality of barcoded targets for the cells of the plurality of cells comprises: barcoding the plurality of targets in the plurality of cells using the plurality of barcodes of a plurality of particles to create the plurality of barcoded targets for each cell of the plurality of cells, wherein each of the plurality of particles comprises a subset of the plurality of barcodes, wherein each of the subset of barcodes comprise an identical cell label sequence and with at least 100 different molecular label sequences.

In some embodiments, the particle is a bead. The bead can be selected from the group consisting of streptavidin beads, agarose beads, magnetic beads, conjugated beads, protein A conjugated beads, protein G conjugated beads, protein A/G conjugated beads, protein L conjugated beads, oligodT conjugated beads, silica beads, silica-like beads, anti-biotin microbead, anti-fluorochrome microbead, and any combination thereof. The particle can comprise a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, sepharose, cellulose, nylon, silicone, and any combination thereof.

In some embodiments, the barcodes of each of the plurality of particles comprise molecular labels with at least 1000 or 10000 different molecular label sequences. The molecular labels of the barcodes can comprise random sequences. Each of the plurality of particles can comprise at least 10000 barcodes.

In some embodiments, barcoding the plurality of targets in the plurality of cells using the plurality of barcodes to create the plurality of barcoded targets for each cell of the plurality of cells comprises: (1) contacting copies of the targets with target-binding regions of the barcodes; and (2) reverse transcribing the plurality targets using the plurality of barcodes to create a plurality of reverse transcribed targets.

In some embodiments, prior to obtaining the sequencing data of the plurality of barcoded targets, amplifying the barcoded targets to generate a plurality of amplified barcoded targets. Amplifying the barcoded targets to generate the plurality of amplified barcoded targets can comprise: amplifying the barcoded targets by polymerase chain reaction (PCR).

In some embodiments, determining the plurality of expression profiles associated with the cell labels of the plurality of barcodes from the sequencing data obtained in (b) comprises: for an expression profile of the plurality of expression profiles associated with a cell label of the cell labels of the plurality of barcodes, determining a number of molecular labels with distinct sequences associated with the cell label and each target of the plurality of targets in the sequencing data obtained in (b). Determining the number of molecular labels with distinct sequences associated with the cell label and each target of the plurality of targets in the sequencing data obtained in (b) can comprise: for one or more of the plurality of targets, (1) counting the number of molecular labels with distinct sequences associated with the target in the sequencing data obtained in (b); and (2) estimating the number of the target based on the number of molecular labels with distinct sequences associated with the target in the sequencing data counted in (1).

Disclosed herein are methods for identifying a multiplet expression profile. In some embodiments, the method comprises: (a) receiving a plurality of expression profiles of a plurality of cells, wherein the expression profiles comprise an occurrence (or a copy or a number) of each target of a plurality of targets for each cell of the plurality of cells; (b) generating a plurality of synthetic multiplet expression profiles from the plurality of expression profiles of the plurality of cells; and (c) identifying an expression profile of the plurality of expression profiles associated with a cell of the plurality of cells as a singlet or a multiplet based on expression profiles of the plurality of synthetic multiplet expression profiles.

In some embodiments, the method comprises: if the expression profile is identified as a multiplet in (c), removing the expression profile from the plurality of expression profiles received in (a). The plurality of multiplets can comprise a doublet, a triplet, or any combination thereof.

In some embodiments, generating the plurality of synthetic multiplet expression profiles from the plurality of expression profiles of the plurality of cells can comprise: for a synthetic multiplet expression profile of the plurality of synthetic multiplet expression profiles, (1) selecting a number of expression profiles of the plurality of expression profiles; and (2) combining the expression profiles selected in (1) to generate a synthetic multiplet expression profile. Combining the expression profiles selected in (1) to generate the synthetic multiplet expression profile can comprise: for each of the plurality of targets, combining occurrences of the target in the expression profiles selected to generate an occurrence of the target in the synthetic multiplet expression profile.

In some embodiments, the occurrence of the target in the synthetic multiplet expression profile can be a sum of the occurrences of the target in the expression profiles selected. The sum can be a weighted sum of the occurrences of the target in the expression profiles selected. In some embodiments, the occurrence of the target in the synthetic multiplet expression profile is an average of the occurrences of the target in the expression profiles selected. The average can be a weighted average of the occurrences of the target in the expression profiles selected.

In some embodiments, the number of the plurality of synthetic multiplet expression profiles is approximately a percentage of the plurality of expression profiles received in (a). The percentage can be approximately 10%.

In some embodiments, identifying the expression profile of the plurality of expression profiles associated with the cell of the plurality of cells as a singlet or a multiplet based on the expression profiles of the plurality of synthetic multiplet expression profiles generated in (b) and the expression profile comprises: (1) training a machine learning model for expression profile multiplet identification from the expression profiles of the plurality of synthetic multiplet expression profiles generated in (b) and one or more expression profiles of the plurality of expression profiles received in (a); and (2) identifying the expression profile of the plurality of expression profiles associated with the cell of the plurality of cell as a singlet or a multiplet based on the expression profile using the machine learning model.

In some embodiments, the one or more expression profiles of the plurality of expression profiles used in training the machine learning model comprises a percentage of the plurality of expression profiles received in (a). The percentage can be approximately 10 %.

In some embodiments, he machine learning model comprises a classification model. The classification model can comprise a supervised classification model, a semi-supervised classification model, an unsupervised classification model, or any combination thereof. The machine learning model can comprise a neural network, a linear regression model, a logistic regression model, a decision tree, a support vector machine, a Naive Bayes network, a k-nearest neighbors (KNN) model, a k-means model, a random forest model, or any combination thereof. The machine learning model can comprise an association rule learning model, an inductive logic programming model, a reinforcement learning model, a feature learning model, a similarity learning model, a sparse dictionary learning model, a genetic algorithm model, a rule-based machine learning model, a learning classifier system model, or any combination thereof.

In some embodiments, training the machine learning model for expression profile multiplet identification from the expression profiles of the plurality of synthetic multiplet expression profiles generated in (b) and one or more expression profiles of the plurality of expression profiles received in (a) comprises: (1) projecting the expression profiles of the plurality of synthetic multiplet expression profiles generated in (b) from an expression profile space into a lower dimensional projection space to generate projected expression profiles of the plurality of synthetic multiplet expression profiles; (2) projecting the one or more expression profiles of the plurality of expression profiles received in (a) from the expression profile space into the lower dimensional projection space to generate one or more projected expression profiles of the plurality of expression profiles; and (3) training the machine learning model for expression profile multiplet identification from the projected expression profiles of the plurality of synthetic multiplet expression profiles from (1) and the one or more projected expression profiles of the plurality of expression profiles in (2).

In some embodiments, the method comprises: projecting the expression profile of the plurality of the plurality of expression profiles associated with the cell of the plurality of cell to generate a projected expression profile of the plurality of expression profiles, wherein identifying the expression profile of the plurality of expression profiles associated with the cell of the plurality of cell as a singlet or a multiplet based on the expression profile using the machine learning model comprises: identifying the expression profile of the plurality of expression profiles associated with the cell of the plurality of cells as a singlet or a multiplet based on the projected expression profile of the plurality of expression profiles using the machine learning model. The lower dimensional space can be a two dimensional space.

In some embodiments, projecting the expression profiles of the plurality of synthetic multiplet expression profiles generated in (b) from the expression profile space into the lower dimensional projection space to generate the projected expression profiles of the plurality of synthetic multiplet expression profiles comprises: projecting the expression profiles of the plurality of synthetic multiplet expression profiles generated in (b) from the expression profile space into the lower dimensional projection space to generate the projected expression profiles of the plurality of synthetic multiplet expression profiles comprises using a t-distributed stochastic neighbor embedding (tSNE) method.

In some embodiments, identifying the expression profile of the plurality of expression profiles associated with the cell of the plurality of cells as a singlet or a multiplet based on the expression profiles of the plurality of synthetic multiplet expression profiles generated in (b) and the expression profile comprises: identifying the expression profile of the plurality of expression profiles associated with the cell of the cells as a singlet or a multiplet based on the expression profile based on: a first distance between the expression profile of the plurality of expression profiles associated with the cell and at least one expression profile of the plurality of expression profiles, and a second distance between the expression profile of the plurality of expression profiles associated with the cell and at least one synthetic multiplet expression profile of the plurality of synthetic multiplet expression profiles.

In some embodiments, identifying the expression profile of the plurality of expression profiles associated with the cell of the plurality of cells as a singlet or a multiplet based on the expression profiles of the plurality of synthetic multiplet expression profiles generated in (b) and the expression profile comprises: (1) clustering the plurality of expression profiles into a first cluster of expression profiles; (2) lustering the plurality of synthetic multiplet expression profiles into a second cluster of synthetic multiplet expression profiles; and (3) identifying the expression profile of the plurality of expression profiles associated with the cell of the cells as a singlet or a multiplet based on the expression profile based on: a first distance between the expression profile of the plurality of expression profiles associated with the cell and the first cluster of expression profiles, and a second distance between the expression profile of the plurality of expression profiles associated with the cell and a second cluster of synthetic multiplet expression profiles.

In some embodiments, identifying the expression profile of the plurality of expression profiles associated with the cell of the plurality of cells as a singlet or a multiplet based on the expression profiles of the plurality of synthetic multiplet expression profiles generated in (b) and the expression profile comprises: (1) clustering the plurality of expression profiles into a first cluster of expression profiles; (2) lustering the plurality of synthetic multiplet expression profiles into a plurality of second clusters of synthetic multiplet expression profiles; and (3) identifying the expression profile of the plurality of expression profiles associated with the cell of the cells as a singlet or a multiplet based on the expression profile based on: a first distance between the expression profile of the plurality of expression profiles associated with the cell and the first cluster of expression profiles, and second distances between the expression profile of the plurality of expression profiles associated with the cell and one or more clusters of the plurality of second clusters of synthetic multiplet expression profiles.

In some embodiments, receiving the plurality of expression profiles of the plurality of cells comprises: (1) barcoding (e.g., stochastically barcoding) the plurality of targets in the plurality of cells using a plurality of barcodes (e.g., stochastic barcodes) to create a plurality of barcoded targets (e.g., stochastically barcoded targets) for cells of the plurality of cells, wherein each of the plurality of barcodes comprises a cell label and a molecular label, wherein molecular labels of at least two barcodes of the plurality of barcodes comprise different molecular label sequences, and wherein at least two barcodes of the plurality of barcodes comprise cell labels with an identical cell label sequence; (2) obtaining sequencing data of the plurality of barcoded targets; and (3) determining the plurality of expression profiles associated with cell labels of the plurality of stochastic barcodes from the sequencing data obtained in (2), wherein an expression profile of the plurality of expression profiles associated with a cell label of the cell labels of the plurality of barcodes comprises a number of molecular labels with distinct sequences associated with the cell label and each target of the plurality of targets in the sequencing data.

In some embodiments, barcoding the plurality of targets in the plurality of cells using the plurality of barcodes to create the plurality of barcoded targets for the cells of the plurality of cells comprises: barcoding the plurality of targets in the plurality of cells using the plurality of barcodes of a plurality of particles to create the plurality of barcoded targets for the cells of the plurality of cells, wherein each of the plurality of particles comprises a subset of the plurality of barcodes, wherein each of the subset of barcodes comprise an identical cell label sequence and with at least 100 different molecular label sequences.

In some embodiments, the particle is a bead. The bead can be selected from the group consisting of streptavidin beads, agarose beads, magnetic beads, conjugated beads, protein A conjugated beads, protein G conjugated beads, protein A/G conjugated beads, protein L conjugated beads, oligodT conjugated beads, silica beads, silica-like beads, anti-biotin microbead, anti-fluorochrome microbead, and any combination thereof. The particle can comprise a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, sepharose, cellulose, nylon, silicone, and any combination thereof.

In some embodiments, the barcodes of each of the plurality of particles comprise molecular labels with at least 1000 or 10000 different molecular label sequences. The molecular labels of the barcodes can comprise random sequences. Each of the plurality of particles can comprise at least 10000 barcodes.

In some embodiments, barcoding the plurality of targets in the plurality of cells using the plurality of barcodes to create the plurality of barcoded targets for each cell of the plurality of cells comprises: (1) contacting copies of the targets with target-binding regions of the barcodes; and (2) reverse transcribing the plurality targets using the plurality of barcodes to create a plurality of reverse transcribed targets.

In some embodiments, the method comprises: prior to obtaining the sequencing data of the plurality of barcoded targets, amplifying the barcoded targets to generate a plurality of amplified barcoded targets. Amplifying the barcoded targets to generate the plurality of amplified barcoded targets can comprise: amplifying the barcoded targets by polymerase chain reaction (PCR).

In some embodiments, determining the plurality of expression profiles associated with the cell labels of the plurality of barcodes from the sequencing data comprises: for an expression profile of the plurality of expression profiles associated with a cell label of the cell labels of the plurality of barcodes, determining a number of molecular labels with distinct sequences associated with the cell label and each target of the plurality of targets in the sequencing data. Determining the number of molecular labels with distinct sequences associated with the cell label and each target of the plurality of targets in the sequencing data can comprise: for one or more of the plurality of targets, (1) counting the number of molecular labels with distinct sequences associated with the target in the sequencing data; and (2) estimating the number of the target based on the number of molecular labels with distinct sequences associated with the target in the sequencing data.

Disclosed herein are methods for identifying a multiplet profile. In some embodiments, the method comprises: (a) receiving a plurality of profiles of a plurality of cells; (b) generating a plurality of synthetic multiplet profiles from the plurality of profiles of the plurality of cells; and (c) identifying a profile of the plurality of profiles associated with a cell of the plurality of cells as a singlet or a multiplet based on profiles of the plurality of synthetic multiplet profiles generated in (b). A profile of the plurality of profiles of the plurality of cells can comprise an mRNA expression profile of the cell, a protein expression profile of the cell, a mutation profile of the cell, a methylation profile of the cell, or any combination thereof.

In some embodiments, the mRNA expression profile can comprise an occurrence of mRNA molecules of each gene of a plurality of genes for each cell of the plurality of cells. The occurrence of each gene can comprise an absolute occurrence of the gene, a normalized occurrence of the gene, or a combination thereof. The normalized occurrence of the gene can be determined in a unit of Reads Per Kilobase of transcript per Million mapped reads (RPKM) or a unit of threshold count (Ct). The mRNA expression profile can determined by sequencing, quantitative polymerase chain reaction (qPCR), digital PCR, hybridization, or any combination thereof. In some embodiments, the protein expression profile of the cell comprises an occurrence of protein molecules corresponding to each gene of a plurality of genes for each cell of the plurality of cells. The mutation profile of the cell can comprise a mutation profile of the cell at multiple genome locations of the cell. The methylation profile of the cell can comprise a methylation profile of the cell at multiple genome locations of the cell.

In some embodiments, the method comprises: if the profile is identified as a multiplet in (c), removing the profile from the plurality of profiles received in (a). The plurality of multiplets can comprise a doublet, a triplet, or any combination thereof. In some embodiments, generating the plurality of synthetic multiplet profiles from the plurality of profiles of the plurality of cells comprises: for a synthetic multiplet profile of the plurality of synthetic multiplet profiles, (1) selecting a number of profiles of the plurality of profiles; and (2) combining the profiles selected in (1) to generate the synthetic multiplet profile.

In some embodiments, combining the profiles selected in (1) to generate the synthetic multiplet profile comprises: for each of the plurality of targets, combining values of corresponding elements in the profiles selected to generate a value of a corresponding element in the synthetic multiplet profile. The value of the corresponding element in the synthetic multiplet profile can be a sum of the values of the corresponding elements in the profiles selected. The sum can be a weighted sum of the values of the corresponding elements in the profiles selected. The occurrence of the target in the synthetic multiplet profile can be an average of the values of the corresponding elements in the profiles selected. The average can be a weighted average of the values of the corresponding elements in the profiles selected.

In some embodiments, the number of the plurality of synthetic multiplet profiles is approximately a percentage of the plurality of profiles received in (a). The percentage can be approximately 10 percent.

In some embodiments, identifying the profile of the plurality of profiles associated with the cell of the plurality of cells as a singlet or a multiplet based on the profiles of the plurality of synthetic multiplet profiles generated in (b) and the profile comprises: (1) training a machine learning model for profile multiplet identification from the profiles of the plurality of synthetic multiplet profiles generated in (b) and one or more profiles of the plurality of profiles received in (a); and (2) identifying the profile of the plurality of profiles associated with the cell of the plurality of cell as a singlet or a multiplet based on the profile using the machine learning model.

In some embodiments, the one or more profiles of the plurality of profiles used in training the machine learning model comprises a percentage of the plurality of profiles received in (b). The percentage can be approximately 10 percent. The machine learning model can comprise a classification model.

In some embodiments, he classification model comprises a supervised classification model, a semi-supervised classification model, an unsupervised classification model, or a combination thereof. The machine learning model can comprise a neural network, a linear regression model, a logistic regression model, a decision tree, a support vector machine, a Naive Bayes network, a k-nearest neighbors (KNN) model, a k-means model, a random forest model, or any combination thereof. The machine learning model can comprise an association rule learning model, an inductive logic programming model, a reinforcement learning model, a feature learning model, a similarity learning model, a sparse dictionary learning model, a genetic algorithm model, a rule-based machine learning model, a learning classifier system model, or any combination thereof.

In some embodiments, training the machine learning model for profile multiplet identification from the profiles of the plurality of synthetic multiplet profiles generated in (b) and one or more profiles of the plurality of profiles received in (a) comprises: (1) projecting the profiles of the plurality of synthetic multiplet profiles generated in (b) from a profile space into a lower dimensional projection space to generate projected profiles of the plurality of synthetic multiplet profiles; (2) projecting the one or more profiles of the plurality of profiles received in (a) from the profile space into the lower dimensional projection space to generate one or more projected profiles of the plurality of profiles; and (3) training the machine learning model for profile multiplet identification from the projected profiles of the plurality of synthetic multiplet profiles from (1) and the one or more projected profiles of the plurality of profiles in (1).

In some embodiments, the method comprises: projecting the profile of the plurality of the plurality of profiles associated with the cell of the plurality of cell to generate a projected profile of the plurality of profiles, wherein identifying the profile of the plurality of profiles associated with the cell of the plurality of cell as a singlet or a multiplet based on the profile using the machine learning model comprises: identifying the profile of the plurality of profiles associated with the cell of the plurality of cells as a singlet or a multiplet based on the projected profile of the plurality of profiles using the machine learning model. The lower dimensional space can be a two dimensional space. In some embodiments, projecting the profiles of the plurality of synthetic multiplet profiles generated in (b) from the profile space into the lower dimensional projection space to generate the projected profiles of the plurality of synthetic multiplet profiles comprises: projecting the profiles of the plurality of synthetic multiplet profiles generated in (b) from the profile space into the lower dimensional projection space to generate the projected profiles of the plurality of synthetic multiplet profiles comprises using a t-distributed stochastic neighbor embedding (tSNE) method.

In some embodiments, identifying the profile of the plurality of profiles associated with the cell of the plurality of cells as a singlet or a multiplet based on the profiles of the plurality of synthetic multiplet profiles generated in (b) and the profile comprises: identifying the profile of the plurality of profiles associated with the cell of the cells as a singlet or a multiplet based on: a first distance between the profile of the plurality of profiles associated with the cell and at least one profile of the plurality of profiles, and a second distance between the profile of the plurality of expression profiles associated with the cell and at least one synthetic multiplet expression profile of the plurality of synthetic multiplet expression profiles.

In some embodiments, identifying the profile of the plurality of profiles associated with the cell of the plurality of cells as a singlet or a multiplet based on the profiles of the plurality of synthetic multiplet profiles generated in (b) and the profile comprises: clustering the plurality of profiles into a first cluster of profiles; (2) clustering the plurality of synthetic multiplet profiles into a second cluster of synthetic multiplet profiles; and (3) identifying the profile of the plurality of profiles associated with the cell of the cells as a singlet or a multiplet based on: a second distance between the profile of the plurality of profiles associated with the cells and the first cluster of profiles, and a second distance between the profile of the plurality of profiles associated with the cell and the second cluster of synthetic multiplet profiles.

In some embodiments, identifying the profile of the plurality of profiles associated with the cell of the plurality of cells as a singlet or a multiplet based on the profiles of the plurality of synthetic multiplet profiles generated in (b) and the profile comprises: clustering the plurality of profiles into a first cluster of profiles; (2) clustering the plurality of synthetic multiplet profiles into a plurality of second clusters of synthetic multiplet profiles; and (3) identifying the profile of the plurality of profiles associated with the cell of the cells as a singlet or a multiplet based on: a second distance between the profile of the plurality of profiles associated with the cells and the first cluster of profiles, and second distances between the profile of the plurality of profiles associated with the cell and one or more clusters of the plurality of second clusters of synthetic multiplet profiles.

Disclosed herein are systems for determining the number of targets. In some embodiments, the system comprises: a hardware processor; and non-transitory memory having instructions stored thereon, which when executed by the hardware processor causes the processor to perform the method of any method above. Disclosed herein are computer readable media for determining the number of targets. In some embodiments, the computer readable medium comprises codes for performing any method above.

### BRIEF DESCRIPTION OF THE DRAWINGS

FIG. 1 illustrates a non-limiting exemplary stochastic barcode.
FIG. 2 shows a non-limiting exemplary workflow of stochastic barcoding and digital counting.
FIG. 3 is a schematic illustration showing a non-limiting exemplary process for generating an indexed library of the stochastically barcoded targets from a plurality of targets.
FIG. 4 shows a non-limiting exemplary workflow of integrating synthetic doublet analysis into an analysis pipeline.
FIG. 5 is a flowchart showing a non-limiting exemplary method of synthetic doublet analysis.
FIG. 6 shows a non-limiting exemplary instrument suitable to use in the methods of the disclosure.
FIG. 7 illustrates a non-limiting exemplary architecture of a computer system that can be used in connection with embodiments of the present disclosure.
FIG. 8 illustrates a non-limiting exemplary architecture showing a network with a plurality of computer systems suitable for use in the methods of the disclosure.
FIG. 9 illustrates a non-limiting exemplary architecture of a multiprocessor computer system using a shared virtual address memory space in accordance with the methods of the disclosure.
FIG. 10A shows a non-limiting exemplary tSNE projection plot of expression profiles of single cells in a sample comprising approximately equal numbers of Jurkat cells and Ramos cells. The tSNE projection plot includes a cluster corresponding to expression profiles of Jurkat cells, a cluster corresponding to expression profiles of Ramos cells, and a cluster corresponding to doublet expression profiles of mixed cell types.
FIG. 10B shows a non-limiting exemplary tSNE projection plot of the expression profiles in FIG. 10A and 2% of synthetic doublet expression profiles. The cluster corresponding to the synthetic doublet expression profiles overlaps the cluster corresponding to the doublet expression profiles in FIG. 10A.
FIG. 11 shows a non-limiting exemplary tSNE projection plot of expression profiles of single cells in a sample comprising approximately equal numbers of Jurkat cells, K562 cells, and Ramos cells. The clusters corresponding to synthetic doublet expression profiles overlap the clusters corresponding to doublet expression profiles of mixed cell types observed.
FIG. 12 shows a non-limiting exemplary tSNE projection plot of expression profiles obtained from single cells in a human PBMC sample.
FIG. 13 is a non-limiting exemplary tSNE projection plot of expression profiles of single cells from 12 samples.
FIG. 14 shows a non-limiting exemplary workflow of generating, visualizing, and removing synthetic doublet expression profiles using a non-limiting exemplary user interface.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. It will be readily understood that the aspects of the present disclosure, as generally described herein, and illustrated in the Figures, can be arranged, substituted, combined, separated, and designed in a wide variety of different configurations.

Quantifying small numbers of nucleic acids or targets, for example messenger ribonucleotide acid (mRNA) molecules, is clinically important for determining, for example, the genes that are expressed in a cell at different stages of development or under different environmental conditions. However, it can be very challenging to determine the absolute number of nucleic acid molecules (e.g., mRNA molecules), especially when the number of molecules is very small. One method to determine the absolute number of molecules in a sample is digital polymerase chain reaction (PCR). Barcodes, such as stochastic barcodes, with unique molecular labels (MLs, also referred to as molecular indexes (MIs)) can be used to count the numbers of molecules. Barcodes with molecular labels that are unique for each cell label can be used to count the numbers of molecules in each cell. Non-limiting exemplary assays for barcoding include the Precise^{™} assay (Becton, Dickinson and Company (Franklin Lakes, NJ)) or the Rhapsody^{™} assay (Becton, Dickinson and Company (Franklin Lakes, NJ)).

The Precise^{™} assay can utilize a non-depleting pool of barcodes (e.g., stochastic barcodes) with large number, for example 6561 to 65536, unique molecular labels on poly(T) oligonucleotides to hybridize to all poly(A)-mRNAs in a sample during the RT step. In addition to molecular labels, cell labels of barcodes can be used to identify each single cell in each well of the Rhapsody^{™} plate. A barcode can comprise a universal PCR priming site. During RT, target gene molecules react randomly with barcodes. Each target molecule can hybridize to a barcode to generate barcoded, such as stochastically barcoded, complementary ribonucleotide acid (cDNA) molecules). After labeling, barcoded cDNA molecules from microwells of a microwell plate can be pooled into a single tube for PCR amplification and sequencing. Raw sequencing data can be analyzed to produce the numbers of barcodes with unique molecular labels.

Disclosed herein are methods for identifying targets to distinguish cell types. In some embodiments, the method comprises: (a) receiving a target counts data structure, wherein the target counts data structure comprises expression profiles of a plurality of cells, and wherein the expression profiles of the plurality of cells comprises a number of each target of a plurality of targets for each cell of the plurality of cells; (b) hierarchically clustering expression profiles of the plurality of cells based on the target counts data structure and distances between the expression profiles of the plurality of cells to generate a dendrogram representing the expression profiles of plurality of cells, wherein the dendrogram comprises a plurality of nodes, wherein the plurality of nodes comprise a root node, a plurality of leaf nodes, and a plurality of non-root, non-leaf nodes, wherein each leaf node of the plurality of leaf nodes represents an expression profile of a different cell of the plurality of cells, and wherein the root node represents expression profiles of the plurality of cells; (c) while traversing through each node of the plurality of nodes of the dendrogram from the root node of the dendrogram to the plurality of leaf nodes of the dendrogram: (1) determining whether a splitting of the node into child nodes of the node is valid or invalid (e.g., the differences between the child nodes are not significant); and (2) if the splitting of the node into the child nodes of the node is invalid, adding the node to a merging cluster set; (d) iteratively, for each first node in the merging cluster set, if a distance between the first node in the merging cluster set and a second node in the merging cluster set that is closest to the first node is within a merging distance threshold, merging the first node with the second node to generate a merged node comprising expression profiles represented by the first node and the second node; and (e) for each node in the merging cluster set, identifying targets for distinguishing cell types based on expression profiles of the plurality of targets of cells represented by the node.

Disclosed herein systems for identifying targets to distinguish cell types. In some embodiments, the system comprises: a hardware processor; and non-transitory memory having instructions stored thereon, which when executed by the hardware processor cause the processor to perform any of the methods disclosed herein. Disclosed herein are computer readable media for identifying targets for distinguishing cell types. In some embodiments, the computer readable medium comprises code for performing any of the methods disclosed herein.

### Definitions

Unless defined otherwise, technical and scientific terms used herein have the same meaning as commonly understood by one of ordinary skill in the art to which the present disclosure belongs. *See, e.g.,* Singleton et al., Dictionary of Microbiology and Molecular Biology 2nd ed., J. Wiley & Sons (New York, NY 1994); Sambrook et al., Molecular Cloning, A Laboratory Manual, Cold Spring Harbor Press (Cold Spring Harbor, NY 1989). For purposes of the present disclosure, the following terms are defined below.

As used herein, the term "adaptor" can mean a sequence to facilitate amplification or sequencing of associated nucleic acids. The associated nucleic acids can comprise target nucleic acids. The associated nucleic acids can comprise one or more of spatial labels, target labels, sample labels, indexing label, barcodes, stochastic barcodes, or molecular labels. The adapters can be linear. The adaptors can be pre-adenylated adapters. The adaptors can be double- or single-stranded. One or more adaptor can be located on the 5' or 3' end of a nucleic acid. When the adaptors comprise known sequences on the 5' and 3' ends, the known sequences can be the same or different sequences. An adaptor located on the 5' and/or 3' ends of a polynucleotide can be capable of hybridizing to one or more oligonucleotides immobilized on a surface. An adapter can, in some embodiments, comprise a universal sequence. A universal sequence can be a region of nucleotide sequence that is common to two or more nucleic acid molecules. The two or more nucleic acid molecules can have regions of different sequence. Thus, for example, the 5' adapters can comprise identical and/or universal nucleic acid sequences and the 3' adapters can comprise identical and/or universal sequences. A universal sequence that may be present in different members of a plurality of nucleic acid molecules can allow the replication or amplification of multiple different sequences using a single universal primer that is complementary to the universal sequence. Similarly, at least one, two (e.g., a pair) or more universal sequences that may be present in different members of a collection of nucleic acid molecules can allow the replication or amplification of multiple different sequences using at least one, two (e.g., a pair) or more single universal primers that are complementary to the universal sequences. Thus, a universal primer includes a sequence that can hybridize to such a universal sequence. The target nucleic acid sequence-bearing molecules may be modified to attach universal adapters (e.g., non-target nucleic acid sequences) to one or both ends of the different target nucleic acid sequences. The one or more universal primers attached to the target nucleic acid can provide sites for hybridization of universal primers. The one or more universal primers attached to the target nucleic acid can be the same or different from each other.

As used herein the term "associated" or "associated with" can mean that two or more species are identifiable as being co-located at a point in time. An association can mean that two or more species are or were within a similar container. An association can be an informatics association, where for example digital information regarding two or more species is stored and can be used to determine that one or more of the species were co-located at a point in time. An association can be a physical association. In some embodiments, two or more associated species are "tethered", "attached", or "immobilized" to one another or to a common solid or semisolid surface. An association may refer to covalent or non-covalent means for attaching labels to solid or semi-solid supports such as beads. An association may be a covalent bond between a target and a label.

As used herein, the term "complementary" can refer to the capacity for precise pairing between two nucleotides. For example, if a nucleotide at a given position of a nucleic acid is capable of hydrogen bonding with a nucleotide of another nucleic acid, then the two nucleic acids are considered to be complementary to one another at that position. Complementarity between two single-stranded nucleic acid molecules may be "partial," in which only some of the nucleotides bind, or it may be complete when total complementarity exists between the single-stranded molecules. A first nucleotide sequence can be said to be the "complement" of a second sequence if the first nucleotide sequence is complementary to the second nucleotide sequence. A first nucleotide sequence can be said to be the "reverse complement" of a second sequence, if the first nucleotide sequence is complementary to a sequence that is the reverse (i.e., the order of the nucleotides is reversed) of the second sequence. As used herein, the terms "complement", "complementary", and "reverse complement" can be used interchangeably. It is understood from the disclosure that if a molecule can hybridize to another molecule it may be the complement of the molecule that is hybridizing.

As used herein, the term "digital counting" can refer to a method for estimating a number of target molecules in a sample. Digital counting can include the step of determining a number of unique labels that have been associated with targets in a sample. This stochastic methodology transforms the problem of counting molecules from one of locating and identifying identical molecules to a series of yes/no digital questions regarding detection of a set of predefined labels.

As used herein, the term "label" or "labels" can refer to nucleic acid codes associated with a target within a sample. A label can be, for example, a nucleic acid label. A label can be an entirely or partially amplifiable label. A label can be entirely or partially sequencable label. A label can be a portion of a native nucleic acid that is identifiable as distinct. A label can be a known sequence. A label can comprise a junction of nucleic acid sequences, for example a junction of a native and non-native sequence. As used herein, the term "label" can be used interchangeably with the terms, "index", "tag," or "label-tag." Labels can convey information. For example, in various embodiments, labels can be used to determine an identity of a sample, a source of a sample, an identity of a cell, and/or a target.

As used herein, the term "non-depleting reservoirs" can refer to a pool of barcodes (e.g., stochastic barcodes) made up of many different labels. A non-depleting reservoir can comprise large numbers of different barcodes such that when the non-depleting reservoir is associated with a pool of targets each target is likely to be associated with a unique barcode (e.g., a stochastic barcode). The uniqueness of each labeled target molecule can be determined by the statistics of random choice, and depends on the number of copies of identical target molecules in the collection compared to the diversity of labels. The size of the resulting set of labeled target molecules can be determined by the stochastic nature of the barcoding process, and analysis of the number of barcodes detected then allows calculation of the number of target molecules present in the original collection or sample. When the ratio of the number of copies of a target molecule present to the number of unique barcodes is low, the labeled target molecules are highly unique (i.e., there is a very low probability that more than one target molecule will have been labeled with a given label).

As used herein, the term "nucleic acid" refers to a polynucleotide sequence, or fragment thereof. A nucleic acid can comprise nucleotides. A nucleic acid can be exogenous or endogenous to a cell. A nucleic acid can exist in a cell-free environment. A nucleic acid can be a gene or fragment thereof. A nucleic acid can be DNA. A nucleic acid can be RNA. A nucleic acid can comprise one or more analogs (e.g., altered backbone, sugar, or nucleobase). Some non-limiting examples of analogs include: 5-bromouracil, peptide nucleic acid, xeno nucleic acid, morpholinos, locked nucleic acids, glycol nucleic acids, threose nucleic acids, dideoxynucleotides, cordycepin, 7-deaza-GTP, fluorophores (e.g., rhodamine or fluorescein linked to the sugar), thiol containing nucleotides, biotin linked nucleotides, fluorescent base analogs, CpG islands, methyl-7-guanosine, methylated nucleotides, inosine, thiouridine, pseudouridine, dihydrouridine, queuosine, and wyosine. "Nucleic acid", "polynucleotide, "target polynucleotide", and "target nucleic acid" can be used interchangeably.

A nucleic acid can comprise one or more modifications (e.g., a base modification, a backbone modification), to provide the nucleic acid with a new or enhanced feature (e.g., improved stability). A nucleic acid can comprise a nucleic acid affinity tag. A nucleoside can be a base-sugar combination. The base portion of the nucleoside can be a heterocyclic base. The two most common classes of such heterocyclic bases are the purines and the pyrimidines. Nucleotides can be nucleosides that further include a phosphate group covalently linked to the sugar portion of the nucleoside. For those nucleosides that include a pentofuranosyl sugar, the phosphate group can be linked to the 2', the 3', or the 5' hydroxyl moiety of the sugar. In forming nucleic acids, the phosphate groups can covalently link adjacent nucleosides to one another to form a linear polymeric compound. In turn, the respective ends of this linear polymeric compound can be further joined to form a circular compound; however, linear compounds are generally suitable. In addition, linear compounds may have internal nucleotide base complementarity and may therefore fold in a manner as to produce a fully or partially double-stranded compound. Within nucleic acids, the phosphate groups can commonly be referred to as forming the internucleoside backbone of the nucleic acid. The linkage or backbone can be a 3' to 5' phosphodiester linkage.

A nucleic acid can comprise a modified backbone and/or modified internucleoside linkages. Modified backbones can include those that retain a phosphorus atom in the backbone and those that do not have a phosphorus atom in the backbone. Suitable modified nucleic acid backbones containing a phosphorus atom therein can include, for example, phosphorothioates, chiral phosphorothioates, phosphorodithioates, phosphotriesters, aminoalkyl phosphotriesters, methyl and other alkyl phosphonate such as 3'-alkylene phosphonates, 5'-alkylene phosphonates, chiral phosphonates, phosphinates, phosphoramidates including 3'-amino phosphoramidate and aminoalkyl phosphoramidates, phosphorodiamidates, thionophosphoramidates, thionoalkylphosphonates, thionoalkylphosphotriesters, selenophosphates, and boranophosphates having normal 3'-5' linkages, 2'-5' linked analogs, and those having inverted polarity wherein one or more internucleotide linkages is a 3' to 3', a 5' to 5' or a 2' to 2' linkage.

A nucleic acid can comprise polynucleotide backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These can include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and CH2 component parts.

A nucleic acid can comprise a nucleic acid mimetic. The term "mimetic" can be intended to include polynucleotides wherein only the furanose ring or both the furanose ring and the internucleotide linkage are replaced with non-furanose groups, replacement of only the furanose ring can be referred as being a sugar surrogate. The heterocyclic base moiety or a modified heterocyclic base moiety can be maintained for hybridization with an appropriate target nucleic acid. One such nucleic acid can be a peptide nucleic acid (PNA). In a PNA, the sugar-backbone of a polynucleotide can be replaced with an amide containing backbone, in particular an aminoethylglycine backbone. The nucleotides can be retained and are bound directly or indirectly to aza nitrogen atoms of the amide portion of the backbone. The backbone in PNA compounds can comprise two or more linked aminoethylglycine units which gives PNA an amide containing backbone. The heterocyclic base moieties can be bound directly or indirectly to aza nitrogen atoms of the amide portion of the backbone.

A nucleic acid can comprise a morpholino backbone structure. For example, a nucleic acid can comprise a 6-membered morpholino ring in place of a ribose ring. In some of these embodiments, a phosphorodiamidate or other non-phosphodiester internucleoside linkage can replace a phosphodiester linkage.

A nucleic acid can comprise linked morpholino units (i.e., morpholino nucleic acid) having heterocyclic bases attached to the morpholino ring. Linking groups can link the morpholino monomeric units in a morpholino nucleic acid. Non-ionic morpholino-based oligomeric compounds can have less undesired interactions with cellular proteins. Morpholino-based polynucleotides can be nonionic mimics of nucleic acids. A variety of compounds within the morpholino class can be joined using different linking groups. A further class of polynucleotide mimetic can be referred to as cyclohexenyl nucleic acids (CeNA). The furanose ring normally present in a nucleic acid molecule can be replaced with a cyclohexenyl ring. CeNA DMT protected phosphoramidite monomers can be prepared and used for oligomeric compound synthesis using phosphoramidite chemistry. The incorporation of CeNA monomers into a nucleic acid chain can increase the stability of a DNA/RNA hybrid. CeNA oligoadenylates can form complexes with nucleic acid complements with similar stability to the native complexes. A further modification can include Locked Nucleic Acids (LNAs) in which the 2'-hydroxyl group is linked to the 4' carbon atom of the sugar ring thereby forming a 2'-C, 4'-C-oxymethylene linkage thereby forming a bicyclic sugar moiety. The linkage can be a methylene (-CH2-), group bridging the 2' oxygen atom and the 4' carbon atom wherein *n* is 1 or 2. LNA and LNA analogs can display very high duplex thermal stabilities with complementary nucleic acid (Tm=+3 to +10 °C), stability towards 3'-exonucleolytic degradation and good solubility properties.

A nucleic acid may also include nucleobase (often referred to simply as "base") modifications or substitutions. As used herein, "unmodified" or "natural" nucleobases can include the purine bases, (e.g., adenine (A) and guanine (G)), and the pyrimidine bases, (e.g., thymine (T), cytosine (C) and uracil (U)). Modified nucleobases can include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C=C-CH3) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-aminoadenine, 8-azaguanine and 8-azaadenine, 7-deazaguanine and 7-deazaadenine and 3-deazaguanine and 3-deazaadenine. Modified nucleobases can include tricyclic pyrimidines such as phenoxazine cytidine(1H-pyrimido(5,4-b)(1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g., 9-(2-aminoethoxy)-H-pyrimido(5,4-(b) (1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g., 9-(2-aminoethoxy)-H-pyrimido(5,4-(b) (1,4)benzoxazin-2(3H)-one), carbazole cytidine (2H-pyrimido(4,5-b)indol-2-one), pyridoindole cytidine (H-pyrido(3',2':4,5)pyrrolo[2,3-d]pyrimidin-2-one).

As used herein, the term "sample" can refer to a composition comprising targets. Suitable samples for analysis by the disclosed methods, devices, and systems include cells, tissues, organs, or organisms.

As used herein, the term "sampling device" or "device" can refer to a device which may take a section of a sample and/or place the section on a substrate. A sample device can refer to, for example, a fluorescence activated cell sorting (FACS) machine, a cell sorter machine, a biopsy needle, a biopsy device, a tissue sectioning device, a microfluidic device, a blade grid, and/or a microtome.

As used herein, the term "solid support" can refer to discrete solid or semi-solid surfaces to which a plurality of barcodes, such as stochastic barcodes, may be attached. A solid support may encompass any type of solid, porous, or hollow sphere, ball, bearing, cylinder, or other similar configuration composed of plastic, ceramic, metal, or polymeric material (e.g., hydrogel) onto which a nucleic acid may be immobilized (e.g., covalently or non-covalently). A solid support may comprise a discrete particle that may be spherical (e.g., microspheres) or have a non-spherical or irregular shape, such as cubic, cuboid, pyramidal, cylindrical, conical, oblong, or disc-shaped, and the like. A plurality of solid supports spaced in an array may not comprise a substrate. A solid support may be used interchangeably with the term "bead."

A solid support can refer to a "substrate." A substrate can be a type of solid support. A substrate can refer to a continuous solid or semi-solid surface on which the methods of the disclosure may be performed. A substrate can refer to an array, a cartridge, a chip, a device, and a slide, for example.

As used here, the term, "spatial label" can refer to a label which can be associated with a position in space.

As used herein, the term "barcode," such as "stochastic barcode," can refer to a polynucleotide sequence comprising labels. A barcode can be a polynucleotide sequence that can be used for barcoding (e.g., stochastic barcoding). Barcodes can be used to quantify targets within a sample. Barcodes can be used to control for errors which may occur after a label is associated with a target. For example, a barcode, such as a stochastic barcode, can be used to assess amplification or sequencing errors. A barcode associated with a target can be called a barcode-target, such as a stochastic barcode-target or stochastic barcode-tag-target.

As used herein, the term "gene-specific barcode," such as "gene-specific stochastic barcode," can refer to a polynucleotide sequence comprising labels and a target-binding region that is gene-specific. A barcode can be a polynucleotide sequence that can be used for barcoding (e.g., stochastic barcoding). Barcodes (e.g., stochastic barcodes) can be used to quantify targets within a sample. Barcodes can be used to control for errors which may occur after a label is associated with a target. For example, a barcode (e.g., a stochastic barcode) can be used to assess amplification or sequencing errors.

As used herein, the term "barcoding," such as "stochastic barcoding," can refer to the random labeling (e.g., barcoding) of nucleic acids. Stochastic barcoding can utilize a recursive Poisson strategy to associate and quantify labels associated with targets. As used herein, the term "barcoding" can be used interchangeably with "gene-specific barcoding," such as "gene-specific stochastic barcoding."

As used here, the term "target" can refer to a composition which can be associated with a barcode (e.g., a stochastic barcode). Exemplary suitable targets for analysis by the disclosed methods, devices, and systems include oligonucleotides, DNA, RNA, mRNA, microRNA, tRNA, and the like. Targets can be single or double stranded. In some embodiments, targets can be proteins, peptides, or polypeptides. In some embodiments, targets are lipids. As used herein, "target" can be used interchangeably with "species."

As used herein, the term "reverse transcriptases" can refer to a group of enzymes having reverse transcriptase activity (i.e., that catalyze synthesis of DNA from an RNA template). In general, such enzymes include, but are not limited to, retroviral reverse transcriptase, retrotransposon reverse transcriptase, retroplasmid reverse transcriptases, retron reverse transcriptases, bacterial reverse transcriptases, group II intron-derived reverse transcriptase, and mutants, variants or derivatives thereof. Non-retroviral reverse transcriptases include non-LTR retrotransposon reverse transcriptases, retroplasmid reverse transcriptases, retron reverse transciptases, and group II intron reverse transcriptases. Examples of group II intron reverse transcriptases include the *Lactococcus lactis* LI.LtrB intron reverse transcriptase, the *Thermosynechococcus* elongates TeI4c intron reverse transcriptase, or the *Geobacillus stearothermophilus* GsI-IIC intron reverse transcriptase. Other classes of reverse transcriptases can include many classes of non-retroviral reverse transcriptases (i.e., retrons, group II introns, and diversity-generating retroelements among others).

Disclosed herein are methods for identifying a multiplet expression profile. In some embodiments, the method comprises: (a) barcoding a plurality of targets in a plurality of cells using a plurality of barcodes to create a plurality of barcoded targets for cells of the plurality of cells, wherein each of the plurality of barcodes comprises a cell label and a molecular label, wherein molecular labels of at least two barcodes of the plurality of barcodes comprise different molecular label sequences, and wherein at least two barcodes of the plurality of barcodes comprise cell labels with an identical cell label sequence; (b) obtaining sequencing data of the plurality of barcoded targets; (c) determining a plurality of expression profiles associated with cell labels of the plurality of barcodes from the sequencing data obtained in (b), wherein an expression profile of the plurality of expression profiles associated with a cell label of the cell labels of the plurality of barcodes comprises a number of molecular labels with distinct sequences associated with the cell label and each target of the plurality of targets in the sequencing data; (d) generating a plurality of synthetic multiplet expression profiles from the plurality of expression profiles associated with the cell labels of the plurality of barcodes determined in (c); and (e) identifying an expression profile of the plurality of expression profiles associated with a cell label of the cell labels of the plurality of barcodes as a singlet or a multiplet based on expression profiles of the plurality of synthetic doublet expression profiles generated in (d).

### Barcodes

Barcoding, such as stochastic barcoding, has been described in, for example, US20150299784, WO2015031691, and Fu et al, Proc Natl Acad Sci U.S.A. 2011 May 31;108(22):9026-31. In some embodiments, the barcode disclosed herein can be a stochastic barcode which can be a polynucleotide sequence that may be used to stochastically label (e.g., barcode, tag) a target. Barcodes can be referred to stochastic barcodes if the ratio of the number of different barcode sequences of the stochastic barcodes and the number of occurrence of any of the targets to be labeled can be, or about, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, 100:1, or a number or a range between any two of these values. A target can be an mRNA species comprising mRNA molecules with identical or nearly identical sequences. Barcodes can be referred to as stochastic barcodes if the ratio of the number of different barcode sequences of the stochastic barcodes and the number of occurrence of any of the targets to be labeled is at least, or at most, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, or 100:1. Barcode sequences of stochastic barcodes can be referred to as molecular labels.

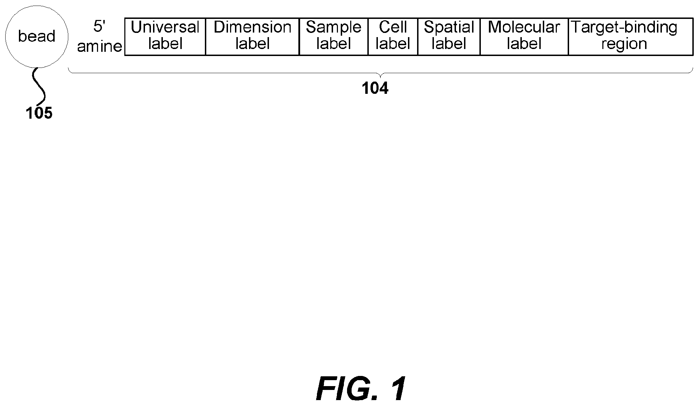

A barcode, for example a stochastic barcode, can comprise one or more labels. Exemplary labels can include a universal label, a cell label, a barcode sequence (e.g., a molecular label), a sample label, a plate label, a spatial label, and/or a pre-spatial label. FIG. 1 illustrates an exemplary barcode 104 with a spatial label. The barcode 104 can comprise a 5'amine that may link the barcode to a solid support 105. The barcode can comprise a universal label, a dimension label, a spatial label, a cell label, and/or a molecular label. The order of different labels (including but not limited to the universal label, the dimension label, the spatial label, the cell label, and the molecule label) in the barcode can vary. For example, as shown in FIG. 1, the universal label may be the 5'-most label, and the molecular label may be the 3'-most label. The spatial label, dimension label, and the cell label may be in any order. In some embodiments, the universal label, the spatial label, the dimension label, the cell label, and the molecular label are in any order. The barcode can comprise a target-binding region. The target-binding region can interact with a target (e.g., target nucleic acid, RNA, mRNA, DNA) in a sample. For example, a target-binding region can comprise an oligo(dT) sequence which can interact with poly(A) tails of mRNAs. In some instances, the labels of the barcode (e.g., universal label, dimension label, spatial label, cell label, and barcode sequence) may be separated by 1, 2, 3, 4, 5, 6, 7, 8, 9, 10, 11, 12, 13, 14, 15, 16, 17, 18, 19, or 20 or more nucleotides.

A label, for example the cell label, can comprise a unique set of nucleic acid sub-sequences of defined length, e.g., seven nucleotides each (equivalent to the number of bits used in some Hamming error correction codes), which can be designed to provide error correction capability. The set of error correction sub-sequences comprise seven nucleotide sequences can be designed such that any pairwise combination of sequences in the set exhibits a defined "genetic distance" (or number of mismatched bases), for example, a set of error correction sub-sequences can be designed to exhibit a genetic distance of three nucleotides. In this case, review of the error correction sequences in the set of sequence data for labeled target nucleic acid molecules (described more fully below) can allow one to detect or correct amplification or sequencing errors. In some embodiments, the length of the nucleic acid sub-sequences used for creating error correction codes can vary, for example, they can be, or be about 1, 2, 3, 4, 5, 6, 7, 8, 9, 10, 15, 20, 30, 31, 40, 50, or a number or a range between any two of these values, nucleotides in length. In some embodiments, nucleic acid sub-sequences of other lengths can be used for creating error correction codes.

The barcode can comprise a target-binding region. The target-binding region can interact with a target in a sample. The target can be, or comprise, ribonucleic acids (RNAs), messenger RNAs (mRNAs), microRNAs, small interfering RNAs (siRNAs), RNA degradation products, RNAs each comprising a poly(A) tail, and any combination thereof. In some embodiments, the plurality of targets can include deoxyribonucleic acids (DNAs).

In some embodiments, a target-binding region can comprise an oligo(dT) sequence which can interact with poly(A) tails of mRNAs. One or more of the labels of the stochastic barcode (e.g., the universal label, the dimension label, the spatial label, the cell label, and the barcode sequences (e.g., molecular label)) can be separated by a spacer from another one or two of the remaining labels of the stochastic barcode. The spacer can be, for example, 1, 2, 3, 4, 5, 6, 7, 8, 9, 10, 11, 12, 13, 14, 15, 16, 17, 18, 19, or 20 or more nucleotides. In some embodiments, none of the labels of the stochastic barcode is separated by spacer.

### Universal Labels

A barcode (e.g., a stochastic barcode) can comprise one or more universal labels or sequences. In some embodiments, the one or more universal labels can be the same for all stochastic barcodes in the set of stochastic barcodes attached to a given solid support. In some embodiments, the one or more universal labels can be the same for all stochastic barcodes attached to a plurality of beads. In some embodiments, a universal label can comprise a nucleic acid sequence that is capable of hybridizing to a sequencing primer. Sequencing primers can be used for sequencing stochastic barcodes comprising a universal label. Sequencing primers (e.g., universal sequencing primers) can comprise sequencing primers associated with high-throughput sequencing platforms. In some embodiments, a universal label can comprise a nucleic acid sequence that is capable of hybridizing to a PCR primer. In some embodiments, the universal label can comprise a nucleic acid sequence that is capable of hybridizing to a sequencing primer and a PCR primer. The nucleic acid sequence of the universal label that is capable of hybridizing to a sequencing or PCR primer can be referred to as a primer binding site. A universal label can comprise a sequence that can be used to initiate transcription of the stochastic barcode. A universal label can comprise a sequence that can be used for extension of the stochastic barcode or a region within the stochastic barcode. A universal label can be, or be about, 1, 2, 3, 4, 5, 10, 15, 20, 25, 30, 35, 40, 45, 50, or a number or a range between any two of these values, nucleotides in length. For example, a universal label can comprise at least about 10 nucleotides. A universal label can be at least, or at most, 1, 2, 3, 4, 5, 10, 15, 20, 25, 30, 35, 40, 45, 50, 100, 200, or 300 nucleotides in length. In some embodiments, a cleavable linker or modified nucleotide can be part of the universal label sequence to enable the stochastic barcode to be cleaved off from the support.

### Dimension Labels

A barcode (e.g., a stochastic barcode) can comprise one or more dimension labels. In some embodiments, a dimension label can comprise a nucleic acid sequence that provides information about a dimension in which the stochastic labeling occurred. For example, a dimension label can provide information about the time at which a target was stochastically barcoded. A dimension label can be associated with a time of stochastic barcoding in a sample. A dimension label can be activated at the time of stochastic labeling. Different dimension labels can be activated at different times. The dimension label provides information about the order in which targets, groups of targets, and/or samples were stochastically barcoded. For example, a population of cells can be stochastically barcoded at the G0 phase of the cell cycle. The cells can be pulsed again with stochastic barcodes at the G1 phase of the cell cycle. The cells can be pulsed again with stochastic barcodes at the S phase of the cell cycle, and so on. Stochastic barcodes at each pulse (e.g., each phase of the cell cycle), can comprise different dimension labels. In this way, the dimension label provides information about which targets were labelled at which phase of the cell cycle. Dimension labels can interrogate many different biological times. Exemplary biological times can include, but are not limited to, the cell cycle, transcription (e.g., transcription initiation), and transcript degradation. In another example, a sample (e.g., a cell, a population of cells) can be stochastically labeled before and/or after treatment with a drug and/or therapy. The changes in the number of copies of distinct targets can be indicative of the sample's response to the drug and/or therapy.

A dimension label can be activatable. An activatable dimension label can be activated at a specific time point. The activatable label can be, for example, constitutively activated (e.g., not turned off). The activatable dimension label can be, for example, reversibly activated (e.g., the activatable dimension label can be turned on and turned off). The dimension label can be, for example, reversibly activatable at least 1, 2, 3, 4, 5, 6, 7, 8, 9, or 10 or more times. The dimension label can be reversibly activatable, for example, at least 1, 2, 3, 4, 5, 6, 7, 8, 9, or 10 or more times. In some embodiments, the dimension label can be activated with fluorescence, light, a chemical event (e.g., cleavage, ligation of another molecule, addition of modifications (e.g., pegylated, sumoylated, acetylated, methylated, deacetylated, demethylated), a photochemical event (e.g., photocaging), and introduction of a non-natural nucleotide.

The dimension label can, in some embodiments, be identical for all stochastic barcodes attached to a given solid support (e.g., bead), but different for different solid supports (e.g., beads). In some embodiments, at least 60%, 70%, 80%, 85%, 90%, 95%, 97%, 99% or 100% of stochastic barcodes on the same solid support can comprise the same dimension label. In some embodiments, at least 60% of stochastic barcodes on the same solid support can comprise the same dimension label. In some embodiments, at least 95% of stochastic barcodes on the same solid support can comprise the same dimension label.

There can be as many as 10⁶ or more unique dimension label sequences represented in a plurality of solid supports (e.g., beads). A dimension label can be, or be about 1, 2, 3, 4, 5, 10, 15, 20, 25, 30, 35, 40, 45, 50, or a number or a range between any two of these values, nucleotides in length. A dimension label can be at least, or at most, 1, 2, 3, 4, 5, 10, 15, 20, 25, 30, 35, 40, 45, 50, 100, 200, or 300 nucleotides in length. A dimension label can comprise between about 5 to about 200 nucleotides. A dimension label can comprise between about 10 to about 150 nucleotides. A dimension label can comprise between about 20 to about 125 nucleotides in length.

### Spatial Labels

A barcode (e.g., a stochastic barcode) can comprise one or more spatial labels. In some embodiments, a spatial label can comprise a nucleic acid sequence that provides information about the spatial orientation of a target molecule which is associated with the stochastic barcode. A spatial label can be associated with a coordinate in a sample. The coordinate can be a fixed coordinate. For example, a coordinate can be fixed in reference to a substrate. A spatial label can be in reference to a two or three-dimensional grid. A coordinate can be fixed in reference to a landmark. The landmark can be identifiable in space. A landmark can be a structure which can be imaged. A landmark can be a biological structure, for example an anatomical landmark. A landmark can be a cellular landmark, for instance an organelle. A landmark can be a non-natural landmark such as a structure with an identifiable identifier such as a color code, bar code, magnetic property, fluorescents, radioactivity, or a unique size or shape. A spatial label can be associated with a physical partition (e.g., a well, a container, or a droplet). In some embodiments, multiple spatial labels are used together to encode one or more positions in space.

The spatial label can be identical for all stochastic barcodes attached to a given solid support (e.g., bead), but different for different solid supports (e.g., beads). In some embodiments, the percentage of stochastic barcodes on the same solid support comprising the same spatial label can be, or be about, 60%, 70%, 80%, 85%, 90%, 95%, 97%, 99%, 100%, or a number or a range between any two of these values. In some embodiments, the percentage of stochastic barcodes on the same solid support comprising the same spatial label can be at least, or at most, 60%, 70%, 80%, 85%, 90%, 95%, 97%, 99%, or 100%. In some embodiments, at least 60% of stochastic barcodes on the same solid support can comprise the same spatial label. In some embodiments, at least 95% of stochastic barcodes on the same solid support can comprise the same spatial label.

There can be as many as 10⁶ or more unique spatial label sequences represented in a plurality of solid supports (e.g., beads). A spatial label can be, or be about, 1, 2, 3, 4, 5, 10, 15, 20, 25, 30, 35, 40, 45, 50, or a number or a range between any two of these values, nucleotides in length. A spatial label can be at least or at most 1, 2, 3, 4, 5, 10, 15, 20, 25, 30, 35, 40, 45, 50, 100, 200, or 300 nucleotides in length. A spatial label can comprise between about 5 to about 200 nucleotides. A spatial label can comprise between about 10 to about 150 nucleotides. A spatial label can comprise between about 20 to about 125 nucleotides in length.

### Cell Labels

A barcode (e.g., a stochastic barcode) can comprise one or more cell labels. In some embodiments, a cell label can comprise a nucleic acid sequence that provides information for determining which target nucleic acid originated from which cell. In some embodiments, the cell label is identical for all stochastic barcodes attached to a given solid support (e.g., bead), but different for different solid supports (e.g., beads). In some embodiments, the percentage of stochastic barcodes on the same solid support comprising the same cell label can be, or be about 60%, 70%, 80%, 85%, 90%, 95%, 97%, 99%, 100%, or a number or a range between any two of these values. In some embodiments, the percentage of stochastic barcodes on the same solid support comprising the same cell label can be at least, or be at most 60%, 70%, 80%, 85%, 90%, 95%, 97%, 99%, or 100%. For example, at least 60% of stochastic barcodes on the same solid support can comprise the same cell label. As another example, at least 95% of stochastic barcodes on the same solid support can comprise the same cell label.

There can be as many as 10⁶ or more unique cell label sequences represented in a plurality of solid supports (e.g., beads). A cell label can be, or be about, 1, 2, 3, 4, 5, 10, 15, 20, 25, 30, 35, 40, 45, 50, or a number or a range between any two of these values, nucleotides in length. A cell label can be at least, or at most, 1, 2, 3, 4, 5, 10, 15, 20, 25, 30, 35, 40, 45, 50, 100, 200, or 300 nucleotides in length. For example, a cell label can comprise between about 5 to about 200 nucleotides. As another example, a cell label can comprise between about 10 to about 150 nucleotides. As yet another example, a cell label can comprise between about 20 to about 125 nucleotides in length.

### Barcode Sequences

A barcode can comprise one or more barcode sequences. In some embodiments, a barcode sequence can comprise a nucleic acid sequence that provides identifying information for the specific type of target nucleic acid species hybridized to the barcode. A barcode sequence can comprise a nucleic acid sequence that provides a counter (e.g., that provides a rough approximation) for the specific occurrence of the target nucleic acid species hybridized to the barcode (e.g., target-binding region).

In some embodiments, a diverse set of barcode sequences are attached to a given solid support (e.g., bead). In some embodiments, there can be, or be about, 10², 10³, 10⁴, 10⁵, 10⁶, 10⁷, 10⁸, 10⁹, or a number or a range between any two of these values, unique molecular label sequences. For example, a plurality of barcodes can comprise about 6561 barcodes sequences with distinct sequences. As another example, a plurality of barcodes can comprise about 65536 barcode sequences with distinct sequences. In some embodiments, there can be at least, or at most, 10², 10³, 10⁴, 10⁵, 10⁶, 10⁷, 10⁸, or 10⁹, unique barcode sequences. The unique molecular label sequences can be attached to a given solid support (e.g., bead).

A barcode can be, or be about, 1, 2, 3, 4, 5, 10, 15, 20, 25, 30, 35, 40, 45, 50, or a number or a range between any two of these values, nucleotides in length. A barcode can be at least, or at most, 1, 2, 3, 4, 5, 10, 15, 20, 25, 30, 35, 40, 45, 50, 100, 200, or 300 nucleotides in length.

### Molecular Labels

A barcode (e.g., a stochastic barcode) can comprise one or more molecular labels. In some embodiments, a molecular label can comprise a nucleic acid sequence that provides identifying information for the specific type of target nucleic acid species hybridized to the stochastic barcode. A molecular label can comprise a nucleic acid sequence that provides a counter for the specific occurrence of the target nucleic acid species hybridized to the stochastic barcode (e.g., target-binding region).

In some embodiments, a diverse set of molecular labels are attached to a given solid support (e.g., bead). In some embodiments, there can be, or be about, 10², 10³, 10⁴, 10⁵, 10⁶, 10⁷, 10⁸, 10⁹, or a number or a range between any two of these values, of unique molecular label sequences. For example, a plurality of stochastic barcodes can comprise about 6561 molecular labels with distinct sequences. As another example, a plurality of stochastic barcodes can comprise about 65536 molecular labels with distinct sequences. In some embodiments, there can be at least, or at most, 10², 10³, 10⁴, 10⁵, 10⁶, 10⁷, 10⁸, or 10⁹, unique molecular label sequences. The unique molecular label sequences attached to a given solid support (e.g., bead).

For stochastic barcoding using a plurality of stochastic barcodes, the ratio of the number of different molecular label sequences and the number of occurrence of any of the targets can be, or about, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, 100:1, or a number or a range between any two of these values. A target can be an mRNA species comprising mRNA molecules with identical or nearly identical sequences. In some embodiments, the ratio of the number of different molecular label sequences and the number of occurrence of any of the targets is at least, or at most, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, or 100:1.

A molecular label can be, or be about, 1, 2, 3, 4, 5, 10, 15, 20, 25, 30, 35, 40, 45, 50, or a number or a range between any two of these values, nucleotides in length. A molecular label can be at least, or at most, 1, 2, 3, 4, 5, 10, 15, 20, 25, 30, 35, 40, 45, 50, 100, 200, or 300 nucleotides in length.

### Target-Binding Region

A barcode (e.g., a stochastic barcode) can comprise one or more target-binding regions. In some embodiments, a target-binding region can hybridize with a target of interest. In some embodiments, the target-binding regions can comprise a nucleic acid sequence that hybridizes specifically to a target (e.g., target nucleic acid, target molecule, e.g., a cellular nucleic acid to be analyzed), for example to a specific gene sequence. In some embodiments, a target-binding region can comprise a nucleic acid sequence that can attach (e.g., hybridize) to a specific location of a specific target nucleic acid. In some embodiments, the target-binding region can comprise a nucleic acid sequence that is capable of specific hybridization to a restriction enzyme site overhang (e.g., an EcoRI sticky-end overhang). The stochastic barcode can then ligate to any nucleic acid molecule comprising a sequence complementary to the restriction site overhang.

In some embodiments, a target-binding region can comprise a non-specific target nucleic acid sequence. A non-specific target nucleic acid sequence can refer to a sequence that can bind to multiple target nucleic acids, independent of the specific sequence of the target nucleic acid. For example, target-binding region can comprise a random multimer sequence, or an oligo(dT) sequence that hybridizes to the poly(A) tail on mRNA molecules. A random multimer sequence can be, for example, a random dimer, trimer, quatramer, pentamer, hexamer, septamer, octamer, nonamer, decamer, or higher multimer sequence of any length. In some embodiments, the target-binding region is the same for all stochastic barcodes attached to a given bead. In some embodiments, the target-binding regions for the plurality of stochastic barcodes attached to a given bead can comprise two or more different target binding sequences. A target-binding region can be, or be about, 5, 10, 15, 20, 25, 30, 35, 40, 45, 50, or a number or a range between any two of these values, nucleotides in length. A target-binding region can be at most about 5, 10, 15, 20, 25, 30, 35, 40, 45, 50 or more nucleotides in length.

In some embodiments, a target-binding region can comprise an oligo(dT) which can hybridize with mRNAs comprising poly-adenylated ends. A target-binding region can be gene-specific. For example, a target-binding region can be configured to hybridize to a specific region of a target. A target-binding region can be, or be about, 1, 2, 3, 4, 5, 6, 7, 8, 9, 10, 11, 12, 13, 14, 15, 16, 17, 18, 19, 20, 21, 22, 23, 24, 25, 26 27, 28, 29, 30, or a number or a range between any two of these values, nucleotides in length. A target-binding region can be at least, or at most, 1, 2, 3, 4, 5, 6, 7, 8, 9, 10, 11, 12, 13, 14, 15, 16, 17, 18, 19, 20, 21, 22, 23, 24, 25, 26 27, 28, 29, or 30, nucleotides in length. A target-binding region can be about 5-30 nucleotides in length. When a stochastic barcode comprises a gene-specific target-binding region, the stochastic barcode can be referred to as a gene-specific stochastic barcode.

### Orientation Property

A barcode (e.g., a stochastic barcode) can comprise one or more orientation properties which can be used to orient (e.g., align) the stochastic barcodes. A stochastic barcode can comprise a moiety for isoelectric focusing. Different stochastic barcodes can comprise different isoelectric focusing points. When these stochastic barcodes are introduced to a sample, the sample can undergo isoelectric focusing in order to orient the stochastic barcodes into a known way. In this way, the orientation property can be used to develop a known map of stochastic barcodes in a sample. Exemplary orientation properties can include, electrophoretic mobility (e.g., based on size of the stochastic barcode), isoelectric point, spin, conductivity, and/or self-assembly. For example, stochastic barcodes with an orientation property of self-assembly, can self-assemble into a specific orientation (e.g., nucleic acid nanostructure) upon activation.

### Affinity Property

A barcode (e.g., a stochastic barcode) can comprise one or more affinity properties. For example, a spatial label can comprise an affinity property. An affinity property can include a chemical and/or biological moiety that can facilitate binding of the stochastic barcode to another entity (e.g., cell receptor). For example, an affinity property can comprise an antibody, for example, an antibody specific for a specific moiety (e.g., receptor) on a sample. In some embodiments, the antibody can guide the stochastic barcode to a specific cell type or molecule. Targets at and/or near the specific cell type or molecule can be stochastically labeled. The affinity property can, in some embodiments, provide spatial information in addition to the nucleotide sequence of the spatial label because the antibody can guide the stochastic barcode to a specific location. The antibody can be a therapeutic antibody, for example a monoclonal antibody or a polyclonal antibody. The antibody can be humanized or chimeric. The antibody can be a naked antibody or a fusion antibody.

The antibody can be a full-length (i.e., naturally occurring or formed by normal immunoglobulin gene fragment recombinatorial processes) immunoglobulin molecule (e.g., an IgG antibody) or an immunologically active (i.e., specifically binding) portion of an immunoglobulin molecule, like an antibody fragment.

The antibody fragment can be, for example, a portion of an antibody such as F(ab')2, Fab', Fab, Fv, sFv and the like. In some embodiments, the antibody fragment can bind with the same antigen that is recognized by the full-length antibody. The antibody fragment can include isolated fragments consisting of the variable regions of antibodies, such as the "Fv" fragments consisting of the variable regions of the heavy and light chains and recombinant single chain polypeptide molecules in which light and heavy variable regions are connected by a peptide linker ("scFv proteins"). Exemplary antibodies can include, but are not limited to, antibodies for cancer cells, antibodies for viruses, antibodies that bind to cell surface receptors (CD8, CD34, CD45), and therapeutic antibodies.

### Universal Adaptor Primer

A barcode can comprise one or more universal adaptor primers. For example, a gene-specific barcode, such as a gene-specific stochastic barcode, can comprise a universal adaptor primer. A universal adaptor primer can refer to a nucleotide sequence that is universal across all barcodes. A universal adaptor primer can be used for building gene-specific barcodes. A universal adaptor primer can be, or be about, 1, 2, 3, 4, 5, 6, 7, 8, 9, 10, 11, 12, 13, 14, 15, 16, 17, 18, 19, 20, 21, 22, 23, 24, 25, 26 27, 28, 29, 30, or a number or a range between any two of these nucleotides in length. A universal adaptor primer can be at least, or at most, 1, 2, 3, 4, 5, 6, 7, 8, 9, 10, 11, 12, 13, 14, 15, 16, 17, 18, 19, 20, 21, 22, 23, 24, 25, 26 27, 28, 29, or 30 nucleotides in length. A universal adaptor primer can be from 5-30 nucleotides in length.

### Linker

When a barcode comprises more than one of a type of label (e.g., more than one cell label or more than one barcode sequence, such as one molecular label), the labels may be interspersed with a linker label sequence. A linker label sequence can be at least about 5, 10, 15, 20, 25, 30, 35, 40, 45, 50 or more nucleotides in length. A linker label sequence can be at most about 5, 10, 15, 20, 25, 30, 35, 40, 45, 50 or more nucleotides in length. In some instances, a linker label sequence is 12 nucleotides in length. A linker label sequence can be used to facilitate the synthesis of the barcode. The linker label can comprise an error-correcting (e.g., Hamming) code.

### Solid Supports

Barcodes (e.g., stochastic barcodes) disclosed herein can, in some embodiments, be associated with a solid support. The solid support can be, for example, a synthetic particle. In some embodiments, some or all of the molecular labels (e.g., the first molecular labels) of a plurality of stochastic barcodes (e.g., the first plurality of stochastic barcodes) on a solid support differ by at least one nucleotide. The cell labels of the stochastic barcodes on the same solid support can be the same. The cell labels of the stochastic barcodes on different solid supports can differ by at least one nucleotide. For example, first cell labels of a first plurality of stochastic barcodes on a first solid support can have the same sequence, and second cell labels of a second plurality of stochastic barcodes on a second solid support can have the same sequence. The first cell labels of the first plurality of stochastic barcodes on the first solid support and the second cell labels of the second plurality of stochastic barcodes on the second solid support can differ by at least one nucleotide. A cell label can be, for example, about 5-20 nucleotides long. A molecular label can be, for example, about 5-20 nucleotides long. The synthetic particle can be, for example, a bead.

The bead can be, for example, a silica gel bead, a controlled pore glass bead, a magnetic bead, a Dynabead, a Sephadex/Sepharose bead, a cellulose bead, a polystyrene bead, or any combination thereof. The bead can comprise a material such as polydimethylsiloxane (PDMS), polystyrene, glass, polypropylene, agarose, gelatin, hydrogel, paramagnetic, ceramic, plastic, glass, methylstyrene, acrylic polymer, titanium, latex, sepharose, cellulose, nylon, silicone, or any combination thereof.

In some embodiments, the bead can be a polymeric bead, for example a deformable bead or a gel bead, functionalized with stochastic barcodes (such as the gel beads from 10X Genomics (San Francisco, CA)). In some implementation, a gel bead can comprise a polymer based gels. Gel beads can be generated, for example, by encapsulating one or more polymeric precursors into droplets. Upon exposure of the polymeric precursors to an accelerator (e.g., tetramethylethylenediamine (TEMED)), a gel bead may be generated.

In some embodiments, the polymeric bead can dissolve, melt, or degrade, for example, under a desired condition. The desired condition can include an environmental condition. The desired condition may result in the polymeric bead dissolving, melting, or degrading in a controlled manner. A gel bead may dissolve, melt, or degrade due to a chemical stimulus, a physical stimulus, a biological stimulus, a thermal stimulus, a magnetic stimulus, an electric stimulus, a light stimulus, or any combination thereof.

Analytes and/or reagents, such as oligonucleotide barcodes, for example, may be coupled/immobilized to the interior surface of a gel bead (e.g., the interior accessible via diffusion of an oligonucleotide barcode and/or materials used to generate an oligonucleotide barcode) and/or the outer surface of a gel bead or any other microcapsule described herein. Coupling/immobilization may be via any form of chemical bonding (e.g., covalent bond, ionic bond) or physical phenomena (e.g., Van der Waals forces, dipole-dipole interactions, etc.). In some cases, coupling/immobilization of a reagent to a gel bead or any other microcapsule described herein may be reversible, such as, for example, via a labile moiety (e.g., via a chemical cross-linker, including chemical cross-linkers described herein). Upon application of a stimulus, the labile moiety may be cleaved and the immobilized reagent set free. In some cases, the labile moiety is a disulfide bond. For example, in the case where an oligonucleotide barcode is immobilized to a gel bead via a disulfide bond, exposure of the disulfide bond to a reducing agent can cleave the disulfide bond and free the oligonucleotide barcode from the bead. The labile moiety may be included as part of a gel bead or microcapsule, as part of a chemical linker that links a reagent or analyte to a gel bead or microcapsule, and/or as part of a reagent or analyte.

In some embodiments, a gel bead can comprise a wide range of different polymers including but not limited to: polymers, heat sensitive polymers, photosensitive polymers, magnetic polymers, pH sensitive polymers, salt-sensitive polymers, chemically sensitive polymers, polyelectrolytes, polysaccharides, peptides, proteins, and/or plastics. Polymers may include but are not limited to materials such as poly(N-isopropylacrylamide) (PNIPAAm), poly(styrene sulfonate) (PSS), poly(allyl amine) (PAAm), poly(acrylic acid) (PAA), poly(ethylene imine) (PEI), poly(diallyldimethyl-ammonium chloride) (PDADMAC), poly(pyrolle) (PPy), poly(vinylpyrrolidone) (PVPON), poly(vinyl pyridine) (PVP), poly(methacrylic acid) (PMAA), poly(methyl methacrylate) (PMMA), polystyrene (PS), poly(tetrahydrofuran) (PTHF), poly(phthaladehyde) (PTHF), poly(hexyl viologen) (PHV), poly(L-lysine) (PLL), poly(L-arginine) (PARG), and poly(lactic-co-glycolic acid) (PLGA).

Numerous chemical stimuli can be used to trigger the disruption or degradation of the beads. Examples of these chemical changes may include, but are not limited to pH-mediated changes to the bead wall, disintegration of the bead wall via chemical cleavage of crosslink bonds, triggered depolymerization of the bead wall, and bead wall switching reactions. Bulk changes may also be used to trigger disruption of the beads.

Bulk or physical changes to the microcapsule through various stimuli also offer many advantages in designing capsules to release reagents. Bulk or physical changes occur on a macroscopic scale, in which bead rupture is the result of mechano-physical forces induced by a stimulus. These processes may include, but are not limited to pressure induced rupture, bead wall melting, or changes in the porosity of the bead wall.

Biological stimuli may also be used to trigger disruption or degradation of beads. Generally, biological triggers resemble chemical triggers, but many examples use biomolecules, or molecules commonly found in living systems such as enzymes, peptides, saccharides, fatty acids, nucleic acids and the like. For example, beads may comprise polymers with peptide cross-links that are sensitive to cleavage by specific proteases. More specifically, one example may comprise a microcapsule comprising GFLGK peptide cross links. Upon addition of a biological trigger such as the protease Cathepsin B, the peptide cross links of the shell well are cleaved and the contents of the beads are released. In other cases, the proteases may be heat-activated. In another example, beads comprise a shell wall comprising cellulose. Addition of the hydrolytic enzyme chitosan serves as biologic trigger for cleavage of cellulosic bonds, depolymerization of the shell wall, and release of its inner contents.

The beads may also be induced to release their contents upon the application of a thermal stimulus. A change in temperature can cause a variety changes to the beads. A change in heat may cause melting of a bead such that the bead wall disintegrates. In other cases, the heat may increase the internal pressure of the inner components of the bead such that the bead ruptures or explodes. In still other cases, the heat may transform the bead into a shrunken dehydrated state. The heat may also act upon heat-sensitive polymers within the wall of a bead to cause disruption of the bead.

Inclusion of magnetic nanoparticles to the bead wall of microcapsules may allow triggered rupture of the beads as well as guide the beads in an array. A device of this disclosure may comprise magnetic beads for either purpose. In one example, incorporation of Fe₃O₄ nanoparticles into polyelectrolyte containing beads triggers rupture in the presence of an oscillating magnetic field stimulus.

A bead may also be disrupted or degraded as the result of electrical stimulation. Similar to magnetic particles described in the previous section, electrically sensitive beads can allow for both triggered rupture of the beads as well as other functions such as alignment in an electric field, electrical conductivity or redox reactions. In one example, beads containing electrically sensitive material are aligned in an electric field such that release of inner reagents can be controlled. In other examples, electrical fields may induce redox reactions within the bead wall itself that may increase porosity.

A light stimulus may also be used to disrupt the beads. Numerous light triggers are possible and may include systems that use various molecules such as nanoparticles and chromophores capable of absorbing photons of specific ranges of wavelengths. For example, metal oxide coatings can be used as capsule triggers. UV irradiation of polyelectrolyte capsules coated with SiO₂ may result in disintegration of the bead wall. In yet another example, photo switchable materials such as azobenzene groups may be incorporated in the bead wall. Upon the application of UV or visible light, chemicals such as these undergo a reversible cis-to-trans isomerization upon absorption of photons. In this aspect, incorporation of photon switches result in a bead wall that may disintegrate or become more porous upon the application of a light trigger.

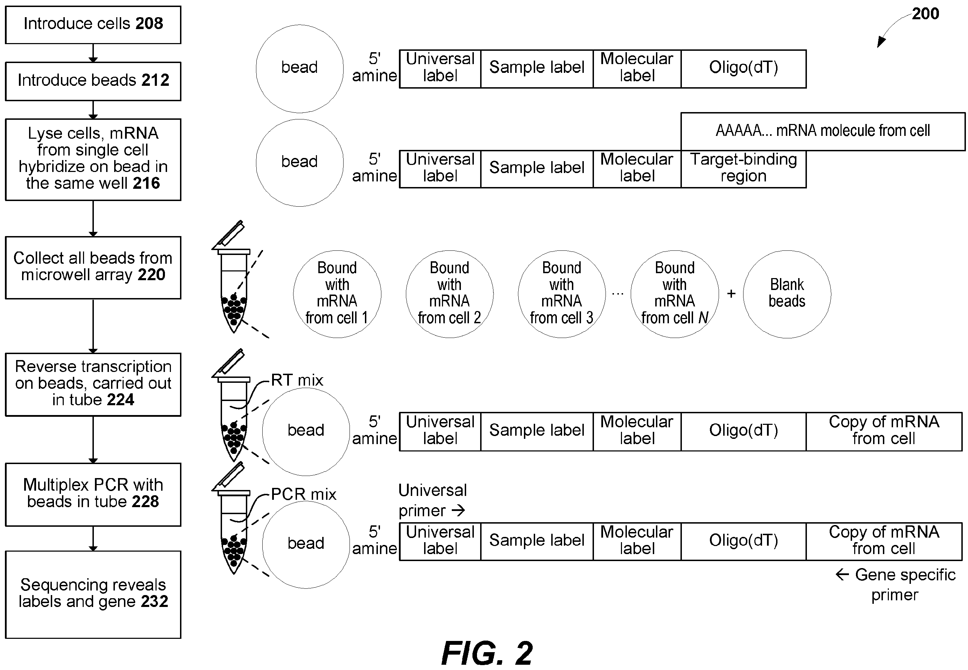

For example, in a non-limiting example 200 of stochastic barcoding illustrated in FIG. 2, after introducing cells such as single cells onto a plurality of microwells of a microwell array at 208, beads can be introduced onto the plurality of microwells of the microwell array at 212. Each microwell can comprise one bead. The beads can comprise a plurality of stochastic barcodes. A stochastic barcode can comprise a 5' amine region attached to a bead. The stochastic barcode can comprise a universal label, a molecular label, a target-binding region, or any combination thereof.

The stochastic barcodes disclosed herein can be associated with (e.g., attached to) a solid support (e.g., a bead). The stochastic barcodes associated with a solid support can each comprise a molecular label selected from a group comprising at least 100 or 1000 molecular labels with unique sequences. In some embodiments, different stochastic barcodes associated with a solid support can comprise molecular labels of different sequences. In some embodiments, a percentage of stochastic barcodes associated with a solid support comprises the same cell label. For example, the percentage can be, or be about 60%, 70%, 80%, 85%, 90%, 95%, 97%, 99%, 100%, or a number or a range between any two of these values. As another example, the percentage can be at least, or at most 60%, 70%, 80%, 85%, 90%, 95%, 97%, 99%, or 100%. In some embodiments, stochastic barcodes associated with a solid support can have the same cell label. The stochastic barcodes associated with different solid supports can have different cell labels selected from a group comprising at least 100 or 1000 cell labels with unique sequences.

In some embodiments, stochastically barcoding the plurality of targets in the sample can be performed with a solid support including a plurality of synthetic particles associated with the plurality of stochastic barcodes. In some embodiments, the solid support can include a plurality of synthetic particles associated with the plurality of stochastic barcodes. The spatial labels of the plurality of stochastic barcodes on different solid supports can differ by at least one nucleotide. The solid support can, for example, include the plurality of stochastic barcodes in two dimensions or three dimensions. The synthetic particles can be beads. The beads can be silica gel beads, controlled pore glass beads, magnetic beads, Dynabeads, Sephadex/Sepharose beads, cellulose beads, polystyrene beads, or any combination thereof. The solid support can include a polymer, a matrix, a hydrogel, a needle array device, an antibody, or any combination thereof. In some embodiments, the solid supports can be free floating. In some embodiments, the solid supports can be embedded in a semi-solid or solid array. The stochastic barcodes may not be associated with solid supports. The stochastic barcodes can be individual nucleotides. The stochastic barcodes can be associated with a substrate.

As used herein, the terms "tethered", "attached", and "immobilized" are used interchangeably, and can refer to covalent or non-covalent means for attaching stochastic barcodes to a solid support. Any of a variety of different solid supports can be used as solid supports for attaching pre-synthesized stochastic barcodes or for *in situ* solid-phase synthesis of stochastic barcode.

In some embodiments, the solid support is a bead. The bead can comprise one or more types of solid, porous, or hollow sphere, ball, bearing, cylinder, or other similar configuration which a nucleic acid can be immobilized (e.g., covalently or non-covalently). The bead can be, for example, composed of plastic, ceramic, metal, polymeric material, or any combination thereof. A bead can be, or comprise, a discrete particle that is spherical (e.g., microspheres) or have a non-spherical or irregular shape, such as cubic, cuboid, pyramidal, cylindrical, conical, oblong, or disc-shaped, and the like. In some embodiments, a bead can be non-spherical in shape.

Beads can comprise a variety of materials including, but not limited to, paramagnetic materials (e.g., magnesium, molybdenum, lithium, and tantalum), superparamagnetic materials (e.g., ferrite (Fe₃O₄; magnetite) nanoparticles), ferromagnetic materials (e.g., iron, nickel, cobalt, some alloys thereof, and some rare earth metal compounds), ceramic, plastic, glass, polystyrene, silica, methylstyrene, acrylic polymers, titanium, latex, sepharose, agarose, hydrogel, polymer, cellulose, nylon, and any combination thereof. In some embodiments, the bead (e.g., the bead to which the stochastic labels are attached) is a hydrogel bead. In some embodiments, the bead comprises hydrogel.

Some embodiments disclosed herein include one or more particles (for example beads). Each of the particles can comprise a plurality of oligonucleotides (e.g., stochastic barcodes). Each of the plurality of oligonucleotides can comprise a molecular label sequence, a cell label sequence, and a target-binding region (e.g., an oligo dT sequence, a gene-specific sequence, a random multimer, or a combination thereof). The cell label sequence of each of the plurality of oligonucleotides can be the same. The cell label sequences of oligonucleotides on different particles can be different such that the oligonucleotides on different particles can be identified. The number of different cell label sequences can be different in different implementations. In some embodiments, the number of cell label sequences can be, or about 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, 10⁹, a number or a range between any two of these values, or more. In some embodiments, the number of cell label sequences can be at least, or at most 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, or 10⁹. In some embodiments, no more than 1, 2, 3, 4, 5, 6, 7, 8, 9, 10, 20, 30, 40, 50, 60, 70, 80, 90, 100, 200, 300, 400, 500, 600, 700, 800, 900, 1000, or more of the plurality of the particles include oligonucleotides with the same cell sequence. In some embodiment, the plurality of particles that include oligonucleotides with the same cell sequence can be at most 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10% or more. In some embodiments, none of the plurality of the particles has the same cell label sequence.

The plurality of oligonucleotides on each particle can comprise different molecular label sequences. In some embodiments, the number of molecular label sequences can be, or about 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, 10⁹, or a number or a range between any two of these values. In some embodiments, the number of molecular label sequences can be at least, or at most 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, or 10⁹. For example, at least 100 of the plurality of oligonucleotides comprise different molecular label sequences. As another example example, in a single particle, at least 100, 500, 1000, 5000, 10000, 15000, 20000, 50000, a number or a range between any two of these values, or more of the plurality of oligonucleotides comprise different molecular label sequences. Some embodiments provide a plurality of the particles comprising stochastic barcodes. In some embodiments, the ratio of an occurrence (or a copy or a number) of a target to be labeled and the different molecular label sequences can be at least 1:1, 1:2, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, 1:30, 1:40, 1:50, 1:60, 1:70, 1:80, 1:90, or more. In some embodiments, each of the plurality of oligonucleotides further comprises a sample label, a universal label, or both. The particle can be, for example, a nanoparticle or microparticle.

The size of the beads can vary. For example, the diameter of the bead can range from 0.1 micrometer to 50 micrometer. In some embodiments, the diameters of beads can be, or be about, 0.1, 0.5, 1, 2, 3, 4, 5, 6, 7, 8, 9, 10, 20, 30, 40, 50 micrometer, or a number or a range between any two of these values.

The diameters of the bead can be related to the diameter of the wells of the substrate. In some embodiments, the diameters of the bead can be, or be about, 10%, 20%, 30%, 40%, 50%, 60%, 70%, 80%, 90%, 100%, or a number or a range between any two of these values, longer or shorter than the diameter of the well. The diameter of the beads can be related to the diameter of a cell (e.g., a single cell entrapped by a well of the substrate). In some embodiments, the diameters of the beads can be, or be about, 10%, 20%, 30%, 40%, 50%, 60%, 70%, 80%, 90%, 100%, 150%, 200%, 250%, 300%, or a number or a range between any two of these values, longer or shorter than the diameter of the cell.

A bead can be attached to and/or embedded in a substrate. A bead can be attached to and/or embedded in a gel, hydrogel, polymer and/or matrix. The spatial position of a bead within a substrate (e.g., gel, matrix, scaffold, or polymer) can be identified using the spatial label present on the stochastic barcode on the bead which can serve as a location address.

Examples of beads can include, but are not limited to, streptavidin beads, agarose beads, magnetic beads, Dynabeads^{®}, MACS^{®} microbeads, antibody conjugated beads (e.g., anti-immunoglobulin microbeads), protein A conjugated beads, protein G conjugated beads, protein A/G conjugated beads, protein L conjugated beads, oligo(dT) conjugated beads, silica beads, silica-like beads, anti-biotin microbeads, anti-fluorochrome microbeads, and BcMag^{™} Carboxyl-Terminated Magnetic Beads.

A bead can be associated with (e.g., impregnated with) quantum dots or fluorescent dyes to make it fluorescent in one fluorescence optical channel or multiple optical channels. A bead can be associated with iron oxide or chromium oxide to make it paramagnetic or ferromagnetic. Beads can be identifiable. For example, a bead can be imaged using a camera. A bead can have a detectable code associated with the bead. For example, a bead can comprise a stochastic barcode. A bead can change size, for example due to swelling in an organic or inorganic solution. A bead can be hydrophobic. A bead can be hydrophilic. A bead can be biocompatible.

A solid support (e.g., bead) can be visualized. The solid support can comprise a visualizing tag (e.g., fluorescent dye). A solid support (e.g., bead) can be etched with an identifier (e.g., a number). The identifier can be visualized through imaging the beads.

### Substrates and Microwell Arrays

As used herein, a substrate can refer to a type of solid support. A substrate can refer to a solid support that can comprise barcodes (e.g., stochastic barcodes) of the disclosure. A substrate can, for example, comprise a plurality of microwells. For example, a substrate can be a well array comprising two or more microwells. In some embodiments, a microwell can comprise a small reaction chamber of defined volume. In some embodiments, a microwell can entrap one or more cells. In some embodiments, a microwell can entrap only one cell. In some embodiments, a microwell can entrap one or more solid supports. In some embodiments, a microwell can entrap only one solid support. In some embodiments, a microwell entraps a single cell and a single solid support (e.g., bead).

### Methods of Stochastic Barcoding

Provided herein are methods for estimating the number of distinct targets at distinct locations in a physical sample (e.g., tissue, organ, tumor, cell). The methods can comprise placing the stochastic barcodes in close proximity with the sample, lysing the sample, associating distinct targets with the stochastic barcodes, amplifying the targets and/or digitally counting the targets. The method can further comprise analyzing and/or visualizing the information obtained from the spatial labels on the stochastic barcodes. In some embodiments, the method comprises visualizing the plurality of targets in the sample. Mapping the plurality of targets onto the map of the sample can include generating a two dimensional map or a three dimensional map of the sample. The two dimensional map and the three dimensional map can be generated prior to or after stochastically barcoding the plurality of targets in the sample. Visualizing the plurality of targets in the sample can include mapping the plurality of targets onto a map of the sample. Mapping the plurality of targets onto the map of the sample can include generating a two dimensional map or a three dimensional map of the sample. The two dimensional map and the three dimensional map can be generated prior to or after stochastically barcoding the plurality of targets in the sample. in some embodiments, the two dimensional map and the three dimensional map can be generated before or after lysing the sample. Lysing the sample before or after generating the two dimensional map or the three dimensional map can include heating the sample, contacting the sample with a detergent, changing the pH of the sample, or any combination thereof.

In some embodiments, stochastically barcoding the plurality of targets comprises hybridizing a plurality of stochastic barcodes with a plurality of targets to create stochastically barcoded targets. Stochastically barcoding the plurality of targets can comprise generating an indexed library of the stochastically barcoded targets. Generating an indexed library of the stochastically barcoded targets can be performed with a solid support comprising the plurality of stochastic barcodes.

### Contacting a Sample and Barcode

The disclosure relates tomethods for contacting a sample (e.g., cells) to a substrate of the disclosure. A sample comprising, for example, a cell, organ, or tissue thin section, can be contacted to barcodes (e.g., stochastic barcodes). The cells can be contacted, for example, by gravity flow wherein the cells can settle and create a monolayer. The sample can be a tissue thin section. The thin section can be placed on the substrate. The sample can be one-dimensional (e.g., form a planar surface). The sample (e.g., cells) can be spread across the substrate, for example, by growing/culturing the cells on the substrate.

When stochastic barcodes are in close proximity to targets, the targets can hybridize to the stochastic barcode. The stochastic barcodes can be contacted at a non-depletable ratio such that each distinct target can associate with a distinct stochastic barcode of the disclosure. To ensure efficient association between the target and the stochastic barcode, the targets can be crosslinked to the stochastic barcode.

### Cell Lysis

Following the distribution of cells and barcodes (e.g., stochastic barcodes), the cells can be lysed to liberate the target molecules. Cell lysis can be accomplished by any of a variety of means, for example, by chemical or biochemical means, by osmotic shock, or by means of thermal lysis, mechanical lysis, or optical lysis. Cells can be lysed by addition of a cell lysis buffer comprising a detergent (e.g., SDS, Li dodecyl sulfate, Triton X-100, Tween-20, or NP-40), an organic solvent (e.g., methanol or acetone), or digestive enzymes (e.g., proteinase K, pepsin, or trypsin), or any combination thereof. To increase the association of a target and a stochastic barcode, the rate of the diffusion of the target molecules can be altered by for example, reducing the temperature and/or increasing the viscosity of the lysate.

In some embodiments, the sample can be lysed using a filter paper. The filter paper can be soaked with a lysis buffer on top of the filter paper. The filter paper can be applied to the sample with pressure which can facilitate lysis of the sample and hybridization of the targets of the sample to the substrate.

In some embodiments, lysis can be performed by mechanical lysis, heat lysis, optical lysis, and/or chemical lysis. Chemical lysis can include the use of digestive enzymes such as proteinase K, pepsin, and trypsin. Lysis can be performed by the addition of a lysis buffer to the substrate. A lysis buffer can comprise Tris HCl. A lysis buffer can comprise at least about 0.01, 0.05, 0.1, 0.5, or 1 M or more Tris HCl. A lysis buffer can comprise at most about 0.01, 0.05, 0.1, 0.5, or 1 M or more Tris HCL. A lysis buffer can comprise about 0.1 M Tris HCl. The pH of the lysis buffer can be at least about 1, 2, 3, 4, 5, 6, 7, 8, 9, or 10 or more. The pH of the lysis buffer can be at most about 1, 2, 3, 4, 5, 6, 7, 8, 9, or 10 or more. In some embodiments, the pH of the lysis buffer is about 7.5. The lysis buffer can comprise a salt (e.g., LiCl). The concentration of salt in the lysis buffer can be at least about 0.1, 0.5, or 1 M or more. The concentration of salt in the lysis buffer can be at most about 0.1, 0.5, or 1 M or more. In some embodiments, the concentration of salt in the lysis buffer is about 0.5M. The lysis buffer can comprise a detergent (e.g., SDS, Li dodecyl sulfate, triton X, tween, NP-40). The concentration of the detergent in the lysis buffer can be at least about 0.0001%, 0.0005%, 0.001%, 0.005%, 0.01%, 0.05%, 0.1%, 0.5%, 1%, 2%, 3%, 4%, 5%, 6%, or 7% or more. The concentration of the detergent in the lysis buffer can be at most about 0.0001%, 0.0005%, 0.001%, 0.005%, 0.01%, 0.05%, 0.1%, 0.5%, 1%, 2%, 3%, 4%, 5%, 6%, or 7% or more. In some embodiments, the concentration of the detergent in the lysis buffer is about 1% Li dodecyl sulfate. The time used in the method for lysis can be dependent on the amount of detergent used. In some embodiments, the more detergent used, the less time needed for lysis. The lysis buffer can comprise a chelating agent (e.g., EDTA, EGTA). The concentration of a chelating agent in the lysis buffer can be at least about 1, 5, 10, 15, 20, 25, or 30 mM or more. The concentration of a chelating agent in the lysis buffer can be at most about 1, 5, 10, 15, 20, 25, or 30mM or more. In some embodiments, the concentration of chelating agent in the lysis buffer is about 10 mM. The lysis buffer can comprise a reducing reagent (e.g., betamercaptoethanol, DTT). The concentration of the reducing reagent in the lysis buffer can be at least about 1, 5, 10, 15, or 20 mM or more. The concentration of the reducing reagent in the lysis buffer can be at most about 1, 5, 10, 15, or 20 mM or more. In some embodiments, the concentration of reducing reagent in the lysis buffer is about 5 mM. In some embodiments, a lysis buffer can comprise about 0.1M TrisHCl, about pH 7.5, about 0.5M LiCl, about 1% lithium dodecyl sulfate, about 10mM EDTA, and about 5mM DTT.

Lysis can be performed at a temperature of about 4, 10, 15, 20, 25, or 30 °C. Lysis can be performed for about 1, 5, 10, 15, or 20 or more minutes. A lysed cell can comprise at least about 100000, 200000, 300000, 400000, 500000, 600000, or 700000 or more target nucleic acid molecules. A lysed cell can comprise at most about 100000, 200000, 300000, 400000, 500000, 600000, or 700000 or more target nucleic acid molecules.

### Attachment of Barcodes to Target Nucleic Acid Molecules

Following lysis of the cells and release of nucleic acid molecules therefrom, the nucleic acid molecules can randomly associate with the barcodes (e.g., stochastic barcodes) of the co-localized solid support. Association can comprise hybridization of a stochastic barcode's target recognition region to a complementary portion of the target nucleic acid molecule (e.g., oligo(dT) of the stochastic barcode can interact with a poly(A) tail of a target). The assay conditions used for hybridization (e.g., buffer pH, ionic strength, temperature, etc.) can be chosen to promote formation of specific, stable hybrids. In some embodiments, the nucleic acid molecules released from the lysed cells can associate with the plurality of probes on the substrate (e.g., hybridize with the probes on the substrate). When the probes comprise oligo(dT), mRNA molecules can hybridize to the probes and be reverse transcribed. The oligo(dT) portion of the oligonucleotide can act as a primer for first strand synthesis of the cDNA molecule. For example, in a non-limiting example of stochastic barcoding illustrated in FIG. 2, at 216, mRNA molecules can hybridize to stochastic barcodes on beads. For example, single-stranded nucleotide fragments can hybridize to the target-binding regions of stochastic barcodes.

Attachment can further comprise ligation of a stochastic barcode's target recognition region and a portion of the target nucleic acid molecule. For example, the target-binding region can comprise a nucleic acid sequence that can be capable of specific hybridization to a restriction site overhang (e.g., an EcoRI sticky-end overhang). The assay procedure can further comprise treating the target nucleic acids with a restriction enzyme (e.g., EcoRI) to create a restriction site overhang. The stochastic barcode can then be ligated to any nucleic acid molecule comprising a sequence complementary to the restriction site overhang. A ligase (e.g., T4 DNA ligase) can be used to join the two fragments.

For example, in a non-limiting example of stochastic barcoding illustrated in FIG. 2, at 220, the labeled targets from a plurality of cells (or a plurality of samples) (e.g., target-barcode molecules) can be subsequently pooled, for example, into a tube. The labeled targets can be pooled by, for example, retrieving the stochastic barcodes and/or the beads to which the target-barcode molecules are attached.

The retrieval of solid support-based collections of attached target-barcode molecules can be implemented by use of magnetic beads and an externally-applied magnetic field. Once the target-barcode molecules have been pooled, all further processing can proceed in a single reaction vessel. Further processing can include, for example, reverse transcription reactions, amplification reactions, cleavage reactions, dissociation reactions, and/or nucleic acid extension reactions. Further processing reactions can be performed within the microwells, that is, without first pooling the labeled target nucleic acid molecules from a plurality of cells.

### Reverse Transcription

The disclosure relates to methods to create a target-barcode conjugate (e.g., a stochastic target-barcode conjugate) using reverse transcription (e.g., in 224 of FIG. 2). The stochastic target-barcode conjugate can comprise the stochastic barcode and a complementary sequence of all or a portion of the target nucleic acid (i.e., a stochastically barcoded cDNA molecule). Reverse transcription of the associated RNA molecule can occur by the addition of a reverse transcription primer along with the reverse transcriptase. The reverse transcription primer can be an oligo(dT) primer, a random hexanucleotide primer, or a target-specific oligonucleotide primer. Oligo(dT) primers can be, or can be about, 12-18 nucleotides in length and bind to the endogenous poly(A) tail at the 3' end of mammalian mRNA. Random hexanucleotide primers can bind to mRNA at a variety of complementary sites. Target-specific oligonucleotide primers typically selectively prime the mRNA of interest.

In some embodiments, reverse transcription of the labeled-RNA molecule can occur by the addition of a reverse transcription primer. In some embodiments, the reverse transcription primer is an oligo(dT) primer, random hexanucleotide primer, or a target-specific oligonucleotide primer. Generally, oligo(dT) primers are 12-18 nucleotides in length and bind to the endogenous poly(A)+ tail at the 3' end of mammalian mRNA. Random hexanucleotide primers can bind to mRNA at a variety of complementary sites. Target-specific oligonucleotide primers typically selectively prime the mRNA of interest.

Reverse transcription can occur repeatedly to produce multiple labeled-cDNA molecules. The methods disclosed herein can comprise conducting at least about 1, 2, 3, 4, 5, 6, 7, 8, 9, 10, 11, 12, 13, 14, 15, 16, 17, 18, 19, or 20 reverse transcription reactions. The method can comprise conducting at least about 25, 30, 35, 40, 45, 50, 55, 60, 65, 70, 75, 80, 85, 90, 95, or 100 reverse transcription reactions.

### Amplification

One or more nucleic acid amplification reactions (e.g., 228 of FIG. 2) can be performed to create multiple copies of the labeled target nucleic acid molecules. Amplification can be performed in a multiplexed manner, wherein multiple target nucleic acid sequences are amplified simultaneously. The amplification reaction can be used to add sequencing adaptors to the nucleic acid molecules. The amplification reactions can comprise amplifying at least a portion of a sample label, if present. The amplification reactions can comprise amplifying at least a portion of the cellular and/or molecular label. The amplification reactions can comprise amplifying at least a portion of a sample tag, a cell label, a spatial label, a molecular label, a target nucleic acid, or a combination thereof. The amplification reactions can comprise amplifying 0.5%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, 100%, or a range or a number between any two of these values, of the plurality of nucleic acids. The method can further comprise conducting one or more cDNA synthesis reactions to produce one or more cDNA copies of target-barcode molecules comprising a sample label, a cell label, a spatial label, and/or a molecular label.

In some embodiments, amplification can be performed using a polymerase chain reaction (PCR). As used herein, PCR can refer to a reaction for the in vitro amplification of specific DNA sequences by the simultaneous primer extension of complementary strands of DNA. As used herein, PCR can encompass derivative forms of the reaction, including but not limited to, RT-PCR, real-time PCR, nested PCR, quantitative PCR, multiplexed PCR, digital PCR, and assembly PCR.

Amplification of the labeled nucleic acids can comprise non-PCR based methods. Examples of non-PCR based methods include, but are not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR-based amplification methods include multiple cycles of DNA-dependent RNA polymerase-driven RNA transcription amplification or RNA-directed DNA synthesis and transcription to amplify DNA or RNA targets, a ligase chain reaction (LCR), and a Qβ replicase (Qβ) method, use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and ramification extension amplification (RAM). In some embodiments, the amplification does not produce circularized transcripts.

In some embodiments, the methods disclosed herein further comprise conducting a polymerase chain reaction on the labeled nucleic acid (e.g., labeled-RNA, labeled-DNA, labeled-cDNA) to produce a labeled amplicon (e.g., a stochastically labeled amplicon). The labeled amplicon can be double-stranded molecule. The double-stranded molecule can comprise a double-stranded RNA molecule, a double-stranded DNA molecule, or a RNA molecule hybridized to a DNA molecule. One or both of the strands of the double-stranded molecule can comprise a sample label, a spatial label, a cell label, and/or a molecular label. The stochastically labeled amplicon can be a single-stranded molecule. The single-stranded molecule can comprise DNA, RNA, or a combination thereof. The nucleic acids of the disclosure can comprise synthetic or altered nucleic acids.

Amplification can comprise use of one or more non-natural nucleotides. Non-natural nucleotides can comprise photolabile or triggerable nucleotides. Examples of non-natural nucleotides can include, but are not limited to, peptide nucleic acid (PNA), morpholino and locked nucleic acid (LNA), as well as glycol nucleic acid (GNA) and threose nucleic acid (TNA). Non-natural nucleotides can be added to one or more cycles of an amplification reaction. The addition of the non-natural nucleotides can be used to identify products as specific cycles or time points in the amplification reaction.

Conducting the one or more amplification reactions can comprise the use of one or more primers. The one or more primers can comprise, for example, 1, 2, 3, 4, 5, 6, 7, 8, 9, 10, 11, 12, 13, 14, or 15 or more nucleotides. The one or more primers can comprise at least 1, 2, 3, 4, 5, 6, 7, 8, 9, 10, 11, 12, 13, 14, or 15 or more nucleotides. The one or more primers can comprise less than 12-15 nucleotides. The one or more primers can anneal to at least a portion of the plurality of stochastically labeled targets. The one or more primers can anneal to the 3' end or 5' end of the plurality of stochastically labeled targets. The one or more primers can anneal to an internal region of the plurality of stochastically labeled targets. The internal region can be at least about 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides from the 3' ends the plurality of stochastically labeled targets. The one or more primers can comprise a fixed panel of primers. The one or more primers can comprise at least one or more custom primers. The one or more primers can comprise at least one or more control primers. The one or more primers can comprise at least one or more gene-specific primers.

The one or more primers can comprise a universal primer. The universal primer can anneal to a universal primer binding site. The one or more custom primers can anneal to a first sample label, a second sample label, a spatial label, a cell label, a molecular label, a target, or any combination thereof. The one or more primers can comprise a universal primer and a custom primer. The custom primer can be designed to amplify one or more targets. The targets can comprise a subset of the total nucleic acids in one or more samples. The targets can comprise a subset of the total stochastically labeled targets in one or more samples. The one or more primers can comprise at least 96 or more custom primers. The one or more primers can comprise at least 960 or more custom primers. The one or more primers can comprise at least 9600 or more custom primers. The one or more custom primers can anneal to two or more different labeled nucleic acids. The two or more different labeled nucleic acids can correspond to one or more genes.

Any amplification scheme can be used in the methods of the present disclosure. For example, in one scheme, the first round PCR can amplify molecules attached to the bead using a gene specific primer and a primer against the universal Illumina sequencing primer 1 sequence. The second round of PCR can amplify the first PCR products using a nested gene specific primer flanked by Illumina sequencing primer 2 sequence, and a primer against the universal Illumina sequencing primer 1 sequence. The third round of PCR adds P5 and P7 and sample index to turn PCR products into an Illumina sequencing library. Sequencing using 150bp x 2 sequencing can reveal the cell label and molecular label on read 1, the gene on read 2, and the sample index on index 1 read.

In some embodiments, nucleic acids can be removed from the substrate using chemical cleavage. For example, a chemical group or a modified base present in a nucleic acid can be used to facilitate its removal from a solid support. For example, an enzyme can be used to remove a nucleic acid from a substrate. For example, a nucleic acid can be removed from a substrate through a restriction endonucelase digestion. For example, treatment of a nucleic acid containing a dUTP or ddUTP with uracil-d-glycosylase (UDG) can be used to remove a nucleic acid from a substrate. For example, a nucleic acid can be removed from a substrate using an enzyme that performs nucleotide excision, such as a base excision repair enzyme, such as an apurinic/apyrimidinic (AP) endonuclease. In some embodiments, a nucleic acid can be removed from a substrate using a photocleavable group and light. In some embodiments, a cleavable linker can be used to remove a nucleic acid from the substrate. For example, the cleavable linker can comprise at least one of biotin/avidin, biotin/streptavidin, biotin/neutravidin, Ig-protein A, a photo-labile linker, acid or base labile linker group, or an aptamer.

When the probes are gene-specific, the molecules can hybridize to the probes and be reverse transcribed and/or amplified. In some embodiments, after the nucleic acid has been synthesized (e.g., reverse transcribed), it can be amplified. Amplification can be performed in a multiplex manner, wherein multiple target nucleic acid sequences are amplified simultaneously. Amplification can add sequencing adaptors to the nucleic acid.

In some embodiments, amplification can be performed on the substrate, for example, with bridge amplification. cDNAs can be homopolymer tailed in order to generate a compatible end for bridge amplification using oligo(dT) probes on the substrate. In bridge amplification, the primer that is complementary to the 3' end of the template nucleic acid can be the first primer of each pair that is covalently attached to the solid particle. When a sample containing the template nucleic acid is contacted with the particle and a single thermal cycle is performed, the template molecule can be annealed to the first primer and the first primer is elongated in the forward direction by addition of nucleotides to form a duplex molecule consisting of the template molecule and a newly formed DNA strand that is complementary to the template. In the heating step of the next cycle, the duplex molecule can be denatured, releasing the template molecule from the particle and leaving the complementary DNA strand attached to the particle through the first primer. In the annealing stage of the annealing and elongation step that follows, the complementary strand can hybridize to the second primer, which is complementary to a segment of the complementary strand at a location removed from the first primer. This hybridization can cause the complementary strand to form a bridge between the first and second primers secured to the first primer by a covalent bond and to the second primer by hybridization. In the elongation stage, the second primer can be elongated in the reverse direction by the addition of nucleotides in the same reaction mixture, thereby converting the bridge to a double-stranded bridge. The next cycle then begins, and the double-stranded bridge can be denatured to yield two single-stranded nucleic acid molecules, each having one end attached to the particle surface via the first and second primers, respectively, with the other end of each unattached. In the annealing and elongation step of this second cycle, each strand can hybridize to a further complementary primer, previously unused, on the same particle, to form new single-strand bridges. The two previously unused primers that are now hybridized elongate to convert the two new bridges to double-strand bridges.

The amplification reactions can comprise amplifying at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, or 100% of the plurality of nucleic acids.

Amplification of the labeled nucleic acids can comprise PCR-based methods or non-PCR based methods. Amplification of the labeled nucleic acids can comprise exponential amplification of the labeled nucleic acids. Amplification of the labeled nucleic acids can comprise linear amplification of the labeled nucleic acids. Amplification can be performed by polymerase chain reaction (PCR). PCR can refer to a reaction for the in vitro amplification of specific DNA sequences by the simultaneous primer extension of complementary strands of DNA. PCR can encompass derivative forms of the reaction, including but not limited to, RT-PCR, real-time PCR, nested PCR, quantitative PCR, multiplexed PCR, digital PCR, suppression PCR, semi-suppressive PCR and assembly PCR.

In some embodiments, amplification of the labeled nucleic acids comprises non-PCR based methods. Examples of non-PCR based methods include, but are not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR-based amplification methods include multiple cycles of DNA-dependent RNA polymerase-driven RNA transcription amplification or RNA-directed DNA synthesis and transcription to amplify DNA or RNA targets, a ligase chain reaction (LCR), a Qβ replicase (Qβ), use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and/or ramification extension amplification (RAM).

In some embodiments, the methods disclosed herein further comprise conducting a nested polymerase chain reaction on the amplified amplicon (e.g., target). The amplicon can be double-stranded molecule. The double-stranded molecule can comprise a double-stranded RNA molecule, a double-stranded DNA molecule, or a RNA molecule hybridized to a DNA molecule. One or both of the strands of the double-stranded molecule can comprise a sample tag or molecular identifier label. Alternatively, the amplicon can be a single-stranded molecule. The single-stranded molecule can comprise DNA, RNA, or a combination thereof. The nucleic acids can comprise synthetic or altered nucleic acids.

In some embodiments, the method comprises repeatedly amplifying the labeled nucleic acid to produce multiple amplicons. The methods disclosed herein can comprise conducting at least about 1, 2, 3, 4, 5, 6, 7, 8, 9, 10, 11, 12, 13, 14, 15, 16, 17, 18, 19, or 20 amplification reactions. Alternatively, the method comprises conducting at least about 25, 30, 35, 40, 45, 50, 55, 60, 65, 70, 75, 80, 85, 90, 95, or 100 amplification reactions.

Amplification can further comprise adding one or more control nucleic acids to one or more samples comprising a plurality of nucleic acids. Amplification can further comprise adding one or more control nucleic acids to a plurality of nucleic acids. The control nucleic acids can comprise a control label.

Amplification can comprise use of one or more non-natural nucleotides. Non-natural nucleotides can comprise photolabile and/or triggerable nucleotides. Examples of non-natural nucleotides include, but are not limited to, peptide nucleic acid (PNA), morpholino and locked nucleic acid (LNA), as well as glycol nucleic acid (GNA) and threose nucleic acid (TNA). Non-natural nucleotides can be added to one or more cycles of an amplification reaction. The addition of the non-natural nucleotides can be used to identify products as specific cycles or time points in the amplification reaction.

Conducting the one or more amplification reactions can comprise the use of one or more primers. The one or more primers can comprise one or more oligonucleotides. The one or more oligonucleotides can comprise at least about 7-9 nucleotides. The one or more oligonucleotides can comprise less than 12-15 nucleotides. The one or more primers can anneal to at least a portion of the plurality of labeled nucleic acids. The one or more primers can anneal to the 3' end and/or 5' end of the plurality of labeled nucleic acids. The one or more primers can anneal to an internal region of the plurality of labeled nucleic acids. The internal region can be at least about 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900, or 1000 nucleotides from the 3' ends the plurality of labeled nucleic acids. The one or more primers can comprise a fixed panel of primers. The one or more primers can comprise at least one or more custom primers. The one or more primers can comprise at least one or more control primers. The one or more primers can comprise at least one or more housekeeping gene primers. The one or more primers can comprise a universal primer. The universal primer can anneal to a universal primer binding site. The one or more custom primers can anneal to the first sample tag, the second sample tag, the molecular identifier label, the nucleic acid or a product thereof. The one or more primers can comprise a universal primer and a custom primer. The custom primer can be designed to amplify one or more target nucleic acids. The target nucleic acids can comprise a subset of the total nucleic acids in one or more samples. In some embodiments, the primers are the probes attached to the array of the disclosure.

In some embodiments, barcoding (e.g., stochastically barcoding) the plurality of targets in the sample further comprises generating an indexed library of the stochastically barcoded fragments. The molecular labels of different stochastic barcodes can be different from one another. Generating an indexed library of the stochastically barcoded targets includes generating a plurality of indexed polynucleotides from the plurality of targets in the sample. For example, for an indexed library of the stochastically barcoded targets comprising a first indexed target and a second indexed target, the label region of the first indexed polynucleotide can differ from the label region of the second indexed polynucleotide by, by about, by at least, or by at most, 1, 2, 3, 4, 5, 6, 7, 8, 9, 10, 20, 30, 40, 50, or a number or a range between any two of these values, nucleotides. In some embodiments, generating an indexed library of the stochastically barcoded targets includes contacting a plurality of targets, for example mRNA molecules, with a plurality of oligonucleotides including a poly(T) region and a label region; and conducting a first strand synthesis using a reverse transcriptase to produce single-strand labeled cDNA molecules each comprising a cDNA region and a label region, wherein the plurality of targets includes at least two mRNA molecules of different sequences and the plurality of oligonucleotides includes at least two oligonucleotides of different sequences. Generating an indexed library of the stochastically barcoded targets can further comprise amplifying the single-strand labeled cDNA molecules to produce double-strand labeled cDNA molecules; and conducting nested PCR on the double-strand labeled cDNA molecules to produce labeled amplicons. In some embodiments, the method can include generating an adaptor-labeled amplicon.

Barcoding (e.g., stochastic barcoding) can use nucleic acid barcodes or tags to label individual nucleic acid (e.g., DNA or RNA) molecules. In some embodiments, it involves adding DNA barcodes or tags to cDNA molecules as they are generated from mRNA. Nested PCR can be performed to minimize PCR amplification bias. Adaptors can be added for sequencing using, for example, next generation sequencing (NGS). The sequencing results can be used to determine cell labels, molecular labels, and sequences of nucleotide fragments of the one or more copies of the targets, for example at 232 of FIG. 2.

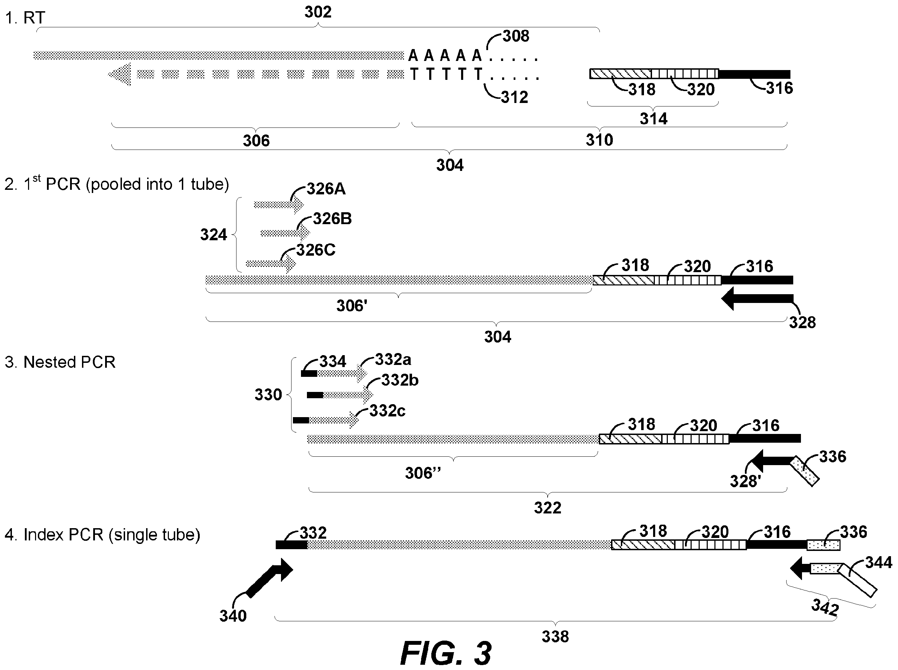

FIG. 3 is a schematic illustration showing a non-limiting exemplary process of generating an indexed library of the stochastically barcoded targets, for example mRNAs. As shown in step 1, the reverse transcription process can encode each mRNA molecule with a unique molecular label, a cell label, and a universal PCR site. In particular, RNA molecules 302 can be reverse transcribed to produce labeled cDNA molecules 304, including a cDNA region 306, by the stochastic hybridization of a set of molecular identifier labels 310 to the poly(A) tail region 308 of the RNA molecules 302. Each of the molecular identifier labels 310 can comprise a target-binding region, for example a poly(dT) region 312, a label region 314, and a universal PCR region 316.

In some embodiments, the cell label can include 3 to 20 nucleotides. In some embodiments, the molecular label can include 3 to 20 nucleotides. In some embodiments, each of the plurality of stochastic barcodes further comprises one or more of a universal label and a cell label, wherein universal labels are the same for the plurality of stochastic barcodes on the solid support and cell labels are the same for the plurality of stochastic barcodes on the solid support. In some embodiments, the universal label can include 3 to 20 nucleotides. In some embodiments, the cell label comprises 3 to 20 nucleotides.

In some embodiments, the label region 314 can include a molecular label 318 and a cell label 320. In some embodiments, the label region 314 can include one or more of a universal label, a dimension label, and a cell label. The molecular label 318 can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. The cell label 320 can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. The universal label can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. Universal labels can be the same for the plurality of stochastic barcodes on the solid support and cell labels are the same for the plurality of stochastic barcodes on the solid support. The dimension label can be, can be about, can be at least, or can be at most 1, 2, 3, 4, 5, 6, 7, 8, 9, 10, 20, 30, 40, 50, 60, 70, 80, 90, 100, or a number or a range between any of these values, of nucleotides in length.

In some embodiments, the label region 314 can comprise, comprise about, comprise at least, or comprise at most, 1, 2, 3, 4, 5, 6, 7, 8, 9, 10, 20, 30, 40, 50, 60, 70, 80, 90, 100, 200, 300, 400, 500, 600, 700, 800, 900, 1000, or a number or a range between any of these values, different labels, such as a molecular label 318 and a cell label 320. Each label can be, can be about, can be at least, or can be at most 1, 2, 3, 4, 5, 6, 7, 8, 9, 10, 20, 30, 40, 50, 60, 70, 80, 90, 100, or a number or a range between any of these values, of nucleotides in length. A set of molecular identifier labels 310 can contain, contain about, contain at least, or can be at most, 10, 20, 40, 50, 70, 80, 90, 10², 10³, 10⁴, 10⁵, 10⁶, 10⁷, 10⁸, 10⁹, 10¹⁰, 10¹¹, 10¹², 10¹³, 10¹⁴, 10¹⁵, 10²⁰, or a number or a range between any of these values, molecular identifier labels 310. And the set of molecular identifier labels 310 can, for example, each contain a unique label region 314. The labeled cDNA molecules 304 can be purified to remove excess molecular identifier labels 310. Purification can comprise Ampure bead purification.

As shown in step 2, products from the reverse transcription process in step 1 can be pooled into 1 tube and PCR amplified with a 1^{st} PCR primer pool and a 1^{st} universal PCR primer. Pooling is possible because of the unique label region 314. In particular, the labeled cDNA molecules 304 can be amplified to produce nested PCR labeled amplicons 322. Amplification can comprise multiplex PCR amplification. Amplification can comprise a multiplex PCR amplification with 96 multiplex primers in a single reaction volume. In some embodiments, multiplex PCR amplification can utilize, utilize about, utilize at least, or utilize at most, 10, 20, 40, 50, 70, 80, 90, 10², 10³, 10⁴, 10⁵, 10⁶, 10⁷, 10⁸, 10⁹, 10¹⁰, 10¹¹, 10¹², 10¹³, 10¹⁴, 10¹⁵, 10²⁰, or a number or a range between any of these values, multiplex primers in a single reaction volume. Amplification can comprise 1^{st} PCR primer pool 324 of custom primers 326A-C targeting specific genes and a universal primer 328. The custom primers 326 can hybridize to a region within the cDNA portion 306' of the labeled cDNA molecule 304. The universal primer 328 can hybridize to the universal PCR region 316 of the labeled cDNA molecule 304.

As shown in step 3 of FIG. 3, products from PCR amplification in step 2 can be amplified with a nested PCR primers pool and a 2^{nd} universal PCR primer. Nested PCR can minimize PCR amplification bias. In particular, the nested PCR labeled amplicons 322 can be further amplified by nested PCR. The nested PCR can comprise multiplex PCR with nested PCR primers pool 330 of nested PCR primers 332a-c and a 2^{nd} universal PCR primer 328' in a single reaction volume. The nested PCR primer pool 328 can contain, contain about, contain at least, or contain at most, 1, 2, 3, 4, 5, 6, 7, 8, 9, 10, 20, 30, 40, 50, 60, 70, 80, 90, 100, 200, 300, 400, 500, 600, 700, 800, 900, 1000, or a number or a range between any of these values, different nested PCR primers 330. The nested PCR primers 332 can contain an adaptor 334 and hybridize to a region within the cDNA portion 306" of the labeled amplicon 322. The universal primer 328' can contain an adaptor 336 and hybridize to the universal PCR region 316 of the labeled amplicon 322. Thus, step 3 produces adaptor-labeled amplicon 338. In some embodiments, nested PCR primers 332 and the 2^{nd} universal PCR primer 328' may not contain the adaptors 334 and 336. The adaptors 334 and 336 can instead be ligated to the products of nested PCR to produce adaptor-labeled amplicon 338.

As shown in step 4, PCR products from step 3 can be PCR amplified for sequencing using library amplification primers. In particular, the adaptors 334 and 336 can be used to conduct one or more additional assays on the adaptor-labeled amplicon 338. The adaptors 334 and 336 can be hybridized to primers 340 and 342. The one or more primers 340 and 342 can be PCR amplification primers. The one or more primers 340 and 342 can be sequencing primers. The one or more adaptors 334 and 336 can be used for further amplification of the adaptor-labeled amplicons 338. The one or more adaptors 334 and 336 can be used for sequencing the adaptor-labeled amplicon 338. The primer 342 can contain a plate index 344 so that amplicons generated using the same set of molecular identifier labels 318 can be sequenced in one sequencing reaction using next generation sequencing (NGS).

### Multiplet Expression Profile Identification using Synthetic Multiplets

When determining expression profiles of single cells, two cells may be identified as one cell and the expression profiles of the two cells may be identified as the expression profile for one cell (referred to as a doublet expression profile). For example, when determining expression profiles of two cells using barcoding (e.g., stochastic barcoding), the mRNA molecules of the two cells may be associated with barcodes having the same cell label. As another example, two cells may be associated with one particle (e.g., a bead). The particle can include barcodes with the same cell label. After lysing the cells, the mRNA molecules in the two cells can be associated with the barcodes of the particle, thus the same cell label. Doublet expression profiles can skew the interpretation of the expression profiles. Disclosed herein are systems and methods for identifying a multiplet expression profile using synthetic multiplet expression profiles.

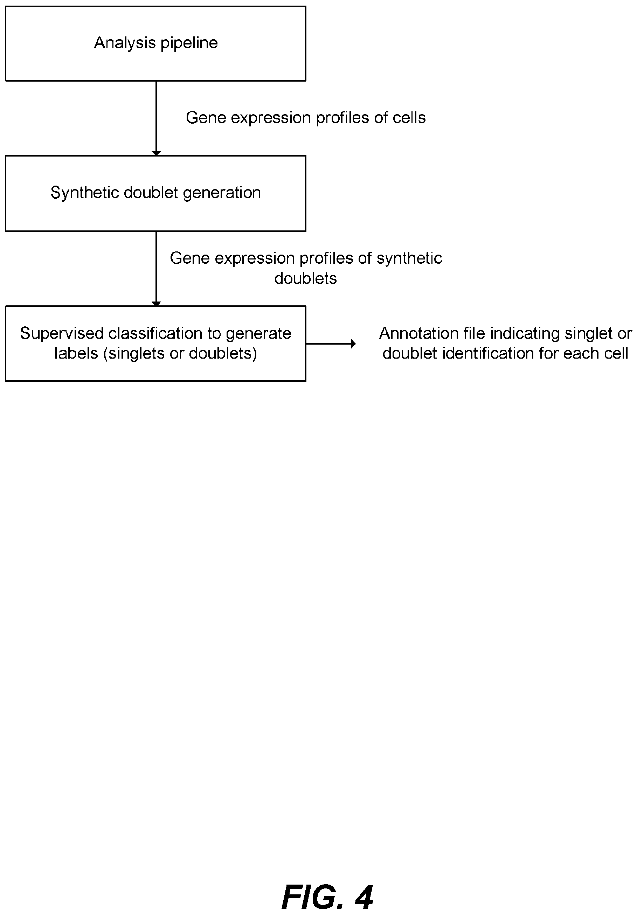

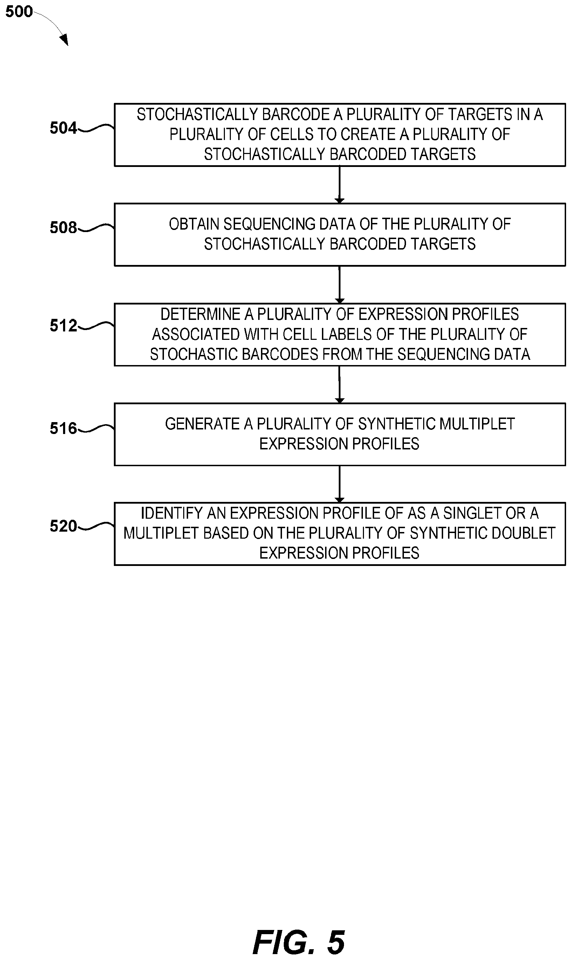

FIG. 4 is shows a non-limiting exemplary workflow of integrating synthetic doublet analysis into an analysis pipeline. An existing analysis pipeline can generate expression profiles of cells, such as expression profiles of thousands of cells. The expression profiles from the analysis pipeline can be used to generate expression profiles of synthetic doublets (also referred to as synthetic doublet expression profiles). Generation of synthetic doublet expression profiles is described in detail below with reference to FIG. 5. Briefly, two expression profiles of two cells can be combined to generate a synthetic doublet expression profile.

In some embodiments, the expression profiles and the synthetic doublet expression profiles can optionally be used to train a machine learning model (e.g., a supervised machine learning model). The supervised machine learning model can be used to identify or classify one or more expression profiles each as a singlet or a doublet. The output of the supervised machine learning model can be an annotation file indicating whether each of one or more expression profiles has a singlet or a doublet identification. The synthetic doublet analysis method disclosed herein can reduce the misinterpretation of doublet expression profiles and ameliorate the risk of confusing doublet expression profiles with biological discoveries and insights. The synthetic doublet analysis method can be fully automated or semi-automated. In some embodiments, a user can use synthetic doublet expression profiles generated to manually identify doublet expression profiles, for example, by inspecting a projection plot of the expression profiles and the synthetic doublet expression profiles.

FIG. 5 is a flowchart showing a non-limiting exemplary method 500 of synthetic doublet analysis. At block 504, a plurality of targets in a plurality of cells can be optionally barcoded (e.g., stochastically barcoded) using a plurality of barcodes (e.g., stochastic barcodes) to create a plurality of barcoded targets (e.g., stochastically barcoded targets) for cells of the plurality of cells. Each of the plurality of barcodes can comprise a cell label and a molecular label. Molecular labels of at least two barcodes of the plurality of barcodes can comprise different molecular label sequences. At least two barcodes of the plurality of barcodes can comprise cell labels with an identical cell label sequence. At block 508, sequencing data of the plurality of barcoded targets can be obtained.

At block 512, a plurality of expression profiles, associated with cell labels of the plurality of barcodes from the sequencing data, can be optionally determined. An expression profile of the plurality of expression profiles associated with a cell label of the cell labels of the plurality of barcodes can comprise a number of molecular labels with distinct sequences associated with the cell label and each target of the plurality of targets in the sequencing data. In some embodiments, determining the plurality of expression profiles associated with the cell labels of the plurality of barcodes from the sequencing data comprises: for an expression profile of the plurality of expression profiles associated with a cell label of the cell labels of the plurality of barcodes, determining a number of molecular labels with distinct sequences associated with the cell label and each target of the plurality of targets in the sequencing data. Determining the number of molecular labels with distinct sequences associated with the cell label and each target of the plurality of targets in the sequencing data can comprise: for one or more of the plurality of targets, (1) counting the number of molecular labels with distinct sequences associated with the target in the sequencing data; and (2) estimating the number of the target based on the number of molecular labels with distinct sequences associated with the target in the sequencing data counted in (1).

In some embodiments, instead of barcoding (e.g., stochastically barcoding) the plurality of targets, obtaining sequencing data of the plurality of barcoded targets (e.g., stochastically barcoded targets), and determining the plurality of expression profiles, a plurality of expression profiles of a plurality of cells can be received. The plurality of expression profiles comprise an occurrence (or a copy or a number) of each target of a plurality of targets for each cell of the plurality of cells.

At block 516, a plurality of synthetic multiplet expression profiles can be generated from the plurality of expression profiles associated with the cell labels of the plurality of barcodes (e.g., stochastic barcodes) determined. Multiplets can be different in different implementations. In some embodiments, the plurality of multiplets can include a doublet, a triplet, a quartet, a quintet, a sextet, a septet, an octet, a nonet, or any combination thereof. A multiplet can be any n-plet. In some embodiments, n is, or about, 2, 3, 4, 5, 6, 7, 8, 9, 10, 11, 12, 13, 14, 15, 16, 17, 18, 19, 20, or a range between any two of these values. In some embodiments, n is at least, or at most, 2, 3, 4, 5, 6, 7, 8, 9, 10, 11, 12, 13, 14, 15, 16, 17, 18, 19, or 20.

In some embodiments, generating the plurality of synthetic multiplet expression profiles from the plurality of expression profiles associated with the cell labels of the plurality of stochastic barcodes determined at block 516 comprises: for a synthetic multiplet expression profile of the plurality of synthetic multiplet expression profiles, (1) selecting a first expression profile of the plurality of expression profiles; (2) selecting a second expression profile of the plurality of expression profiles; and (3) combining the first expression profile selected in (1) and the second expression profile selected in (2) to generate a synthetic multiplet expression profile. The first expression profile can be associated with a first cell label sequence. The second expression profile can be associated with a second cell label sequence. The first cell label sequence and the second cell label sequence can comprise different cell label sequences

In some embodiments, combining the first expression profile selected in (1) and the second expression profile selected in (2) to generate the synthetic multiplet expression profile comprises: for each of the plurality of targets, combining a number of molecular labels with distinct sequences associated with the target in the first expression profile and a number of molecular labels with distinct sequences associated with the target in the second expression profile to generate a number of molecular labels with distinct sequences associated with the synthetic multiplet expression profile.

In some embodiments, the number of molecular labels with distinct sequences associated with the synthetic multiplet expression profile is a sum of the number of molecular labels with distinct sequences associated with the target in the first expression profile and the number of molecular labels with distinct sequences associated with the target in the second expression profile. The sum can be a weighted sum of the number of molecular labels with distinct sequences associated with the target in the first expression profile and the number of molecular labels with distinct sequences associated with the target in the second expression profile.

In some embodiments, the number of molecular labels with distinct sequences associated with the synthetic multiplet expression profile is an average of the number of molecular labels with distinct sequences associated with the target in the first expression profile and the number of molecular labels with distinct sequences associated with the target in the second expression profile. The average can be a weighted average of the number of molecular labels with distinct sequences associated with the target in the first expression profile and the number of molecular labels with distinct sequences associated with the target in the second expression profile.

At block 520, an expression profile of the plurality of expression profiles associated with a cell label of the cell labels of the plurality of barcodes (e.g., stochastic barcodes) can be identified as a singlet or a multiplet based on expression profiles of the plurality of synthetic doublet expression profiles generated at block 516. Identifying the expression profile of the plurality of expression profiles associated with the cell label of the cell labels of the plurality of barcodes as a singlet or a multiplet based on the expression profiles of the plurality of synthetic multiplet expression profiles comprises: (1) training a machine learning model for expression profile multiplet identification from the expression profiles of the plurality of synthetic multiplet expression profiles and one or more expression profiles of the plurality of expression profiles; and (2) identifying the expression profile of the plurality of expression profiles associated with the cell label of the cell labels of the plurality of barcodes as a singlet or a multiplet based on the expression profile using the machine learning model. The one or more expression profiles of the plurality of expression profiles used in training the machine learning model can comprise a percentage of the plurality of expression profiles.

The percentage of the plurality of expression profiles used in training the machine learning model can be different in different implementations, ranging from 1% to 50%, such as approximately 10 percent. In some embodiments, the percentage can be, or about, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or a range between any two of these values. In some embodiments, the percentage can be at least, or at most, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or a range between any two of these values.

In some embodiments, the machine learning model comprises a classification model. The classification model can comprise a supervised classification model, a semi-supervised classification model, an unsupervised classification model, or a combination thereof. The machine learning model can comprise a neural network, a linear regression model, a logistic regression model, a decision tree, a support vector machine, a Naive Bayes network, a k-nearest neighbors (KNN) model, a k-means model, a random forest model, or any combination thereof. The machine learning model can comprise an association rule learning model, an inductive logic programming model, a reinforcement learning model, a feature learning model, a similarity learning model, a sparse dictionary learning model, a genetic algorithm model, a rule-based machine learning model, a learning classifier system model, or any combination thereof.

In some embodiments, the method comprises: projecting the expression profile of the plurality of barcodes (e.g., stochastic barcodes) associated with the cell label of the cell labels of the plurality of barcodes to generate a projected expression profile of the plurality of barcodes. Identifying the expression profile of the plurality of expression profiles associated with the cell label of the cell labels of the plurality of barcodes as a singlet or a multiplet based on the expression profile using the machine learning model can comprise: identifying the expression profile of the plurality of expression profiles associated with the cell label of the cell labels of the plurality of barcodes as a singlet or a multiplet based on the expression profile using the machine learning model and the projected expression profile of the plurality of barcodes.

In some embodiments, the method optionally comprises: if the expression profile is identified as a multiplet, removing sequencing data associated with the expression profile from the sequencing data. The method can comprise: if the expression profile is identified as a multiplet, removing the expression profile from the plurality of expression profiles.

In some embodiments, training the machine learning model for expression profile multiplet identification from the expression profiles of the plurality of synthetic multiplet expression profiles and the one or more expression profiles of the plurality of expression profiles comprises: (1) projecting the expression profiles of the plurality of synthetic multiplet expression profiles from an expression profile space into a lower dimensional projection space to generate projected expression profiles of the plurality of synthetic multiplet expression profiles; (2) projecting the one or more expression profiles of the plurality of expression profiles from the expression profile space into the lower dimensional projection space to generate one or more projected expression profiles of the plurality of expression profiles; and (3) training the machine learning model for expression profile multiplet identification from the projected expression profiles of the plurality of synthetic multiplet expression profiles from (1) and the one or more projected expression profiles of the plurality of expression profiles in (2).

In some embodiments, the lower dimensional space can be a two dimensional space. Projecting the expression profiles of the plurality of synthetic multiplet expression profiles from the expression profile space into the lower dimensional projection space to generate projected expression profiles of the plurality of synthetic multiplet expression profiles can comprise: projecting the expression profiles of the plurality of synthetic multiplet expression profiles from the expression profile space into the lower dimensional projection space to generate projected expression profiles of the plurality of synthetic multiplet expression profiles using a t-distributed stochastic neighbor embedding (tSNE) method.

### Multiplet Identification using Synthetic Multiplets

Disclosed herein are methods for identifying a multiplet profile. In some embodiments, the method comprises: (a) receiving a plurality of profiles of a plurality of cells; (b) generating a plurality of synthetic multiplet profiles from the plurality of profiles of the plurality of cells; and (c) identifying a profile of the plurality of profiles associated with a cell of the plurality of cells as a singlet or a multiplet based on profiles of the plurality of synthetic multiplet profiles generated in (b). A profile of the plurality of profiles of the plurality of cells can comprise an mRNA expression profile of the cell, a protein expression profile of the cell, a mutation profile of the cell, a methylation profile of the cell, or any combination thereof.

In some embodiments, the mRNA expression profile can comprise an occurrence of mRNA molecules of each gene of a plurality of genes for each cell of the plurality of cells. The occurrence of each gene can comprise an absolute occurrence of the gene, a normalized occurrence of the gene, or a combination thereof. The normalized occurrence of the gene can be determined in a unit of Reads Per Kilobase of transcript per Million mapped reads (RPKM) or a unit of threshold count (Ct). The mRNA expression profile can determined by sequencing, quantitative polymerase chain reaction (qPCR), digital PCR, hybridization, or any combination thereof. In some embodiments, the protein expression profile of the cell comprises an occurrence of protein molecules corresponding to each gene of a plurality of genes for each cell of the plurality of cells. The mutation profile of the cell can comprise a mutation profile of the cell at multiple genome locations of the cell. The methylation profile of the cell can comprise a methylation profile of the cell at multiple genome locations of the cell.

Multiplets can be different in different implementations. In some embodiments, the plurality of multiplets can include a doublet, a triplet, a quartet, a quintet, a sextet, a septet, an octet, a nonet, or any combination thereof. A multiplet can be any n-plet. In some embodiments, n is, or about, 2, 3, 4, 5, 6, 7, 8, 9, 10, 11, 12, 13, 14, 15, 16, 17, 18, 19, 20, or a range between any two of these values. In some embodiments, n is at least, or at most, 2, 3, 4, 5, 6, 7, 8, 9, 10, 11, 12, 13, 14, 15, 16, 17, 18, 19, or 20.

In some embodiments, the method comprises: if the profile is identified as a multiplet in (c), removing the profile from the plurality of profiles received in (a). The plurality of multiplets can comprise a doublet, a triplet, or any combination thereof. In some embodiments, generating the plurality of synthetic multiplet profiles from the plurality of profiles of the plurality of cells comprises: for a synthetic multiplet profile of the plurality of synthetic multiplet profiles, (1) selecting a number of profiles of the plurality of profiles; and (2) combining the profiles selected in (1) to generate the synthetic multiplet profile.

In some embodiments, combining the profiles selected in (1) to generate the synthetic multiplet profile comprises: for each of the plurality of targets, combining values of corresponding elements in the profiles selected to generate a value of a corresponding element in the synthetic multiplet profile. The value of the corresponding element in the synthetic multiplet profile can be a sum of the values of the corresponding elements in the profiles selected. The sum can be a weighted sum of the values of the corresponding elements in the profiles selected. The occurrence of the target in the synthetic multiplet profile can be an average of the values of the corresponding elements in the profiles selected. The average can be a weighted average of the values of the corresponding elements in the profiles selected.

In some embodiments, the number of the plurality of synthetic multiplet profiles is approximately a percentage of the plurality of profiles received in (a). The percentage can be approximately 10 percent. In some embodiments, the percentage can be, or about, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or a range between any two of these values. In some embodiments, the percentage can be at least, or at most, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or a range between any two of these values.

In some embodiments, identifying the profile of the plurality of profiles associated with the cell of the plurality of cells as a singlet or a multiplet based on the profiles of the plurality of synthetic multiplet profiles generated in (b) and the profile comprises: (1) training a machine learning model for profile multiplet identification from the profiles of the plurality of synthetic multiplet profiles generated in (b) and one or more profiles of the plurality of profiles received in (a); and (2) identifying the profile of the plurality of profiles associated with the cell of the plurality of cell as a singlet or a multiplet based on the profile using the machine learning model.

In some embodiments, the one or more profiles of the plurality of profiles used in training the machine learning model comprises a percentage of the plurality of profiles received in (b). The percentage can be approximately 10 percent. In some embodiments, the percentage can be, or about, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or a range between any two of these values. In some embodiments, the percentage can be at least, or at most, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or a range between any two of these values.

In some embodiments, the machine learning model can comprise a classification model. In some embodiments, he classification model comprises a supervised classification model, a semi-supervised classification model, an unsupervised classification model, or a combination thereof. The machine learning model can comprise a neural network, a linear regression model, a logistic regression model, a decision tree, a support vector machine, a Naive Bayes network, a k-nearest neighbors (KNN) model, a k-means model, a random forest model, or any combination thereof. The machine learning model can comprise an association rule learning model, an inductive logic programming model, a reinforcement learning model, a feature learning model, a similarity learning model, a sparse dictionary learning model, a genetic algorithm model, a rule-based machine learning model, a learning classifier system model, or any combination thereof.

In some embodiments, training the machine learning model for profile multiplet identification from the profiles of the plurality of synthetic multiplet profiles generated in (b) and one or more profiles of the plurality of profiles received in (a) comprises: (1) projecting the profiles of the plurality of synthetic multiplet profiles generated in (b) from a profile space into a lower dimensional projection space to generate projected profiles of the plurality of synthetic multiplet profiles; (2) projecting the one or more profiles of the plurality of profiles received in (a) from the profile space into the lower dimensional projection space to generate one or more projected profiles of the plurality of profiles; and (3) training the machine learning model for profile multiplet identification from the projected profiles of the plurality of synthetic multiplet profiles from (1) and the one or more projected profiles of the plurality of profiles in (1).

In some embodiments, the method comprises: projecting the profile of the plurality of the plurality of profiles associated with the cell of the plurality of cell to generate a projected profile of the plurality of profiles, wherein identifying the profile of the plurality of profiles associated with the cell of the plurality of cell as a singlet or a multiplet based on the profile using the machine learning model comprises: identifying the profile of the plurality of profiles associated with the cell of the plurality of cells as a singlet or a multiplet based on the projected profile of the plurality of profiles using the machine learning model. The lower dimensional space can be a two dimensional space. In some embodiments, projecting the profiles of the plurality of synthetic multiplet profiles generated in (b) from the profile space into the lower dimensional projection space to generate the projected profiles of the plurality of synthetic multiplet profiles comprises: projecting the profiles of the plurality of synthetic multiplet profiles generated in (b) from the profile space into the lower dimensional projection space to generate the projected profiles of the plurality of synthetic multiplet profiles comprises using a t-distributed stochastic neighbor embedding (tSNE) method.

**In** some embodiments, identifying the profile of the plurality of profiles associated with the cell of the plurality of cells as a singlet or a multiplet based on the profiles of the plurality of synthetic multiplet profiles generated in (b) and the profile comprises: identifying the profile of the plurality of profiles associated with the cell of the cells as a singlet or a multiplet based on: a first distance between the profile of the plurality of profiles associated with the cell and at least one profile of the plurality of profiles, and a second distance between the profile of the plurality of expression profiles associated with the cell and at least one synthetic multiplet expression profile of the plurality of synthetic multiplet expression profiles.

**In** some embodiments, identifying the profile of the plurality of profiles associated with the cell of the plurality of cells as a singlet or a multiplet based on the profiles of the plurality of synthetic multiplet profiles generated in (b) and the profile comprises: clustering the plurality of profiles into a first cluster of profiles; (2) clustering the plurality of synthetic multiplet profiles into a second cluster of synthetic multiplet profiles; and (3) identifying the profile of the plurality of profiles associated with the cell of the cells as a singlet or a multiplet based on: a first distance between the profile of the plurality of profiles associated with the cells and the first cluster of profiles, and a second distance between the profile of the plurality of profiles associated with the cell and the second cluster of synthetic multiplet profiles.

In some embodiments, identifying the profile of the plurality of profiles associated with the cell of the plurality of cells as a singlet or a multiplet based on the profiles of the plurality of synthetic multiplet profiles generated in (b) and the profile comprises: clustering the plurality of profiles into a first cluster of profiles; (2) clustering the plurality of synthetic multiplet profiles into a second plurality of clusters of synthetic multiplet profiles; and (3) identifying the profile of the plurality of profiles associated with the cell of the cells as a singlet or a multiplet based on: a first distance between the profile of the plurality of profiles associated with the cells and the first cluster of profiles, and second distances between the profile of the plurality of profiles associated with the cell and the second clusters of synthetic multiplet profiles.

### Sequencing

In some embodiments, estimating the number of different barcoded targets (e.g., stochastically barcoded targets) can comprise determining the sequences of the labeled targets, the spatial label, the molecular label, the sample label, the cell label, or any product thereof (e.g., labeled amplicons, or labeled cDNA molecules). An amplified target can be subjected to sequencing. Determining the sequence of the stochastically barcoded target or any product thereof can comprise conducting a sequencing reaction to determine the sequence of at least a portion of a sample label, a spatial label, a cell label, a molecular label, at least a portion of the stochastically labeled target, a complement thereof, a reverse complement thereof, or any combination thereof.

Determination of the sequence of a barcoded target or a stochastically barcoded target (e.g., amplified nucleic acid, labeled nucleic acid, cDNA copy of a labeled nucleic acid, etc.) can be performed using variety of sequencing methods including, but not limited to, sequencing by hybridization (SBH), sequencing by ligation (SBL), quantitative incremental fluorescent nucleotide addition sequencing (QIFNAS), stepwise ligation and cleavage, fluorescence resonance energy transfer (FRET), molecular beacons, TaqMan reporter probe digestion, pyrosequencing, fluorescent in situ sequencing (FISSEQ), FISSEQ beads, wobble sequencing, multiplex sequencing, polymerized colony (POLONY) sequencing; nanogrid rolling circle sequencing (ROLONY), allele-specific oligo ligation assays (e.g., oligo ligation assay (OLA), single template molecule OLA using a ligated linear probe and a rolling circle amplification (RCA) readout, ligated padlock probes, or single template molecule OLA using a ligated circular padlock probe and a rolling circle amplification (RCA) readout), and the like.

In some embodiments, determining the sequence of the barcoded target (e.g., stochastically barcoded target) or any product thereof comprises paired-end sequencing, nanopore sequencing, high-throughput sequencing, shotgun sequencing, dye-terminator sequencing, multiple-primer DNA sequencing, primer walking, Sanger dideoxy sequencing, Maxim-Gilbert sequencing, pyrosequencing, true single molecule sequencing, or any combination thereof. Alternatively, the sequence of the stochastically barcoded target or any product thereof can be determined by electron microscopy or a chemical-sensitive field effect transistor (chemFET) array.

High-throughput sequencing methods, such as cyclic array sequencing using platforms such as Roche 454, Illumina Solexa, ABI-SOLiD, ION Torrent, Complete Genomics, Pacific Bioscience, Helicos, or the Polonator platform, can be utilized. In some embodiment, sequencing can comprise MiSeq sequencing. In some embodiment, sequencing can comprise HiSeq sequencing.

The labeled targets (e.g., stochastically labeled targets) can comprise nucleic acids representing from about 0.01% of the genes of an organism's genome to about 100% of the genes of an organism's genome. For example, about 0.01% of the genes of an organism's genome to about 100% of the genes of an organism's genome can be sequenced using a target complimentary region comprising a plurality of multimers by capturing the genes containing a complimentary sequence from the sample. In some embodiments, the stochastically barcoded targets comprise nucleic acids representing from about 0.01% of the transcripts of an organism's transcriptome to about 100% of the transcripts of an organism's transcriptome. For example, about 0.501% of the transcripts of an organism's transcriptome to about 100% of the transcripts of an organism's transcriptome can be sequenced using a target complimentary region comprising a poly(T) tail by capturing the mRNAs from the sample.

Determining the sequences of the spatial labels and the molecular labels of the plurality of the barcodes (e.g., stochastic barcodes) can include sequencing 0.00001%, 0.0001%, 0.001%, 0.01%, 0.1%, 1%, 2%, 3%, 4%, 5%, 6%, 7%, 8%, 9%, 10%, 20%, 30%, 40%, 50%, 60%, 70%, 80%, 90%, 99%, 100%, or a number or a range between any two of these values, of the plurality of stochastic barcodes. Determining the sequences of the labels of the plurality of stochastic barcodes, for example the sample labels, the spatial labels, and the molecular labels, can include sequencing 1, 10, 20, 30, 40, 50, 60, 70, 80, 90, 100, 10³, 10⁴, 10⁵, 10⁶, 10⁷, 10⁸, 10⁹, 10¹⁰, 10¹¹, 10¹², 10¹³, 10¹⁴, 10¹⁵, 10¹⁶, 10¹⁷, 10¹⁸, 10¹⁹, 10²⁰, or a number or a range between any two of these values, of the plurality of stochastic barcodes. Sequencing some or all of the plurality of stochastic barcodes can include generating sequences with read lengths of, of about, of at least, or of at most, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, 10000, or a number or a range between any two of these values, of nucleotides or bases.

Sequencing can comprise sequencing at least or at least about 10, 20, 30, 40, 50, 60, 70, 80, 90, 100 or more nucleotides or base pairs of the barcoded targets (e.g., stochastically barcoded targets). For example, sequencing can comprise generating sequencing data with sequences with read lengths of 50, 75, or 100, or more nucleotides by performing polymerase chain reaction (PCR) amplification on the plurality of stochastically barcoded targets. Sequencing can comprise sequencing at least or at least about 200, 300, 400, 500, 600, 700, 800, 900, 1,000 or more nucleotides or base pairs of the stochastically barcoded targets. Sequencing can comprise sequencing at least or at least about 1500, 2000, 3000, 4000, 5000, 6000, 7000, 8000, 9000, or 10000 or more nucleotides or base pairs of the stochastically barcoded targets.

Sequencing can comprise at least about 200, 300, 400, 500, 600, 700, 800, 900, 1,000 or more sequencing reads per run. In some embodiments, sequencing comprises sequencing at least or at least about 1500, 2000, 3000, 4000, 5000, 6000, 7000, 8000, 9000, or 10000 or more sequencing reads per run. Sequencing can comprise less than or equal to about 1,600,000,000 sequencing reads per run. Sequencing can comprise less than or equal to about 200,000,000 reads per run.

### Samples

In some embodiments, the plurality of targets can be comprised in one or more samples. A sample can comprise one or more cells, or nucleic acids from one or more cells. A sample can be a single cell or nucleic acids from a single cell. The one or more cells can be of one or more cell types. At least one of the one or more cell types can be brain cell, heart cell, cancer cell, circulating tumor cell, organ cell, epithelial cell, metastatic cell, benign cell, primary cell, circulatory cell, or any combination thereof.

A sample for use in the method of the disclosure can comprise one or more cells. A sample can refer to one or more cells. In some embodiments, the plurality of cells can include one or more cell types. At least one of the one or more cell types can be brain cell, heart cell, cancer cell, circulating tumor cell, organ cell, epithelial cell, metastatic cell, benign cell, primary cell, circulatory cell, or any combination thereof. In some embodiments, the cells are cancer cells excised from a cancerous tissue, for example, breast cancer, lung cancer, colon cancer, prostate cancer, ovarian cancer, pancreatic cancer, brain cancer, melanoma and non-melanoma skin cancers, and the like. In some embodiments, the cells are derived from a cancer but collected from a bodily fluid (e.g., circulating tumor cells). Non-limiting examples of cancers can include, adenoma, adenocarcinoma, squamous cell carcinoma, basal cell carcinoma, small cell carcinoma, large cell undifferentiated carcinoma, chondrosarcoma, and fibrosarcoma. The sample can include a tissue, a cell monolayer, fixed cells, a tissue section, or any combination thereof. The sample can include a biological sample, a clinical sample, an environmental sample, a biological fluid, a tissue, or a cell from a subject. The sample can be obtained from a human, a mammal, a dog, a rat, a mouse, a fish, a fly, a worm, a plant, a fungus, a bacterium, a virus, a vertebrate, or an invertebrate.

In some embodiments, the cells are cells that have been infected with virus and contain viral oligonucleotides. In some embodiments, the viral infection can be caused by a virus such as single-stranded (+ strand or "sense") DNA viruses (e.g., parvoviruses), or double-stranded RNA viruses (e.g., reoviruses). In some embodiments, the cells are bacteria. These can include either gram-positive or gram-negative bacteria. In some embodiments, the cells are fungi. In some embodiments, the cells are protozoans or other parasites.

As used herein, the term "cell" can refer to one or more cells. In some embodiments, the cells are normal cells, for example, human cells in different stages of development, or human cells from different organs or tissue types. In some embodiments, the cells are non-human cells, for example, other types of mammalian cells (e.g., mouse, rat, pig, dog, cow, and horse). In some embodiments, the cells are other types of animal or plant cells. In other embodiments, the cells can be any prokaryotic or eukaryotic cells.

In some embodiments, the cells are sorted prior to associating a cell with a bead. For example, the cells can be sorted by fluorescence-activated cell sorting or magnetic-activated cell sorting, or more generally by flow cytometry. The cells can be filtered by size. In some embodiments, a retentate contains the cells to be associated with the bead. In some embodiments, the flow through contains the cells to be associated with the bead.

A sample can refer to a plurality of cells. The sample can refer to a monolayer of cells. The sample can refer to a thin section (e.g., tissue thin section). The sample can refer to a solid or semi-solid collection of cells that can be place in one dimension on an array.

### Data Analysis and Display Software

### Data Analysis and Visualization of Spatial Resolution of Targets

The disclosure relates to methods for estimating the number and position of targets with barcoding (e.g., stochastic barcoding) and digital counting using spatial labels. The data obtained from the methods of the disclosure can be visualized on a map. A map of the number and location of targets from a sample can be constructed using information generated using the methods described herein. The map can be used to locate a physical location of a target. The map can be used to identify the location of multiple targets. The multiple targets can be the same species of target, or the multiple targets can be multiple different targets. For example, a map of a brain can be constructed to show the digital count and location of multiple targets.

The map can be generated from data from a single sample. The map can be constructed using data from multiple samples, thereby generating a combined map. The map can be constructed with data from tens, hundreds, and/or thousands of samples. A map constructed from multiple samples can show a distribution of digital counts of targets associated with regions common to the multiple samples. For example, replicated assays can be displayed on the same map. At least 1, 2, 3, 4, 5, 6, 7, 8, 9, or 10, or more replicates can be displayed (e.g., overlaid) on the same map. At most 1, 2, 3, 4, 5, 6, 7, 8, 9, or 10, or more replicates can be displayed (e.g., overlaid) on the same map. The spatial distribution and number of targets can be represented by a variety of statistics.

Combining data from multiple samples can increase the locational resolution of the combined map. The orientation of multiple samples can be registered by common landmarks, wherein the individual locational measurements across samples are at least in part non-contiguous. A particular example is sectioning a sample using a microtome on one axis and then sectioning a second sample along a different access. The combined dataset will give three dimensional spatial locations associated with digital counts of targets. Multiplexing the above approach will allow for high resolution three dimensional maps of digital counting statistics.

In some embodiments of the instrument system, the system will comprise computer-readable media that includes code for providing data analysis for the sequence datasets generated by performing single cell, barcoding assays (e.g., stochastic barcoding assays). Examples of data analysis functionality that can be provided by the data analysis software include, but are not limited to, (i) algorithms for decoding/demultiplexing of the sample label, cell label, spatial label, and molecular label, and target sequence data provided by sequencing the stochastic barcode library created in running the assay, (ii) algorithms for determining the number of reads per gene per cell, and the number of unique transcript molecules per gene per cell, based on the data, and creating summary tables, (iii) statistical analysis of the sequence data, e.g., for clustering of cells by gene expression data, or for predicting confidence intervals for determinations of the number of transcript molecules per gene per cell, etc., (iv) algorithms for identifying sub-populations of rare cells, for example, using principal component analysis, hierarchical clustering, k-mean clustering, self-organizing maps, neural networks etc., (v) sequence alignment capabilities for alignment of gene sequence data with known reference sequences and detection of mutation, polymorphic markers and splice variants, and (vi) automated clustering of molecular labels to compensate for amplification or sequencing errors. In some embodiments, commercially-available software can be used to perform all or a portion of the data analysis, for example, the Seven Bridges (https://www.sbgenomics.com/) software can be used to compile tables of the number of copies of one or more genes occurring in each cell for the entire collection of cells. In some embodiments, the data analysis software can include options for outputting the sequencing results in useful graphical formats, e.g., heatmaps that indicate the number of copies of one or more genes occurring in each cell of a collection of cells. In some embodiments, the data analysis software can further comprise algorithms for extracting biological meaning from the sequencing results, for example, by correlating the number of copies of one or more genes occurring in each cell of a collection of cells with a type of cell, a type of rare cell, or a cell derived from a subject having a specific disease or condition. In some embodiment, the data analysis software can further comprise algorithms for comparing populations of cells across different biological samples.

In some embodiments, all of the data analysis functionality can be packaged within a single software package. In some embodiments, the complete set of data analysis capabilities can comprise a suite of software packages. In some embodiments, the data analysis software can be a standalone package that is made available to users independently of the assay instrument system. In some embodiments, the software can be web-based, and can allow users to share data.

In some embodiments, all of the data analysis functionality can be packaged within a single software package. In some embodiments, the complete set of data analysis capabilities can comprise a suite of software packages. In some embodiments, the data analysis software can be a standalone package that is made available to users independently of the assay instrument system. In some embodiments, the software can be web-based, and can allow users to share data.

### System Processors and Networks

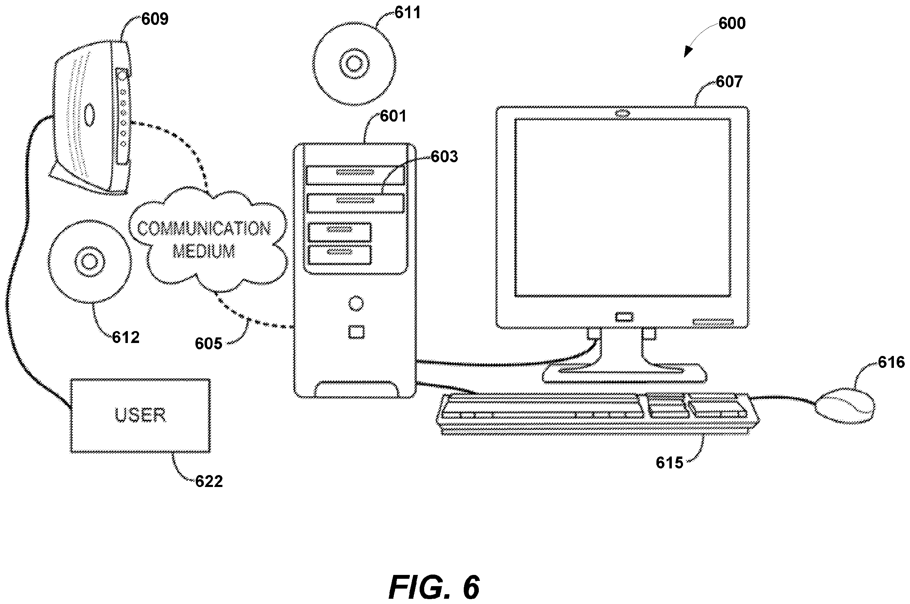

In general, the computer or processor suitable for use in the methods of the presently disclosed instrument systems, as illustrated in FIG. 6, can be further understood as a logical apparatus that can read instructions from media 611 or a network port 605, which can optionally be connected to server 609 having fixed media 612. The system 600, such as shown in FIG. 6 can include a CPU 601, disk drives 603, optional input devices such as keyboard 615 or mouse 616 and optional monitor 607. Data communication can be achieved through the indicated communication medium to a server at a local or a remote location. The communication medium can include any means of transmitting or receiving data. For example, the communication medium can be a network connection, a wireless connection or an internet connection. Such a connection can provide for communication over the World Wide Web. It is envisioned that data relating to the present disclosure can be transmitted over such networks or connections for reception or review by a party 622 as illustrated in FIG. 6.

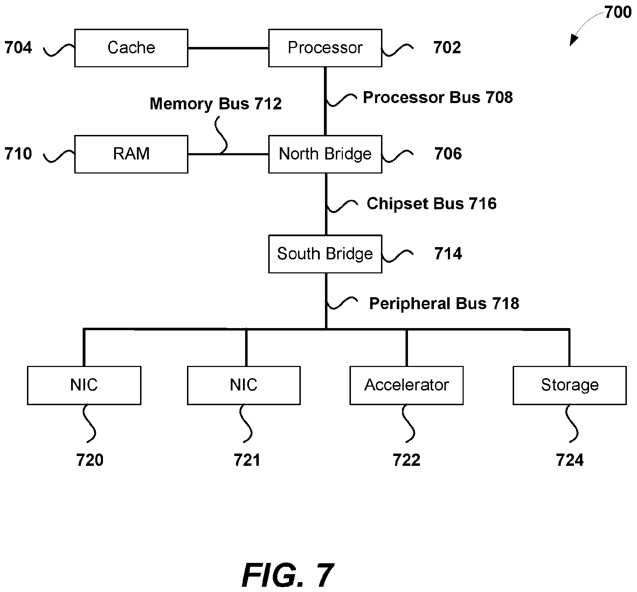

FIG. 7 illustrates an exemplary embodiment of a first example architecture of a computer system 700 that can be used in connection with example embodiments of the present disclosure. As depicted in FIG. 7, the example computer system can include a processor 702 for processing instructions. Non-limiting examples of processors include: Intel XeonTM processor, AMD Opteron^{™} processor, Samsung 32-bit RISC ARM 1176JZ(F)-S v1.0^{™} processor, ARM Cortex-A8 Samsung SSPC100TM processor, ARM Cortex-A8 Apple A4TM processor, Marvell PXA 930TM processor, or a functionally-equivalent processor. Multiple threads of execution can be used for parallel processing. In some embodiments, multiple processors or processors with multiple cores can be used, whether in a single computer system, in a cluster, or distributed across systems over a network comprising a plurality of computers, cell phones, or personal data assistant devices.

As illustrated in FIG. 7, a high speed cache 704 can be connected to, or incorporated in, the processor 702 to provide a high speed memory for instructions or data that have been recently, or are frequently, used by processor 702. The processor 702 is connected to a north bridge 706 by a processor bus 708. The north bridge 706 is connected to random access memory (RAM) 710 by a memory bus 712 and manages access to the RAM 710 by the processor 702. The north bridge 706 is also connected to a south bridge 714 by a chipset bus 716. The south bridge 714 is, in turn, connected to a peripheral bus 718. The peripheral bus can be, for example, PCI, PCI-X, PCI Express, or other peripheral bus. The north bridge and south bridge are often referred to as a processor chipset and manage data transfer between the processor, RAM, and peripheral components on the peripheral bus 718. In some alternative architectures, the functionality of the north bridge can be incorporated into the processor instead of using a separate north bridge chip.

In some embodiments, system 700 can include an accelerator card 722 attached to the peripheral bus 718. The accelerator can include field programmable gate arrays (FPGAs) or other hardware for accelerating certain processing. For example, an accelerator can be used for adaptive data restructuring or to evaluate algebraic expressions used in extended set processing.

Software and data are stored in external storage 724 and can be loaded into RAM 710 or cache 704 for use by the processor. The system 700 includes an operating system for managing system resources; non-limiting examples of operating systems include: Linux, Windows^{™}, MACOS^{™}, BlackBerry OS^{™}, iOS^{™}, and other functionally-equivalent operating systems, as well as application software running on top of the operating system for managing data storage and optimization.

In this example, system 700 also includes network interface cards (NICs) 720 and 721 connected to the peripheral bus for providing network interfaces to external storage, such as Network Attached Storage (NAS) and other computer systems that can be used for distributed parallel processing.

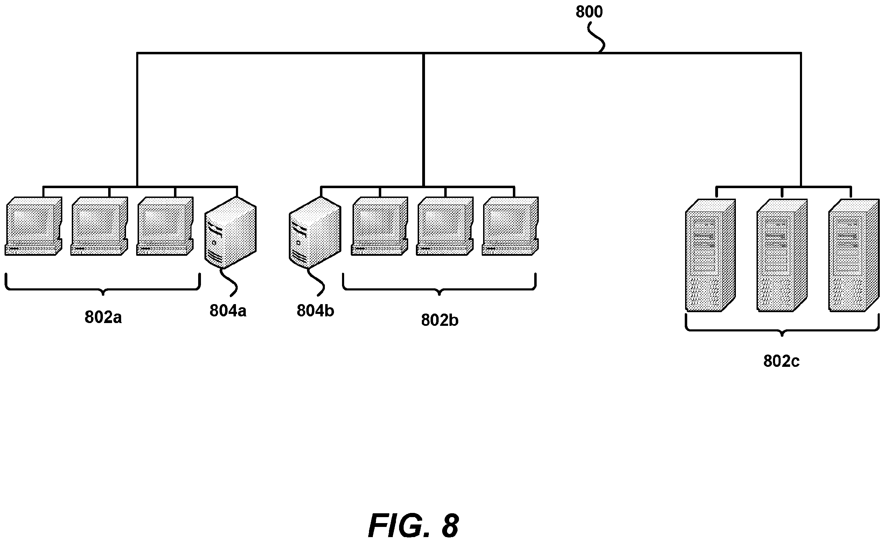

FIG. 8 illustrates an exemplary diagram showing a network 800 with a plurality of computer systems 802a, and 802b, a plurality of cell phones and personal data assistants 802c, and Network Attached Storage (NAS) 804a, and 804b suitable for use in the methods of the disclosure. In example embodiments, systems 812a, 812b, and 812c can manage data storage and optimize data access for data stored in Network Attached Storage (NAS) 814a and 814b. A mathematical model can be used for the data and be evaluated using distributed parallel processing across computer systems 812a, and 812b, and cell phone and personal data assistant systems 812c. Computer systems 812a, and 812b, and cell phone and personal data assistant systems 812c can provide parallel processing for adaptive data restructuring of the data stored in Network Attached Storage (NAS) 814a and 814b. FIG. 8 illustrates an example only, and a wide variety of other computer architectures and systems can be used in conjunction with the various embodiments of the present invention. For example, a blade server can be used to provide parallel processing. Processor blades can be connected through a back plane to provide parallel processing. Storage can be connected to the back plane or as Network Attached Storage (NAS) through a separate network interface.

In some example embodiments, processors can maintain separate memory spaces and transmit data through network interfaces, back plane or other connectors for parallel processing by other processors. In other embodiments, some or all of the processors can use a shared virtual address memory space.

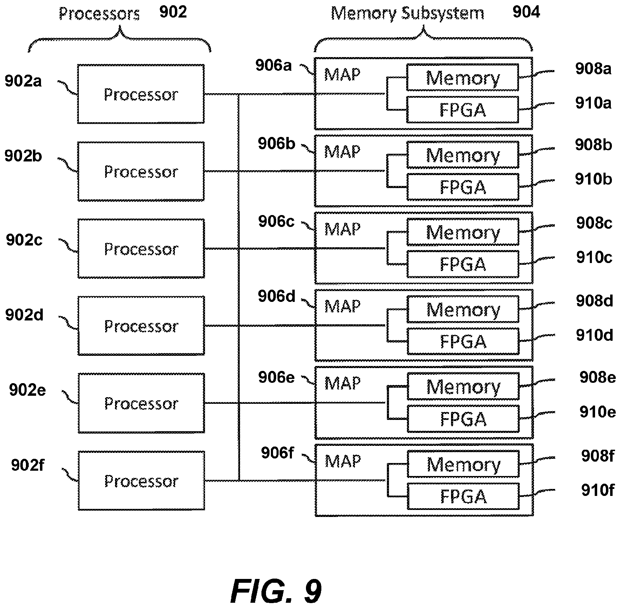

FIG. 9 illustrates an exemplary a block diagram of a multiprocessor computer system 900 using a shared virtual address memory space in accordance with an example embodiment. The system includes a plurality of processors 902a-f that can access a shared memory subsystem 904. The system incorporates a plurality of programmable hardware memory algorithm processors (MAPs) 906a-f in the memory subsystem 904. Each MAP 906a-f can comprise a memory 908a-f and one or more field programmable gate arrays (FPGAs) 910a-f. The MAP provides a configurable functional unit and particular algorithms or portions of algorithms can be provided to the FPGAs 910a-f for processing in close coordination with a respective processor. For example, the MAPs can be used to evaluate algebraic expressions regarding the data model and to perform adaptive data restructuring in example embodiments. In this example, each MAP is globally accessible by all of the processors for these purposes. In one configuration, each MAP can use Direct Memory Access (DMA) to access an associated memory 908a-f, allowing it to execute tasks independently of, and asynchronously from, the respective microprocessor 902a-f. In this configuration, a MAP can feed results directly to another MAP for pipelining and parallel execution of algorithms.

The above computer architectures and systems are examples only, and a wide variety of other computer, cell phone, and personal data assistant architectures and systems can be used in connection with example embodiments, including systems using any combination of general processors, co-processors, FPGAs and other programmable logic devices, system on chips (SOCs), application specific integrated circuits (ASICs), and other processing and logic elements. In some embodiments, all or part of the computer system can be implemented in software or hardware. Any variety of data storage media can be used in connection with example embodiments, including random access memory, hard drives, flash memory, tape drives, disk arrays, Network Attached Storage (NAS) and other local or distributed data storage devices and systems.

In example embodiments, the computer subsystem of the present disclosure can be implemented using software modules executing on any of the above or other computer architectures and systems. In other embodiments, the functions of the system can be implemented partially or completely in firmware, programmable logic devices such as field programmable gate arrays (FPGAs), system on chips (SOLs), application specific integrated circuits (ASICs), or other processing and logic elements. For example, the Set Processor and Optimizer can be implemented with hardware acceleration through the use of a hardware accelerator card, such as accelerator card.

### EXAMPLES

Some aspects of the embodiments discussed above are disclosed in further detail in the following examples, which are not in any way intended to limit the scope of the present disclosure.

### Example 1

### Synthetic Doublet Expression Profiles of 1:1 Mixed Cell Types

This example describes determining expression profiles of 1:1 mixed cell types in a sample as doublet expression profiles using synthetic doublet expression profiles.

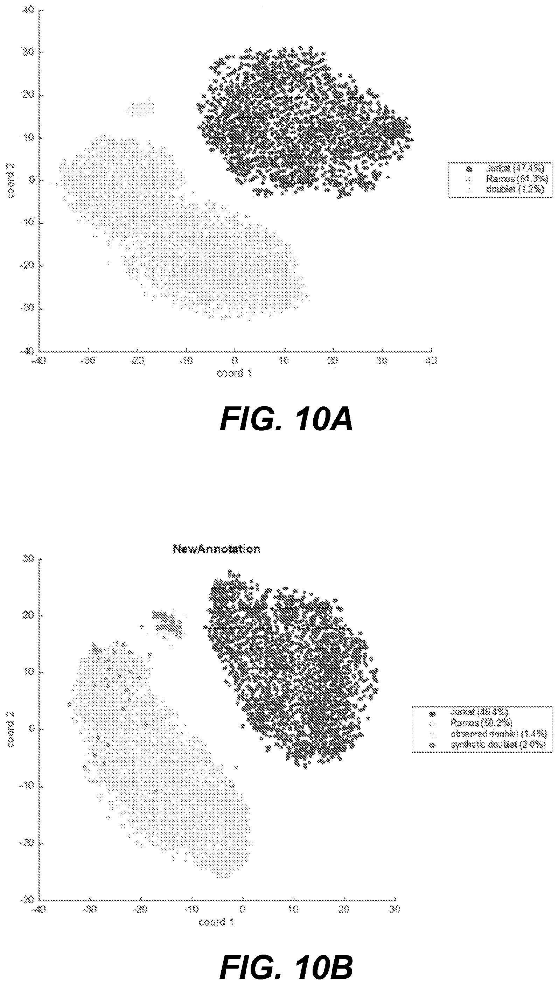

Expression profiles of single Jurkat cells and Ramos cells were determined. The sample contained approximately equal numbers of Jurkat cells and Ramos cells (also referred to as 1:1 mixed cell types). The expression profiles were in three clusters in the expression profile space: a cluster corresponding to expression profiles of Jurkat cells, a cluster corresponding to expression profiles of Ramos cells, and a cluster corresponding to doublet expression profiles of mixed cell types. Each doublet expression profile may have been caused by two cells being identified as one cell when determining expression profiles of single cells. Visualization of these three clusters is shown in FIG. 10A. FIG. 10A shows a non-limiting exemplary tSNE projection plot of expression profiles of single cells in the sample comprising approximately equal numbers of Jurkat cells and Ramos cells. The tSNE projection plot in FIG. 10A includes a cluster corresponding to expression profiles of Jurkat cells, a cluster corresponding to expression profiles of Ramos cells, and a cluster corresponding to doublet expression profiles of mixed cell types.

A synthetic doublet expression profile was generated by combining observed expression profiles of two cells. By randomly sampling pairs of cells multiple times, a plurality of synthetic doublet expression profiles were generated. Visualization of the plurality of synthetic doublet expression profiles is shown in FIG 10B. FIG. 10B shows a non-limiting exemplary tSNE projection plot of the expression profiles in FIG. 10A and 2% of synthetic doublet expression profiles. Some synthetic doublet expression profiles represented mixed cell types (Jurkat cells and Ramos cells). As shown in FIG. 10B, a cluster corresponding to these synthetic doublet expression profiles overlaps the cluster corresponding to the doublet expression profiles in FIG. 10A. Some synthetic doublet expression profiles represented single cell types (Jurkat cells or Ramos cells). A synthetic doublet expression profile generated from expression profiles of two cells of the Jurkat cell type appear as an expression profile of a Jurkat cell in FIG. 10. Similarly, a synthetic doublet expression profile generated from expression profiles of two cells of the Ramos cell type appear as an expression profile of a Ramos cell in FIG. 10.

Altogether, these data demonstrate that doublet expression profiles of cells of a 1:1 mixed cell types were identified using synthetic doublet expression profiles because of their close proximities in the expression profile space.

### Example 2

### Synthetic Doublet Expression Profiles of 1:1:1 Mixed Cell Types

This example describes determining expression profiles of 1:1:1 mixed cell types in a sample as doublet expression profiles using synthetic doublet expression profiles.

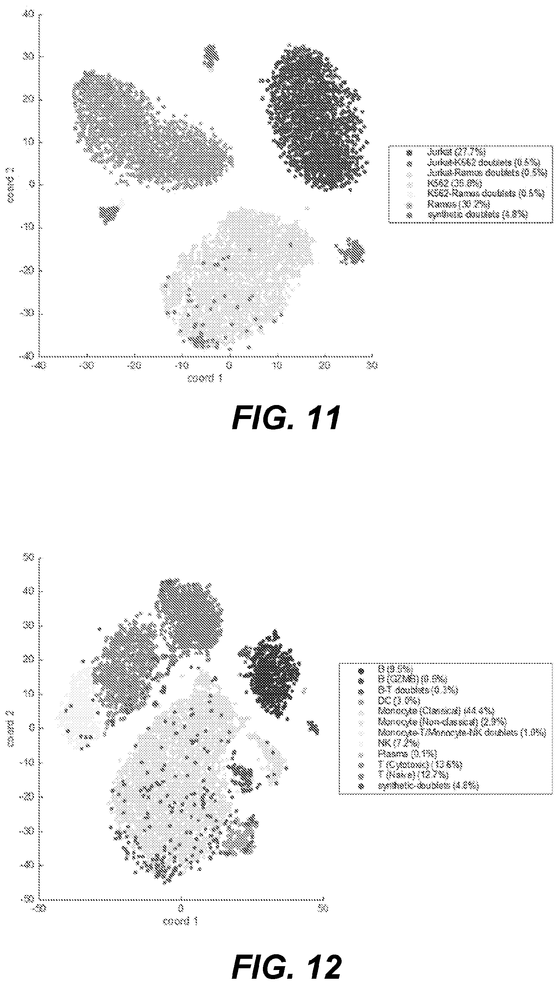

Expression profiles of single Jurkat cells and Ramos cells were determined. The sample contained approximately equal numbers of Jurkat cells, K562 cells, and Ramos cells (also referred to as 1:1:1 mixed cell types). The expression profiles were in six clusters in the expression profile space: a cluster corresponding to expression profiles of Jurkat cells, a cluster corresponding to expression profiles of K562 cells, a cluster corresponding to expression profiles of Ramos cells, and three clusters corresponding to doublet expression profiles of mixed cell types. The three clusters correspond to doublet expression profiles of Jurkat cells and K562 cells, Jurkat cells and Ramos cells, and K562 cells and Ramos cells. Visualization of these three clusters is shown in FIG. 11. FIG. 11 shows a non-limiting exemplary tSNE projection plot of expression profiles of single cells in the sample comprising approximately equal numbers of Jurkat cells, K562 cells, and Ramos cells.

A synthetic doublet expression profile was generated by summing observed expression profiles of two cells. By randomly sampling pairs of cells multiple times, a plurality of synthetic doublet expression profiles were generated. There were three types of synthetic doublet expression profiles: an expression profile generated from expression profiles of a single Jurkat cell and a single K562 cell, an expression profile generated from expression profiles of a single Jurkat cell and a single Ramos cell, and an expression profile generated from expression profiles of a single K562 cell and a single Ramos cell. Visualization of the plurality of synthetic doublet expression profiles is also shown in FIG. 11. In FIG. 11, the three clusters corresponding to the three types of synthetic doublet expression profiles overlap the clusters corresponding to doublet expression profiles of mixed cell types observed.

Altogether, these data demonstrate that doublet expression profiles of cells of a 1:1:1 mixed cell types were identified using synthetic doublet expression profiles because of their close proximities in the expression profile space.

### Example 3

### Synthetic Doublet Expression Profiles of Cells in a Human PBMC Sample

This example describes determining expression profiles of cells in a human peripheral blood mononuclear cell (PBMC) sample as doublet expression profiles using synthetic doublet expression profiles.

Expression profiles of single cells in a human PBMC sample were determined. The expression profiles were in multiple clusters in the expression profile space, including clusters corresponding to different types of doublet expression profiles. A cluster corresponding to a type of doublet expression profile and a corresponding cluster of synthetic doublet expression profiles were in close proximity in the expression profile space. Visualization of these clusters in the lower dimensional tSNE projection space is shown in FIG. 12. FIG. 12 shows a non-limiting exemplary tSNE projection plot of expression profiles obtained from the human PBMC sample. Because of the diversity of cells in certain samples, such as the human PBMC sample, the close proximity of a cluster corresponding to a type of doublet expression profile and a corresponding cluster of synthetic doublet expression profiles may not be easily visualized in the tSNE projection plot of expression profiles in FIG. 12. The closer proximity of the clusters may be visualized in a different lower dimensional projection space.

Altogether, these data demonstrate that doublet expression profiles of diverse cells may be identified using synthetic doublet expression profiles in a higher dimensional expression profile space or an appropriate lower dimensional projection space.

### Example 4

### Synthetic Doublet Expression Profiles of 12 Samples of Mouse Cells

This example describes determining expression profiles of cells from 12 mouse cell samples as singlet expression profiles and multiplet expression profiles using synthetic multiplet expression profiles.

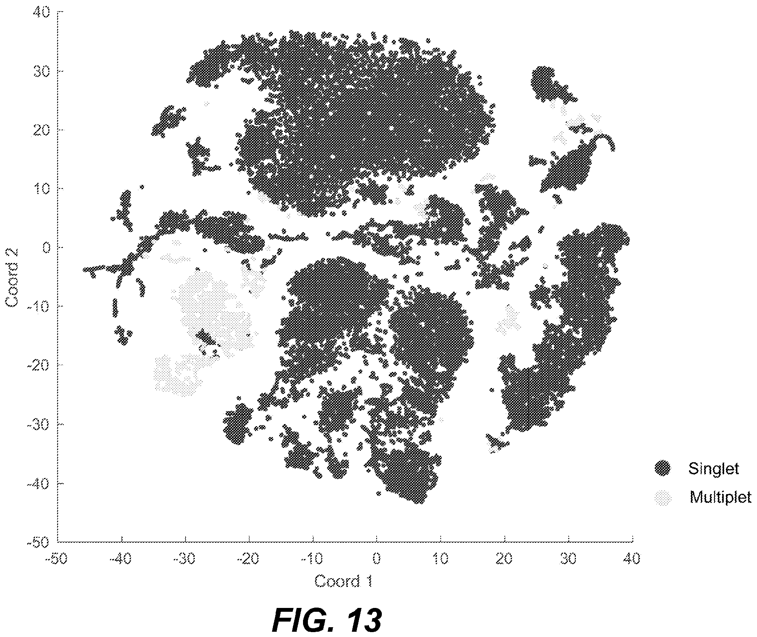

Six tissues (bone marrow, fat (gonadal white adipose tissue (gWAT)), colon, liver, lung, and spleen) from two mice were isolated. CD45+ single cells from the isolated tissues were isolated and sorted using fluorescence-activated cell sorting (FACS) to create 12 samples. CD45+ single cells of the six tissues from each mouse were tagged with six different nucleic acid sample tags using BD^{™} Single-Cell Multiplexing Kit for RNA-Sequencing and loaded onto a Rhapsody^{™} cartridge. The mRNA molecules from the cells were captured using Rhapsody^{™} magnetic beads, and barcoded and amplified using Rhapsody^{™} Immune Response Panel-Mouse (Mouse). Expression profiles of the cells were determined and identified as singlet expression profiles and multiplet expression profiles using synthetic multiplet expression profiles. FIG. 13 is a non-limiting exemplary tSNE projection plot of expression profiles of the CD45+ single cells from the 12 samples of six tissues from two mice. FIG. 13 shows the multiplet expression profiles as multiple clusters. Synthetic multiplet expression profiles were used to identify multiplet expression profiles each including the expression profiles of cells of two or more cell types or subtypes. Multiplets of CD45+ cells from two different tissues were identified based on the presence of the sample tag sequences in the sequence data obtained from the cells. The performance of using synthetic multiplet expression profiles to identify multiplets was comparable to the performance of using nucleic acid sample tags to index samples and identify multiplets. Furthermore, additional expression profiles were identified as multiplets using synthetic multiplet expression profiles. An expression profile identified as a multiplet using synthetic multiplet expression profile, not sample tags, may include the expression profiles of two or more cells of two of more cell types or subtypes from one tissue, which could be tagged using one sample tag sequence in this example.

Altogether, these data demonstrate that multiplet expression profiles of cells from different tissues, or of different cell types or subtypes, can be identified using synthetic multiplet expression profiles.

### Example 5

### Generating, Visualizing, and Removing Synthetic Doublet Expression Profiles

This example describes a workflow of generating, visualizing, and removing synthetic doublet expression profiles manually.

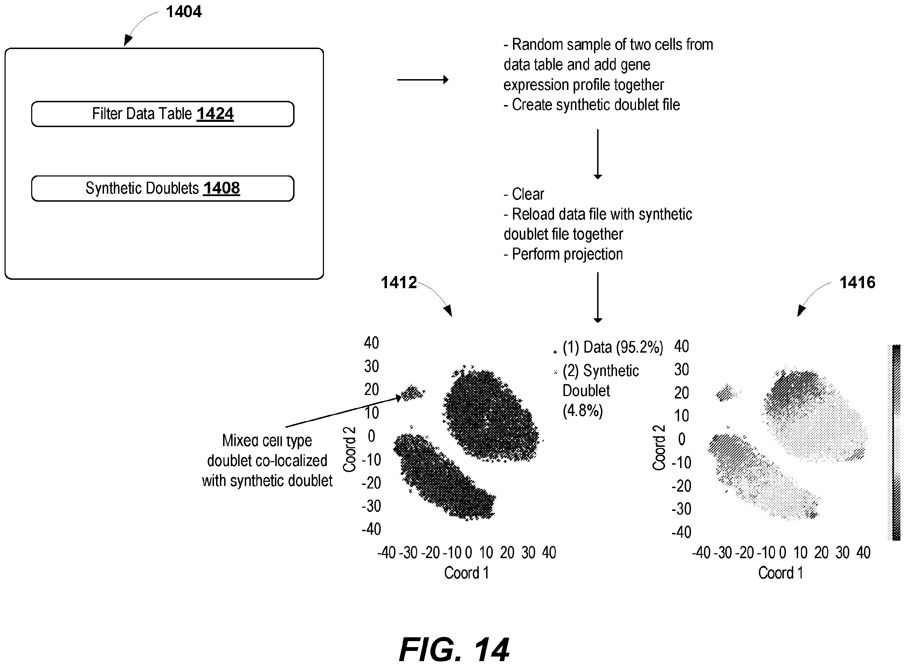

A workflow for generating, visualizing, and removing synthetic doublet expression profiles which included the following steps were performed. The workflow is illustrated with reference to FIG. 14, which shows a non-limiting exemplary workflow of generating, visualizing, and removing synthetic doublet expression profiles using a non-limiting exemplary user interface 1404 of, for example, BD^{™} (Franklin Lake, NJ) Data View.

Step 1. Load a data file containing expression profiles.

Step 2. Generate a synthetic doublet file containing synthetic doublet expression profiles by, for example, selecting the "Synthetic Doublets" button 1408. To generate the synthetic doublet expression profiles, randomly sample pairs of expression profiles in the data file loaded and sum the two expression profiles in each pair. Save the synthetic doublet expression profiles as the synthetic doublet file.

Step 3. Clear and reload the data file and the synthetic doublet file.

Step 4. Generate one or more projection plots 1412, 1416, showing the expression profiles and the synthetic doublet expression profiles as two different colors or with heatmap coloring.

Step 5. Annotate the plot(s) based on the origin of expression profiles (from the data file or form the synthetic doublet file).

Step 6. Use "Add Annotation" and the "Draw" options to circle the clusters containing a large number of synthetic doublet expression profiles. Annotate these as doublets.

Step 7. Use "Add Annotation" and "Combine Existing Annotation" option to create a new annotation with four groups based on the origin of expression profiles and the "doublet" annotations. The four groups correspond to: the expression profiles from the data file not annotated as "doublets;" the expression profiles from the data file annotated as "doublets;" the expression profiles from the synthetic doublet data file not annotated as "doublets;" and the expression profiles from the synthetic doublet data file annotated as "doublets."

Step 8. Highlight this new annotation. Filter the expression profiles from the data file, e.g., by selecting the "Filter Expression Profiles" button 1420. Choose the option to filter cells based on the currently highlighted annotation. Select the three groups to remove: the expression profiles from the data file not annotated as "doublets;" the expression profiles from the data file annotated as "doublets;" and the expression profiles from the synthetic doublet data file annotated as "doublets."

Step 9. Save the remaining expression profiles as a new data file.

Altogether, these data demonstrate generating, visualizing, and removing synthetic doublet expression profiles manually using one or more tSNE projection plot.

In at least some of the previously described embodiments, one or more elements used in an embodiment can interchangeably be used in another embodiment unless such a replacement is not technically feasible. It will be appreciated by those skilled in the art that various other omissions, additions and modifications may be made to the methods and structures described above without departing from the scope of the claimed subject matter..

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. As used in this specification and the appended claims, the singular forms "a," "an," and "the" include plural references unless the context clearly dictates otherwise. Any reference to "or" herein is intended to encompass "and/or" unless otherwise stated.

It will be understood by those within the art that, in general, terms used herein, and especially in the appended claims (*e.g.,* bodies of the appended claims) are generally intended as "open" terms (*e.g*., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (*e.g.,* "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (*e.g*., the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (*e.g*., " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (*e.g.,* " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

While various aspects and embodiments have been disclosed herein, other aspects and embodiments will be apparent to those skilled in the art.

## Claims

1. A method for identifying a multiplet expression profile in a plurality of expression profiles determined from sequencing data of a plurality of stochastically barcoded targets, and removing the multiplet expression profile from the plurality of expression profiled or removing the sequencing data associated with the multiplet expression profile, comprising:
(a) obtaining sequencing data of a plurality of stochastically barcoded targets, wherein the plurality of stochastically barcoded targets were generated by stochastically barcoding a plurality of targets in a plurality of cells using a plurality of stochastic barcodes to create a plurality of stochastically barcoded targets for each cell of the plurality of cells, wherein each of the plurality of stochastic barcodes comprises a cell label and a molecular label, wherein molecular labels of at least two stochastic barcodes of the plurality of stochastic barcodes comprise different molecular label sequences, and wherein at least two stochastic barcodes of the plurality of stochastic barcodes comprise cell labels with an identical cell label sequence;
(c) determining a plurality of expression profiles associated with cell labels of the plurality of stochastic barcodes from the sequencing data obtained in (b), wherein an expression profile of the plurality of expression profiles associated with a cell label of the cell labels of the plurality of stochastic barcodes comprises a number of molecular labels with distinct sequences associated with the cell label and each target of the plurality of targets in the sequencing data;
(d) generating a plurality of synthetic multiplet expression profiles from the plurality of expression profiles associated with the cell labels of the plurality of stochastic barcodes determined in (c), comprising for a synthetic multiplet expression profile of the plurality of synthetic multiplet expression profiles:
(1) selecting a first expression profile of the plurality of expression profiles determined in (c), wherein the first expression profile is associated with a first cell label sequence;
(2) selecting a second expression profile of the plurality of expression profiles determined in (c), wherein the second expression profile is associated with a second cell label sequence, and wherein the first cell label sequence and the second cell label sequence comprise different cell label sequences; and
(3) combining the first expression profile selected in (1) and the second expression profile selected in (2) to generate the synthetic multiplet expression profile; and
(e) identifying an expression profile of the plurality of expression profiles associated with a cell label of the cell labels of the plurality of stochastic barcodes as a singlet or a multiplet based on plurality of synthetic doublet expression profiles generated in (d); and
(f) if the expression profile is identified as a multiplet in (e):
(1) removing sequencing data associated with the expression profile from the sequencing data obtained in (b); or
(2) removing the expression profile from the plurality of expression profiles determined in (c).

2. The method of claim 1, wherein the plurality of multiplets comprises a doublet, a triplet, or any combination thereof.

3. The method of claim 1 or claim 2, wherein combining the first expression profile selected in (1) and the second expression profile selected in (2) to generate the synthetic multiplet expression profile comprises: for each of the plurality of targets, combining a number of molecular labels with distinct sequences associated with the target in the first expression profile and a number of molecular labels with distinct sequences associated with the target in the second expression profile to generate a number of molecular labels with distinct sequences associated with the synthetic multiplet expression profile, optionally wherein:
(a) the number of molecular labels with distinct sequences associated with the synthetic multiplet expression profile is a sum of the number of molecular labels with distinct sequences associated with the target in the first expression profile and the number of molecular labels with distinct sequences associated with the target in the second expression profile, optionally wherein the sum is a weighted sum of the number of molecular labels with distinct sequences associated with the target in the first expression profile and the number of molecular labels with distinct sequences associated with the target in the second expression profile; or
(b) the number of molecular labels with distinct sequences associated with the synthetic multiplet expression profile is an average of the number of molecular labels with distinct sequences associated with the target in the first expression profile and the number of molecular labels with distinct sequences associated with the target in the second expression profile, optionally wherein the average is a weighted average of the number of molecular labels with distinct sequences associated with the target in the first expression profile and the number of molecular labels with distinct sequences associated with the target in the second expression profile.

4. The method of any one of claims 1-3, wherein identifying the expression profile of the plurality of expression profiles associated with the cell label of the cell labels of the plurality of stochastic barcodes as a singlet or a multiplet based on the expression profiles of the plurality of synthetic multiplet expression profiles generated comprises:
(1) training a machine learning model for expression profile multiplet identification from the expression profiles of the plurality of synthetic multiplet expression profiles generated in (d) and one or more expression profiles of the plurality of expression profiles determined in (c); and
(2) identifying the expression profile of the plurality of expression profiles associated with the cell label of the cell labels of the plurality of stochastic barcodes as a singlet or a multiplet based on the expression profile using the machine learning model.

5. The method of claim 4, wherein:
(a) the one or more expression profiles of the plurality of expression profiles used in training the machine learning model comprises a percentage of the plurality of expression profiles determined in (c), optionally wherein the percentage is in the range of 1-50%, preferably wherein the percentage is approximately 10 percent;
(b) the machine learning model comprises a classification model, optionally wherein the classification model comprises a supervised classification model, a semi-supervised classification model, an unsupervised classification model, or a combination thereof;
(c) the machine learning model comprises a neural network, a linear regression model, a logistic regression model, a decision tree, a support vector machine, a Naive Bayes network, a k-nearest neighbors (KNN) model, a k-means model, a random forest model, or any combination thereof; and/or
(d) the machine learning model comprises an association rule learning model, an inductive logic programming model, a reinforcement learning model, a feature learning model, a similarity learning model, a sparse dictionary learning model, a genetic algorithm model, a rule-based machine learning model, a learning classifier system model, or any combination thereof.

6. The method of claim 4 or claim 5, wherein training the machine learning model for expression profile multiplet identification from the expression profiles of the plurality of synthetic multiplet expression profiles generated in (d) and the one or more expression profiles of the plurality of expression profiles determined in (c) comprises:
(1) projecting the expression profiles of the plurality of synthetic multiplet expression profiles generated in (d) from an expression profile space into a lower dimensional projection space to generate projected expression profiles of the plurality of synthetic multiplet expression profiles;
(2) projecting the one or more expression profiles of the plurality of expression profiles determined in (c) from the expression profile space into the lower dimensional projection space to generate one or more projected expression profiles of the plurality of expression profiles; and
(3) training the machine learning model for expression profile multiplet identification from the projected expression profiles of the plurality of synthetic multiplet expression profiles from (1) and the one or more projected expression profiles of the plurality of expression profiles in (2).

7. The method of claim 6, comprising:
projecting the expression profile of the plurality of stochastic barcodes associated with the cell label of the cell labels of the plurality of stochastic barcodes to generate a projected expression profile of the plurality of stochastic barcodes,
wherein identifying the expression profile of the plurality of expression profiles associated with the cell label of the cell labels of the plurality of stochastic barcodes as a singlet or a multiplet based on the expression profile using the machine learning model comprises:
identifying the expression profile of the plurality of expression profiles associated with the cell label of the cell labels of the plurality of stochastic barcodes as a singlet or a multiplet based on the expression profile using the machine learning model and the projected expression profile of the plurality of stochastic barcodes.

8. The method of claim 6 or claim 7, wherein:
(a) the lower dimensional space is a two dimensional space; and/or
(b) projecting the expression profiles of the plurality of synthetic multiplet expression profiles generated in (d) from the expression profile space into the lower dimensional projection space to generate projected expression profiles of the plurality of synthetic multiplet expression profiles comprises: projecting the expression profiles of the plurality of synthetic multiplet expression profiles generated in (d) from the expression profile space into the lower dimensional projection space to generate projected expression profiles of the plurality of synthetic multiplet expression profiles using a t-distributed stochastic neighbor embedding (tSNE) method.

9. The method of any one of claims 1-8, wherein identifying the expression profile of the plurality of expression profiles associated with the cell label of the cell labels of the plurality of stochastic barcodes as a singlet or a multiplet based on the expression profiles of the plurality of synthetic multiplet expression profiles generated comprises:
identifying the expression profile of the plurality of expression profiles associated with the cell label of the cell labels of the plurality of stochastic barcodes as a singlet or a multiplet based on the expression profile based on:
a first distance between the expression profile of the plurality of expression profiles associated with the cell label and at least one expression profile of the plurality of expression profiles, and
a second distance between the expression profile of the plurality of expression profiles associated with the cell label and at least one synthetic multiplet expression profile of the plurality of synthetic multiplet expression profiles.

10. The method of any one of claims 1-8, wherein identifying the expression profile of the plurality of expression profiles associated with the cell label of the cell labels of the plurality of stochastic barcodes as a singlet or a multiplet based on the expression profiles of the plurality of synthetic multiplet expression profiles generated comprises:
(1) clustering the plurality of expression profiles into a first cluster of expression profiles;
(2) clustering the plurality of synthetic multiplet expression profiles into a second cluster of synthetic multiplet expression profiles; and
(3) identifying the expression profile of the plurality of expression profiles associated with the cell label of the cell labels of the plurality of stochastic barcodes as a singlet or a multiplet based on the expression profile based on:
a first distance between the expression profile of the plurality of expression profiles associated with the cell label and the first cluster of expression profiles, and
a second distance between the expression profile of the plurality of expression profiles associated with the cell label and a second cluster of synthetic multiplet expression profiles.

11. The method of any one of claims 1-8, wherein identifying the expression profile of the plurality of expression profiles associated with the cell label of the cell labels of the plurality of stochastic barcodes as a singlet or a multiplet based on the expression profiles of the plurality of synthetic multiplet expression profiles generated comprises:
(1) clustering the plurality of expression profiles into a first cluster of expression profiles;
(2) clustering the plurality of synthetic multiplet expression profiles into a plurality of second clusters of synthetic multiplet expression profiles; and
(3) identifying the expression profile of the plurality of expression profiles associated with the cell label of the cell labels of the plurality of stochastic barcodes as a singlet or a multiplet based on the expression profile based on:
a first distance between the expression profile of the plurality of expression profiles associated with the cell label and the first cluster of expression profiles, and
second distances between the expression profile of the plurality of expression profiles associated with the cell label and one or more clusters of the plurality of second clusters of synthetic multiplet expression profiles.

12. The method of any one of claims 1-11, wherein stochastically barcoding the plurality of targets in the plurality of cells using the plurality of stochastic barcodes to create the plurality of stochastically barcoded targets for each cell of the plurality of cells comprises:
stochastically barcoding the plurality of targets in the plurality of cells using the plurality of stochastic barcodes of a plurality of particles to create the plurality of stochastically barcoded targets for each cell of the plurality of cells,
wherein each of the plurality of particles comprises a subset of the plurality of stochastic barcodes, wherein each of the subset of stochastic barcodes comprise an identical cell label sequence and with at least 100 different molecular label sequences.

13. The method of claim 12, wherein:
(a) the particle is a bead, optionally wherein the bead is selected from the group consisting of streptavidin beads, agarose beads, magnetic beads, conjugated beads, protein A conjugated beads, protein G conjugated beads, protein A/G conjugated beads, protein L conjugated beads, oligodT conjugated beads, silica beads, silica-like beads, anti-biotin microbead, anti-fluorochrome microbead, and any combination thereof;
(b) the particle comprises a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, sepharose, cellulose, nylon, silicone, and any combination thereof;
(c) the stochastic barcodes of each of the plurality of particles comprise molecular labels with at least 1000 different molecular label sequences;
(d) the stochastic barcodes of each of the plurality of particles comprise molecular labels with at least 10000 different molecular label sequences;
(e) the molecular labels of the stochastic barcodes comprise random sequences; and/or
(f) each of the plurality of particles comprises at least 10000 stochastic barcodes.

14. The method of any one of claims 1-13, wherein:
(a) stochastically barcoding the plurality of targets in the plurality of cells using the plurality of stochastic barcodes to create the plurality of stochastically barcoded targets for each cell of the plurality of cells comprises:
(1) contacting copies of the targets with target binding regions of the stochastic barcodes; and
(2) reverse transcribing the plurality targets using the plurality of stochastic barcodes to create a plurality of reverse transcribed targets; and/or
(b) the method comprises: prior to obtaining the sequencing data of the plurality of stochastically barcoded targets, amplifying the stochastically barcoded targets to generate a plurality of amplified stochastically barcoded targets, optionally wherein amplifying the stochastically barcoded targets to generate the plurality of amplified stochastically barcoded targets comprises: amplifying the stochastically barcoded targets by polymerase chain reaction (PCR).

15. The method of any one of claims 1-14, wherein determining the plurality of expression profiles associated with the cell labels of the plurality of stochastic barcodes from the sequencing data obtained in (b) comprises: for an expression profile of the plurality of expression profiles associated with a cell label of the cell labels of the plurality of stochastic barcodes, determining a number of molecular labels with distinct sequences associated with the cell label and each target of the plurality of targets in the sequencing data obtained in (b), optionally wherein determining the number of molecular labels with distinct sequences associated with the cell label and each target of the plurality of targets in the sequencing data obtained in (b) comprises:
for one or more of the plurality of targets,
(1) counting the number of molecular labels with distinct sequences associated with the target in the sequencing data obtained in (b); and
(2) estimating the number of the target based on the number of molecular labels with distinct sequences associated with the target in the sequencing data counted in (1).

## Patentansprüche

1. Verfahren zum Identifizieren eines Multiplett-Expressionsprofils in einer Vielzahl von Expressionsprofilen, bestimmt aus einer Vielzahl von stochastisch barcodierten Zielen, sowie zum Entfernen des Multiplett-Expressionsprofils aus der Vielzahl von Expressionsprofilen oder zum Entfernen der mit dem Multiplett-Expressionsprofil verknüpften Sequenzierungsdaten, umfassend:
(a) Erhalten von Sequenzierungsdaten einer Vielzahl von stochastisch barcodierten Zielen, wobei die Vielzahl von stochastisch barcodierten Zielen durch stochastisches Barcodieren einer Vielzahl von Zielen in einer Vielzahl von Zellen unter Einsatz einer Vielzahl von stochastischen Barcodes erzeugt wurde, um eine Vielzahl von stochastisch barcodierten Zielen für jede Zelle der Vielzahl von Zellen zu erzeugen, wobei jeder der Vielzahl von stochastischen Barcodes eine Zellmarkierung und eine molekulare Markierung umfasst, wobei molekulare Markierungen von mindestens zwei stochastischen Barcodes der Vielzahl von stochastischen Barcodes unterschiedliche molekulare Markierungssequenzen umfassen, und wobei mindestens zwei stochastische Barcodes der Vielzahl von stochastischen Barcodes Zellmarkierungen mit einer identischen Zellmarkierungssequenz umfassen;
(c) Bestimmen einer Vielzahl von Expressionsprofilen, die verknüpft sind mit Zellmarkierungen der Vielzahl von stochastischen Barcodes, aus den in (b) erhaltenen Sequenzierungsdaten, wobei ein Expressionsprofil der Vielzahl von Expressionsprofilen, die verknüpft sind mit einer Zellmarkierung der Zellmarkierungen der Vielzahl von stochastischen Barcodes, eine Anzahl von molekularen Markierungen mit unterschiedlichen Sequenzen umfasst, die verknüpft sind mit der Zellmarkierung und jedem Ziel der Vielzahl von Zielen in den Sequenzierungsdaten;
(d) Erzeugen einer Vielzahl von synthetischen Multiplett-Expressionsprofilen aus der Vielzahl von Expressionsprofilen, die verknüpft sind mit den in (c) bestimmten Zellmarkierungen der Vielzahl von stochastischen Barcodes, umfassend, für ein synthetisches Multiplett-Expressionsprofil der Vielzahl von synthetischen Multiplett-Expressionsprofilen:
(1) Auswählen eines ersten Expressionsprofils aus der Vielzahl von in (c) bestimmten Expressionsprofilen, wobei das erste Expressionsprofil verknüpft ist mit einer ersten Zellmarkierungssequenz;
(2) Auswählen eines zweiten Expressionsprofils aus der Vielzahl von in (c) bestimmten Expressionsprofilen, wobei das zweite Expressionsprofil verknüpft ist mit einer zweiten Zellmarkierungssequenz; und wobei die erste Zellmarkierungssequenz und die zweite Zellmarkierungssequenz verschiedene Zellmarkierungssequenzen umfassen; und
(3) Kombinieren des in (1) ausgewählten ersten Expressionsprofils und des in (2) ausgewählten zweiten Expressionsprofils, um das synthetische Multiplett-Expressionsprofil zu erzeugen; und
(e) Identifizieren eines Expressionsprofils der Vielzahl von Expressionsprofilen, die verknüpft sind mit einer Zellmarkierung der Zellmarkierungen der Vielzahl von stochastischen Barcodes, als ein Singulett oder ein Multiplett basierend auf einer Vielzahl von in (d) erzeugten synthetischen Doublett-Expressionsprofilen; und
(f) wenn das Expressionsprofil in (e) als Multiplett identifiziert wird:
(1) Entfernen der mit dem Expressionsprofil verknüpften Sequenzierungsdaten aus den in b) erhaltenen Sequenzierungsdaten; oder
(2) Entfernen des Expressionsprofils aus der in (c) bestimmten Vielzahl von Expressionsprofilen.

2. Verfahren nach Anspruch 1, wobei die Vielzahl von Multipletts ein Doublett, ein Triplett oder eine beliebige Kombination daraus umfasst.

3. Verfahren nach Anspruch 1 oder 2, wobei das Kombinieren des in (1) ausgewählten ersten Expressionsprofils und des in (2) ausgewählten zweiten Expressionsprofils, um das synthetische Multiplett-Expressionsprofil zu erzeugen, umfasst: für jedes der Vielzahl von Zielen, Kombinieren einer Anzahl molekularer Markierungen mit verschiedenen Sequenzen, die verknüpft sind mit dem Ziel im ersten Expressionsprofil, und einer Anzahl molekularer Markierungen mit verschiedenen Sequenzen, die verknüpft sind mit dem Ziel im zweiten Expressionsprofil, um eine Anzahl molekularer Markierungen mit verschiedenen Sequenzen zu erzeugen, die verknüpft sind mit dem synthetischen Multiplett-Expressionsprofil, optional wobei:
(a) die Anzahl molekularer Markierungen mit verschiedenen Sequenzen, die verknüpft sind mit dem synthetischen Multiplett-Expressionsprofil, eine Summe der Anzahl molekularer Markierungen mit verschiedenen Sequenzen, die verknüpft sind mit dem Ziel im ersten Expressionsprofil, und der Anzahl molekularer Markierungen mit verschiedenen Sequenzen, die verknüpft sind mit dem Ziel in dem zweiten Expressionsprofil, ist, optional wobei die Summe eine gewichtete Summe der Anzahl molekularer Markierungen mit verschiedenen Sequenzen, die verknüpft sind mit dem Ziel im ersten Expressionsprofil, und der Anzahl molekularer Markierungen mit verschiedenen Sequenzen, die verknüpft sind mit dem Ziel in dem zweiten Expressionsprofil, ist; oder
(b) die Anzahl molekularer Markierungen mit verschiedenen Sequenzen, die verknüpft sind mit dem synthetischen Multiplett-Expressionsprofil, ein Durchschnitt der Anzahl molekularer Markierungen mit verschiedenen Sequenzen, die verknüpft sind mit dem Ziel im ersten Expressionsprofil, und der Anzahl molekularer Markierungen mit verschiedenen Sequenzen, die verknüpft sind mit dem Ziel in dem zweiten Expressionsprofil, ist, optional wobei der Durchschnitt ein gewichteter Durchschnitt der Anzahl molekularer Markierungen mit verschiedenen Sequenzen, die verknüpft sind mit dem Ziel im ersten Expressionsprofil, und der Anzahl molekularer Markierungen mit verschiedenen Sequenzen, die verknüpft sind mit dem Ziel in dem zweiten Expressionsprofil, ist.

4. Verfahren nach einem der Ansprüche 1-3, wobei das Identifizieren des Expressionsprofils der Vielzahl von Expressionsprofilen, die verknüpft sind mit der Zellmarkierung der Zellmarkierungen der Vielzahl von stochastischen Barcodes, als ein Singulett oder ein Multiplett basierend auf den Expressionsprofilen der Vielzahl von erzeugten synthetischen Multiplett-Expressionsprofilen, umfasst:
(1) Trainieren eines Maschinenlernmodells zur Identifizierung von Expressionsprofil-Multipletts aus den Expressionsprofilen der Vielzahl von in (d) erzeugten synthetischen Multiplett-Expressionsprofilen und einem oder mehreren Expressionsprofilen der Vielzahl von in (c) bestimmten Expressionsprofilen; und
(2) Identifizieren des Expressionsprofils der Vielzahl von Expressionsprofilen, die verknüpft sind mit der Zellmarkierung der Zellmarkierungen der Vielzahl von stochastischen Barcodes, als ein Singulett oder ein Multiplett basierend auf dem Expressionsprofil unter Einsatz des Maschinenlernmodells.

5. Verfahren nach Anspruch 4, wobei:
(a) das eine oder die mehreren Expressionsprofile der Vielzahl von Expressionsprofilen, die beim Trainieren des Maschinenlernmodells verwendet werden, einen Prozentsatz der in (c) bestimmten Vielzahl von Expressionsprofilen umfassen, optional wobei der Prozentsatz im Bereich von 1-50 % liegt und vorzugsweise etwa 10 % beträgt;
(b) das Maschinenlernmodell ein Klassifizierungsmodell umfasst, optional wobei das Klassifizierungsmodell ein überwachtes Klassifizierungsmodell, ein halbüberwachtes Klassifizierungsmodell, ein unüberwachtes Klassifizierungsmodell oder eine Kombination daraus umfasst;
(c) das Maschinenlernmodell ein neuronales Netzwerk, ein lineares Regressionsmodell, ein logistisches Regressionsmodell, einen Entscheidungsbaum, eine Support-Vector-Maschine, ein Naive-Bayes-Netzwerk, ein K-Nearest-Neighbors(KNN)-Modell, ein K-Means-Modell, ein Random-Forest-Modell oder eine beliebige Kombination daraus umfasst; und/oder
(d) das Maschinenlernmodell ein Assoziationsregel-Lernmodell, ein induktives Logikprogrammiermodell, ein Verstärkungslernmodell, ein Merkmalslernmodell, ein Ähnlichkeitslernmodell, ein Sparse-Dictionary-Lernmodell, ein genetisches Algorithmusmodell, ein regelbasiertes Maschinenlernmodell, ein Lernklassifizierungssystemmodell oder eine beliebige Kombination daraus umfasst.

6. Verfahren nach Anspruch 4 oder 5, wobei das Trainieren des Maschinenlernmodells zur Identifizierung von Expressionsprofil-Multipletts aus den Expressionsprofilen der Vielzahl von in (d) erzeugten synthetischen Multiplett-Expressionsprofilen und dem einen oder den mehreren Expressionsprofilen der Vielzahl von in (c) bestimmten Expressionsprofilen umfasst:
(1) Projizieren der Expressionsprofile der Vielzahl von in (d) erzeugten synthetischen Multiplett-Expressionsprofilen aus einem Expressionsprofilraum in einen niedrigdimensionalen Projektionsraum zum Erzeugen von projizierten Expressionsprofilen der Vielzahl von synthetischen Multiplett-Expressionsprofilen;
(2) Projizieren des einen oder der mehreren Expressionsprofile der Vielzahl von in (c) bestimmten synthetischen Expressionsprofilen aus dem Expressionsprofilraum in den niedrigdimensionalen Projektionsraum zum Erzeugen von einem oder mehreren projizierten Expressionsprofilen der Vielzahl von Expressionsprofilen; und
(3) Trainieren des Maschinenlernmodells zur Identifizierung von Expressionsprofil-Multipletts aus den projizierten Expressionsprofilen der Vielzahl von in synthetischen Multiplett-Expressionsprofilen aus (1) und dem einem oder den mehreren projizierten Expressionsprofilen der Vielzahl von Expressionsprofilen in (2).

7. Verfahren nach Anspruch 6, umfassend:
Projizieren des Expressionsprofils der Vielzahl von stochastischen Barcodes, die verknüpft sind mit der Zellmarkierung der Zellmarkierungen der Vielzahl von stochastischen Barcodes, um ein projiziertes Expressionsprofil der Vielzahl von stochastischen Barcodes zu erzeugen,
wobei das Identifizieren des Expressionsprofils der Vielzahl von Expressionsprofilen, die verknüpft sind mit der Zellmarkierung der Zellmarkierungen der Vielzahl von stochastischen Barcodes, als ein Singulett oder ein Multiplett basierend auf dem Expressionsprofil unter Einsatz des Maschinenlernmodells, umfasst:
Identifizieren des Expressionsprofils der Vielzahl von Expressionsprofilen, die verknüpft sind mit der Zellmarkierung der Zellmarkierungen der Vielzahl von stochastischen Barcodes, als ein Singulett oder ein Multiplett basierend auf dem Expressionsprofil unter Einsatz des Maschinenlernmodells und des projizierten Expressionsprofils der Vielzahl von stochastischen Barcodes.

8. Verfahren nach Anspruch 6 oder 7, wobei:
(a) der niedrigdimensionale Raum ein zweidimensionaler Raum ist; und/oder
(b) das Projizieren der Expressionsprofile der Vielzahl von in (d) erzeugten synthetischen Multiplett-Expressionsprofilen aus dem Expressionsprofilraum in den niedrigdimensionalen Projektionsraum zum Erzeugen von projizierten Expressionsprofilen der Vielzahl von synthetischen Multiplett-Expressionsprofilen, umfasst: Projizieren der Expressionsprofile der Vielzahl von in (d) erzeugten synthetischen Multiplett-Expressionsprofilen aus dem Expressionsprofilraum in den niedrigdimensionalen Projektionsraum zum Erzeugen von projizierten Expressionsprofilen der Vielzahl von synthetischen Multiplett-Expressionsprofilen unter Einsatz eines Verfahrens der t-verteilten stochastische Nachbareinbettung (tSNE).

9. Verfahren nach einem der Ansprüche 1-8, wobei das Identifizieren des Expressionsprofils der Vielzahl von Expressionsprofilen, die verknüpft sind mit der Zellmarkierung der Zellmarkierungen der Vielzahl von stochastischen Barcodes, als ein Singulett oder ein Multiplett basierend auf den Expressionsprofilen der Vielzahl von erzeugten synthetischen Multiplett-Expressionsprofilen, umfasst:
Identifizieren des Expressionsprofils der Vielzahl von Expressionsprofilen, die verknüpft sind mit der Zellmarkierung der Zellmarkierungen der Vielzahl von stochastischen Barcodes, als ein Singulett oder ein Multiplett basierend auf dem Expressionsprofil, basierend auf:
einem ersten Abstand zwischen dem Expressionsprofil der Vielzahl von Expressionsprofilen, verknüpft mit der Zellmarkierung, und mindestens einem Expressionsprofil der Vielzahl von Expressionsprofilen, und
einem zweiten Abstand zwischen dem Expressionsprofil der Vielzahl von Expressionsprofilen, verknüpft mit der Zellmarkierung, und mindestens einem synthetischen Multiplett-Expressionsprofil der Vielzahl von synthetischen Multiplett-Expressionsprofilen.

10. Verfahren nach einem der Ansprüche 1-8, wobei das Identifizieren des Expressionsprofils der Vielzahl von Expressionsprofilen, die verknüpft sind mit der Zellmarkierung der Zellmarkierungen der Vielzahl von stochastischen Barcodes, als ein Singulett oder ein Multiplett basierend auf den Expressionsprofilen der Vielzahl von erzeugten synthetischen Multiplett-Expressionsprofilen, umfasst:
(1) Clustern der Vielzahl von Expressionsprofilen zu einem ersten Cluster von Expressionsprofilen;
(2) Clustern der Vielzahl von synthetischen Multiplett-Expressionsprofilen zu einem zweiten Cluster von synthetischen Multiplett-Expressionsprofilen; und
(3) Identifizieren des Expressionsprofils der Vielzahl von Expressionsprofilen, die verknüpft sind mit der Zellmarkierung der Zellmarkierungen der Vielzahl von stochastischen Barcodes, als ein Singulett oder ein Multiplett basierend auf dem Expressionsprofil, basierend auf:
einem ersten Abstand zwischen dem Expressionsprofil der Vielzahl von Expressionsprofilen, verknüpft mit der Zellmarkierung, und dem ersten Cluster von Expressionsprofilen, und
einem zweiten Abstand zwischen dem Expressionsprofil der Vielzahl von Expressionsprofilen, verknüpft mit der Zellmarkierung, und einem zweiten Cluster von synthetischen Multiplett-Expressionsprofilen.

11. Verfahren nach einem der Ansprüche 1-8, wobei das Identifizieren des Expressionsprofils der Vielzahl von Expressionsprofilen, die verknüpft sind mit der Zellmarkierung der Zellmarkierungen der Vielzahl von stochastischen Barcodes, als ein Singulett oder ein Multiplett basierend auf den Expressionsprofilen der Vielzahl von erzeugten synthetischen Multiplett-Expressionsprofilen, umfasst:
(1) Clustern der Vielzahl von Expressionsprofilen zu einem ersten Cluster von Expressionsprofilen;
(2) Clustern der Vielzahl von synthetischen Multiplett-Expressionsprofilen zu einer Vielzahl von zweiten Clustern von synthetischen Multiplett-Expressionsprofilen; und
(3) Identifizieren des Expressionsprofils der Vielzahl von Expressionsprofilen, die verknüpft sind mit der Zellmarkierung der Zellmarkierungen der Vielzahl von stochastischen Barcodes, als ein Singulett oder ein Multiplett basierend auf dem Expressionsprofil, basierend auf:
einem ersten Abstand zwischen dem Expressionsprofil der Vielzahl von Expressionsprofilen, verknüpft mit der Zellmarkierung, und dem ersten Cluster von Expressionsprofilen, und
zweiten Abständen zwischen dem Expressionsprofil der Vielzahl von Expressionsprofilen, verknüpft mit der Zellmarkierung und einem oder mehreren Clustern der Vielzahl von zweiten Clustern von synthetischen Multiplett-Expressionsprofilen.

12. Verfahren nach einem der Ansprüche 1-11, wobei stochastisches Barcodieren der Vielzahl von Zielen in der Vielzahl von Zellen unter Einsatz der Vielzahl von stochastischen Barcodes, um die Vielzahl von stochastisch barcodierten Zielen für jede Zelle der Vielzahl von Zellen zu erzeugen, umfasst:
stochastisches Barcodieren der Vielzahl von Zielen in der Vielzahl von Zellen unter Einsatz der Vielzahl von stochastischen Barcodes einer Vielzahl von Partikeln, um die Vielzahl von stochastisch barcodierten Zielen für jede Zelle der Vielzahl von Zellen zu erzeugen,
wobei jedes der Vielzahl von Partikeln eine Teilmenge der Vielzahl von stochastischen Barcodes umfasst, wobei jeder der Teilmenge von stochastischen Barcodes eine identische Zellmarkierungssequenz und mindestens 100 verschiedene molekulare Markierungssequenzen umfasst.

13. Verfahren nach Anspruch 12, wobei:
(a) das Partikel ein Bead ist, optional wobei das Bead optional ausgewählt ist aus der Gruppe bestehend aus Streptavidin-Beads, Agarose-Beads, magnetischen Beads, konjugierten Beads, Protein-A-konjugierten Beads, Protein-G-konjugierten Beads, Protein-A/G-konjugierten Beads, Protein-L-konjugierten Beads, OligodTkonjugierten Beads, Siliciumoxid-Beads, Siliciumoxidähnlichen Beads, Anti-Biotin-Mikrobeads, Anti-Fluorchrom-Mikrobeads sowie jeglichen Kombinationen daraus;
(b) das Partikel ein Material umfasst, ausgewählt aus der Gruppe bestehend aus Polydimethylsiloxan (PDMS), Polystyrol, Glas, Polypropylen, Agarose, Gelatine, Hydrogel, Paramagnetikum, Keramik, Kunststoff, Glas, Methylstyrol, Acrylpolymer, Titan, Latex, Sepharose, Zellulose, Nylon, Silikon sowie einer beliebigen Kombination daraus;
(c) die stochastischen Barcodes jedes der Vielzahl von Partikeln molekulare Markierungen mit mindestens 1.000 verschiedenen molekularen Markierungssequenzen umfassen;
(d) die stochastischen Barcodes jedes der Vielzahl von Partikeln molekulare Markierungen mit mindestens 10.000 verschiedenen molekularen Markierungssequenzen umfassen;
(e) die molekularen Markierungen der stochastischen Barcodes Zufallssequenzen umfassen; und/oder
(f) jedes der Vielzahl von Partikeln mindestens 10.000 stochastische Barcodes umfasst.

14. Verfahren nach einem der Ansprüche 1-13, wobei:
(a) stochastisches Barcodieren der Vielzahl von Zielen in der Vielzahl von Zellen unter Einsatz der Vielzahl von stochastischen Barcodes, um die Vielzahl von stochastisch barcodierten Zielen für jede Zelle der Vielzahl von Zellen zu erzeugen, umfasst:
(1) Inberührungbringen von Kopien der Ziele mit Zielbindungsregionen der stochastischen Barcodes; und
(2) reverses Transkribieren der Vielzahl von Zielen unter Einsatz der Vielzahl von stochastischen Barcodes, um eine Vielzahl von revers transkribierten Zielen zu erzeugen; und/oder
(b) das Verfahren umfasst: vor dem Erhalten der Sequenzierungsdaten der Vielzahl von stochastisch barcodierten Zielen, Amplifizieren der stochastisch barcodierten Ziele, um eine Vielzahl von amplifizierten stochastisch barcodierten Zielen zu erzeugen, optional wobei das Amplifizieren der stochastisch barcodierten Ziele zum Erzeugen der Vielzahl amplifizierter stochastisch barcodierter Ziele umfasst: Amplifizieren der stochastisch barcodierten Ziele durch Polymerasekettenreaktion (PCR).

15. Verfahren nach einem der Ansprüche 1-14, wobei das Bestimmen der Vielzahl von Expressionsprofilen, die verknüpft sind mit den Zellmarkierungen der Vielzahl von stochastischen Barcodes, aus den in (b) erhaltenen Sequenzierungsdaten, umfasst: für ein Expressionsprofil der Vielzahl von Expressionsprofilen, die verknüpft sind mit einer Zellmarkierung der Zellmarkierungen der Vielzahl von stochastischen Barcodes, eine Anzahl von molekularen Markierungen mit unterschiedlichen Sequenzen bestimmt, die verknüpft sind mit der Zellmarkierung und jedem Ziel der Vielzahl von Zielen in den in (b) erhaltenen Sequenzierungsdaten, optional wobei das Bestimmen der Anzahl der molekularen Markierungen mit verschiedenen Sequenzen, die verknüpft sind mit der Zellmarkierung und jedem Ziel der Vielzahl von Zielen in den in (b) erhaltenen Sequenzierungsdaten, umfasst: für eines oder mehrere der Vielzahl von Zielen,
(1) Zählen der Anzahl der molekularen Markierungen mit verschiedenen Sequenzen, die mit dem Ziel verknüpft sind, in den in (b) erhaltenen Sequenzierungsdaten; und
(2) Abschätzen der Anzahl der Ziele auf der Grundlage der Anzahl der molekularen Markierungen mit verschiedenen Sequenzen, die mit dem Ziel verknüpft sind, in den in (1) gezählten Sequenzierungsdaten.

## Revendications

1. Procédé d'identification d'un profil d'expression multiplet dans une pluralité de profils d'expression déterminés à partir de données de séquençage d'une pluralité de cibles dotées d'un code-barres de manière stochastique, et de suppression du profil d'expression multiplet de la pluralité de profils d'expression ou de suppression des données de séquençage associées au profil d'expression multiplet, comprenant :
(a) l'obtention de données de séquençage d'une pluralité de cibles dotées d'un code-barres de manière stochastique, la pluralité de cibles dotées d'un code-barres de manière stochastique étant générée par attribution d'un code-barres de manière stochastique à une pluralité de cibles dans une pluralité de cellules à l'aide d'une pluralité de codes-barres stochastiques pour créer une pluralité de cibles dotées d'un code-barres de manière stochastique pour chaque cellule de la pluralité de cellules, chacun de la pluralité de codes-barres stochastiques comprenant un marqueur cellulaire et un marqueur moléculaire, les marqueurs moléculaires d'au moins deux codes-barres stochastiques de la pluralité de codes-barres stochastiques comprenant des séquences de marqueur moléculaire différentes, et au moins deux codes-barres stochastiques de la pluralité de codes-barres stochastiques comprenant des marqueurs cellulaires avec une séquence de marqueur cellulaire identique ;
(c) la détermination d'une pluralité de profils d'expression associés à des marqueurs cellulaires de la pluralité de codes-barres stochastiques à partir des données de séquençage obtenues dans (b), un profil d'expression de la pluralité de profils d'expression associés à un marqueur cellulaire des marqueurs cellulaires de la pluralité de codes-barres stochastiques comprenant une pluralité de marqueurs moléculaires ayant des séquences distinctes associées au marqueur cellulaire et à chaque cible de la pluralité de cibles dans les données de séquençage ;
(d) la génération d'une pluralité de profils d'expression multiplets synthétiques à partir de la pluralité de profils d'expression associés aux marqueurs cellulaires de la pluralité de codes-barres stochastiques déterminés dans (c), comprenant, pour un profil d'expression multiplet synthétique de la pluralité de profils d'expression multiplets synthétiques :
(1) la sélection d'un premier profil d'expression de la pluralité de profils d'expression déterminés dans (c), le premier profil d'expression étant associé à une première séquence de marqueur cellulaire ;
(2) la sélection d'un deuxième profil d'expression de la pluralité de profils d'expression déterminés dans (c), le deuxième profil d'expression étant associé à une deuxième séquence de marqueur cellulaire, et la première séquence de marqueur cellulaire et la deuxième séquence de marqueur cellulaire comprenant des séquences de marqueur cellulaire différentes ; et
(3) la combinaison du premier profil d'expression sélectionné dans (1) et du deuxième profil d'expression sélectionné dans (2) pour générer le profil d'expression multiplet synthétique ; et
(e) l'identification d'un profil d'expression de la pluralité de profils d'expression associés à un marqueur cellulaire des marqueurs cellulaires de la pluralité de codes-barres stochastiques en tant que singulet ou multiplet sur la base de la pluralité de profils d'expression doublets synthétiques générés dans (d) ; et
(f) si le profil d'expression est identifié comme un multiplet dans (e) :
(1) la suppression des données de séquençage associées au profil d'expression des données de séquençage obtenues dans (b) ; ou
(2) la suppression du profil d'expression de la pluralité de profils d'expression déterminés dans (c).

2. Procédé selon la revendication 1, dans lequel la pluralité de multiplets comprend un doublet, un triplet ou une combinaison quelconque de ceux-ci.

3. Procédé selon la revendication 1 ou la revendication 2, dans lequel la combinaison du premier profil d'expression sélectionné dans (1) et du deuxième profil d'expression sélectionné dans (2) pour générer le profil d'expression multiplet synthétique comprend: pour chacune de la pluralité de cibles, la combinaison d'une pluralité de marqueurs moléculaires avec des séquences distinctes associées à la cible dans le premier profil d'expression et d'une pluralité de marqueurs moléculaires avec des séquences distinctes associées à la cible dans le deuxième profil d'expression pour générer une pluralité de marqueurs moléculaires ayant des séquences distinctes associées au profil d'expression multiplet synthétique, éventuellement dans lequel :
(a) le nombre de marqueurs moléculaires avec des séquences distinctes associées au profil d'expression multiplet synthétique est une somme du nombre de marqueurs moléculaires avec des séquences distinctes associées à la cible dans le premier profil d'expression et du nombre de marqueurs moléculaires avec des séquences distinctes associées à la cible dans le deuxième profil d'expression, éventuellement dans lequel la somme est une somme pondérée du nombre de marqueurs moléculaires avec des séquences distinctes associées à la cible dans le premier profil d'expression et du nombre de marqueurs moléculaires avec des séquences distinctes associées à la cible dans le deuxième profil d'expression ; ou
(b) le nombre de marqueurs moléculaires avec des séquences distinctes associées au profil d'expression multiplet synthétique est une moyenne du nombre de marqueurs moléculaires avec des séquences distinctes associées à la cible dans le premier profil d'expression et du nombre de marqueurs moléculaires avec des séquences distinctes associées à la cible dans le deuxième profil d'expression, éventuellement dans lequel la moyenne est une moyenne pondérée du nombre de marqueurs moléculaires avec des séquences distinctes associées à la cible dans le premier profil d'expression et du nombre de marqueurs moléculaires avec des séquences distinctes associées à la cible dans le deuxième profil d'expression.

4. Procédé selon l'une quelconque des revendications 1 à 3, dans lequel l'identification du profil d'expression de la pluralité de profils d'expression associés au marqueur cellulaire des marqueurs cellulaires de la pluralité de codes-barres stochastiques en tant que singulet ou multiplet sur la base des profils d'expression de la pluralité de profils d'expression multiplets synthétiques générés comprend :
(1) l'entraînement d'un modèle d'apprentissage automatique pour l'identification de multiplet de profil d'expression à partir des profils d'expression de la pluralité de profils d'expression multiplets synthétiques générés dans (d) et d'un ou plusieurs profils d'expression de la pluralité de profils d'expression déterminés dans (c) ; et
(2) l'identification du profil d'expression de la pluralité de profils d'expression associés au marqueur cellulaire des marqueurs cellulaires de la pluralité de codes-barres stochastiques en tant que singulet ou multiplet sur la base du profil d'expression à l'aide du modèle d'apprentissage automatique.

5. Procédé selon la revendication 4, dans lequel :
(a) les un ou plusieurs profils d'expression de la pluralité de profils d'expression utilisés dans l'entraînement du modèle d'apprentissage automatique comprennent un pourcentage de la pluralité de profils d'expression déterminés dans (c), le pourcentage étant éventuellement dans la plage de 1 à 50 %, le pourcentage étant de préférence d'environ 10 pour cent ;
(b) le modèle d'apprentissage automatique comprend un modèle de classification, le modèle de classification comprenant éventuellement un modèle de classification supervisée, un modèle de classification semi-supervisée, un modèle de classification non supervisée ou une combinaison de ceux-ci ;
(c) le modèle d'apprentissage automatique comprend un réseau neuronal, un modèle de régression linéaire, un modèle de régression logistique, un arbre de décision, une machine à vecteurs de support, un réseau bayésien naïf, un modèle des k plus proches voisins (KNN), un modèle à k moyennes, un modèle de forêt aléatoire, ou une combinaison quelconque de ceux-ci ; et/ou
(d) le modèle d'apprentissage automatique comprend un modèle d'apprentissage de règles d'association, un modèle de programmation logique inductive, un modèle d'apprentissage par renforcement, un modèle d'apprentissage de représentations, un modèle d'apprentissage de similarités, un modèle d'apprentissage de codage parcimonieux, un modèle d'algorithme génétique, un modèle d'apprentissage automatique basé sur des règles, un modèle de système de classificateur d'apprentissage, ou une combinaison quelconque de ceux-ci.

6. Procédé selon la revendication 4 ou la revendication 5, dans lequel l'entraînement du modèle d'apprentissage automatique pour l'identification de multiplet de profil d'expression à partir des profils d'expression de la pluralité de profils d'expression multiplets synthétiques générés dans (d) et les un ou plusieurs profils d'expression de la pluralité de profils d'expression déterminés dans (c) comprend :
(1) la projection des profils d'expression de la pluralité de profils d'expression multiplets synthétiques générés dans (d) à partir d'un espace de profil d'expression dans un espace de projection de dimension inférieure pour générer des profils d'expression projetés de la pluralité de profils d'expression multiplets synthétiques ;
(2) la projection des un ou plusieurs profils d'expression de la pluralité de profils d'expression déterminés dans (c) à partir de l'espace de profil d'expression dans l'espace de projection de dimension inférieure pour générer un ou plusieurs profils d'expression projetés de la pluralité de profils d'expression ; et
(3) l'entraînement du modèle d'apprentissage automatique pour l'identification de multiplet de profil d'expression à partir des profils d'expression projetés de la pluralité de profils d'expression multiplets synthétiques de (1) et des un ou plusieurs profils d'expression projetés de la pluralité de profils d'expression dans (2).

7. Procédé selon la revendication 6, comprenant :
la projection du profil d'expression de la pluralité de codes-barres stochastiques associés au marqueur cellulaire des marqueurs cellulaires de la pluralité de codes-barres stochastiques pour générer un profil d'expression projeté de la pluralité de codes-barres stochastiques,
dans lequel l'identification du profil d'expression de la pluralité de profils d'expression associés au marqueur cellulaire des marqueurs cellulaires de la pluralité de codes-barres stochastiques en tant que singulet ou multiplet sur la base du profil d'expression à l'aide du modèle d'apprentissage automatique comprend :
l'identification du profil d'expression de la pluralité de profils d'expression associés au marqueur cellulaire des marqueurs cellulaires de la pluralité de codes-barres stochastiques en tant que singulet ou multiplet sur la base du profil d'expression à l'aide du modèle d'apprentissage automatique et du profil d'expression projeté de la pluralité de codes-barres stochastiques.

8. Procédé selon la revendication 6 ou la revendication 7, dans lequel :
(a) l'espace de dimension inférieure est un espace bidimensionnel ; et/ou
(b) la projection des profils d'expression de la pluralité de profils d'expression de multiplets synthétiques générés dans (d) à partir de l'espace de profil d'expression dans l'espace de projection de dimension inférieure pour générer des profils d'expression projetés de la pluralité de profils d'expression multiplets synthétiques comprend : la projection des profils d'expression de la pluralité de profils d'expression multiplets synthétiques générés dans (d) à partir de l'espace de profil d'expression dans l'espace de projection de dimension inférieure pour générer des profils d'expression projetés de la pluralité de profils d'expression multiplets synthétiques à l'aide d'une méthode tSNE (t-distributed stochastic neighbor embedding).

9. Procédé selon l'une quelconque des revendications 1 à 8, dans lequel l'identification du profil d'expression de la pluralité de profils d'expression associés au marqueur cellulaire des marqueurs cellulaires de la pluralité de codes-barres stochastiques en tant que singulet ou multiplet sur la base des profils d'expression de la pluralité de profils d'expression multiplets synthétiques générés comprend :
l'identification du profil d'expression de la pluralité de profils d'expression associés au marqueur cellulaire des marqueurs cellulaires de la pluralité de codes-barres stochastiques en tant que singulet ou multiplet sur la base du profil d'expression à l'aide du modèle au moyen de :
une première distance entre le profil d'expression de la pluralité de profils d'expression associés au marqueur cellulaire et au moins un profil d'expression de la pluralité de profils d'expression, et
une deuxième distance entre le profil d'expression de la pluralité de profils d'expression associés au marqueur cellulaire et au moins un profil d'expression multiplet synthétique de la pluralité de profils d'expression multiplets synthétiques.

10. Procédé selon l'une quelconque des revendications 1 à 8, dans lequel l'identification du profil d'expression de la pluralité de profils d'expression associés au marqueur cellulaire des marqueurs cellulaires de la pluralité de codes-barres stochastiques en tant que singulet ou multiplet sur la base des profils d'expression de la pluralité de profils d'expression multiplets synthétiques générés comprend :
(1) le partitionnement de la pluralité de profils d'expression dans un premier cluster de profils d'expression ;
(2) le partitionnement de la pluralité de profils d'expression multiplets synthétiques dans un deuxième cluster de profils d'expression multiplets synthétiques ; et
(3) l'identification du profil d'expression de la pluralité de profils d'expression associés au marqueur cellulaire des marqueurs cellulaires de la pluralité de codes-barres stochastiques en tant que singulet ou multiplet sur la base du profil d'expression à l'aide du modèle au moyen de :
une première distance entre le profil d'expression de la pluralité de profils d'expression associés au marqueur cellulaire et le premier cluster de profils d'expression, et
une deuxième distance entre le profil d'expression de la pluralité de profils d'expression associés au marqueur cellulaire et un deuxième cluster de profils d'expression multiplets synthétiques.

11. Procédé selon l'une quelconque des revendications 1 à 8, dans lequel l'identification du profil d'expression de la pluralité de profils d'expression associés au marqueur cellulaire des marqueurs cellulaires de la pluralité de codes-barres stochastiques en tant que singulet ou multiplet sur la base des profils d'expression de la pluralité de profils d'expression multiplets synthétiques générés comprend :
(1) le partitionnement de la pluralité de profils d'expression dans un premier cluster de profils d'expression ;
(2) le partitionnement de la pluralité de profils d'expression multiplets synthétiques en une pluralité de deuxièmes clusters de profils d'expression multiplets synthétiques ; et
(3) l'identification du profil d'expression de la pluralité de profils d'expression associés au marqueur cellulaire des marqueurs cellulaires de la pluralité de codes-barres stochastiques en tant que singulet ou multiplet sur la base du profil d'expression à l'aide du modèle au moyen de :
une première distance entre le profil d'expression de la pluralité de profils d'expression associés au marqueur cellulaire et le premier cluster de profils d'expression, et
des deuxièmes distances entre le profil d'expression de la pluralité de profils d'expression associés au marqueur cellulaire et un ou plusieurs clusters de la pluralité de deuxièmes clusters de profils d'expression multiplets synthétiques.

12. Procédé selon l'une quelconque des revendications 1 à 11, dans lequel l'attribution de code-barres de manière stochastique à la pluralité de cibles dans la pluralité de cellules au moyen de la pluralité de codes-barres stochastiques pour créer la pluralité de cibles dotées d'un code-barres de manière stochastique pour chaque cellule de la pluralité de cellules comprend :
l'attribution de code-barres de manière stochastique à la pluralité de cibles dans la pluralité de cellules au moyen de la pluralité de codes-barres stochastiques d'une pluralité de particules pour créer la pluralité de cibles dotées d'un code-barres de manière stochastique pour chaque cellule de la pluralité de cellules,
dans lequel chacune de la pluralité de particules comprend un sous-ensemble de la pluralité de codes-barres stochastiques, chacun du sous-ensemble de codes-barres stochastiques comprenant une séquence de marqueur cellulaire identique et avec au moins 100 séquences de marqueur moléculaire différentes.

13. Procédé selon la revendication 12, dans lequel :
(a) la particule est une bille, la bille étant éventuellement choisie dans le groupe constitué de billes de streptavidine, de billes d'agarose, de billes magnétiques, de billes conjuguées, de billes conjuguées à la protéine A, de billes conjuguées à la protéine G, de billes conjuguées à la protéine A/G, de billes conjuguées à la protéine L, de billes conjuguées à oligodT, de billes de silice, de billes de type silice, de microbilles anti-biotine, de microbilles anti-fluorochrome, et une combinaison quelconque de celles-ci ;
(b) la particule comprend un matériau choisi dans le groupe constitué par le polydiméthylsiloxane (PDMS), le polystyrène, le verre, le polypropylène, l'agarose, la gélatine, un hydrogel, un matériau paramagnétique, une céramique, une matière plastique, le verre, le méthylstyrène, un polymère acrylique, le titane, le latex, le Sepharose, la cellulose, le nylon, le silicone et une combinaison quelconque de ceux-ci ;
(c) les codes-barres stochastiques de chacune de la pluralité de particules comprennent des marqueurs moléculaires avec au moins 1 000 séquences de marqueur moléculaire différentes ;
(d) les codes-barres stochastiques de chacune de la pluralité de particules comprennent des marqueurs moléculaires avec au moins 10 000 séquences de marqueurs moléculaires différentes ;
(e) les marqueurs moléculaires des codes-barres stochastiques comprennent des séquences aléatoires ; et/ou
(f) chacune de la pluralité de particules comprend au moins 10 000 codes-barres stochastiques.

14. Procédé selon l'une quelconque des revendications 1 à 13, dans lequel :
(a) l'attribution de code-barres de manière stochastique à la pluralité de cibles dans la pluralité de cellules au moyen de la pluralité de codes-barres stochastiques pour créer la pluralité de cibles dotées d'un code-barres de manière stochastique pour chaque cellule de la pluralité de cellules comprend :
(1) la mise en contact de copies des cibles avec des régions de liaison cibles des codes-barres stochastiques ; et
(2) la transcription inverse de la pluralité de cibles à l'aide de la pluralité de codes-barres stochastiques pour créer une pluralité de cibles transcrites par transcription inverse ; et/ou
(b) le procédé comprend : avant d'obtenir les données de séquençage de la pluralité de cibles dotées d'un code-barres de manière stochastique, l'amplification des cibles dotées d'un code-barres de manière stochastique pour générer une pluralité de cibles dotées d'un code-barres de manière stochastique amplifiées, l'amplification des cibles dotées d'un code-barres de manière stochastique pour générer la pluralité de cibles dotées d'un code-barres de manière stochastique amplifiées comprenant éventuellement : l'amplification des cibles dotées d'un code-barres de manière stochastique par amplification en chaîne par polymérase (PCR) .

15. Procédé selon l'une quelconque des revendications 1 à 14, dans lequel la détermination de la pluralité de profils d'expression associés aux marqueurs cellulaires de la pluralité de codes-barres stochastiques à partir des données de séquençage obtenues dans (b) comprend : pour un profil d'expression de la pluralité de profils d'expression associés à un marqueur cellulaire des marqueurs cellulaires de la pluralité de codes-barres stochastiques, la détermination d'une pluralité de marqueurs moléculaires avec des séquences distinctes associées au marqueur cellulaire et à chaque cible de la pluralité de cibles dans les données de séquençage obtenues dans (b), la détermination du nombre de marqueurs moléculaires avec des séquences distinctes associées au marqueur cellulaire et à chaque cible de la pluralité de cibles dans les données de séquençage obtenues dans (b) comprenant éventuellement :
pour l'une ou plusieurs de la pluralité de cibles,
(1) le comptage du nombre de marqueurs moléculaires avec des séquences distinctes associées à la cible dans les données de séquençage obtenues dans (b) ; et
(2) l'estimation du nombre de cibles en fonction du nombre de marqueurs moléculaires avec des séquences distinctes associés à la cible dans les données de séquençage comptées dans (1).
